(19) Europäisches Patentamt
European Patent Office
Office européen des brevets

(11) EP 2 358 345 B1

(12) **FASCICULE DE BREVET EUROPEEN**

(45) Date de publication et mention de la délivrance du brevet:
**08.04.2015 Bulletin 2015/15**

(21) Numéro de dépôt: **09801480.6**

(22) Date de dépôt: **02.12.2009**

(51) Int Cl.:
***A61K 8/58*** *(2006.01)* ***A61K 8/891*** *(2006.01)*
***A61Q 1/02*** *(2006.01)* ***A61Q 1/06*** *(2006.01)*
***A61Q 1/10*** *(2006.01)* ***A61Q 1/12*** *(2006.01)*
***A61K 8/11*** *(2006.01)* ***A61Q 1/04*** *(2006.01)*

(86) Numéro de dépôt international:
**PCT/FR2009/052381**

(87) Numéro de publication internationale:
**WO 2010/063954 (10.06.2010 Gazette 2010/23)**

(54) **PROCÉDÉ COSMÉTIQUE UTILISANT UNE COMPOSITION COMPRENANT DES RÉSINES DE SILOXANE ET UNE MATIÈRE COLORANTE PULVÉRULENTE**

KOSMETISCHES VERFAHREN MIT EINER SILOXANHARZE UND EINEN PULVERFARBSTOFF ENTHALTENDEN ZUSAMMENSETZUNG

COSMETIC METHOD USING A COMPOSITION CONTAINING SILOXANE RESINS AND A POWDER DYE

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **02.12.2008 FR 0858195**
**09.12.2008 US 120863 P**

(43) Date de publication de la demande:
**24.08.2011 Bulletin 2011/34**

(73) Titulaire: **L'Oréal**
**75008 Paris (FR)**

(72) Inventeur: **DOP, Florence**
**F-91190 Villiers Le Bacle (FR)**

(74) Mandataire: **Tezier Herman, Béatrice et al**
**Cabinet Becker & Associés**
**25, rue Louis le Grand**
**75002 Paris (FR)**

(56) Documents cités:
**US-A1- 2007 166 271**

- **VERONIQUE KOWANDY ET AL: "Bodied MQ-T Propyl Silicone Resins in Color Cosmetic Applications" IP.COM JOURNAL, IP.COM INC., WEST HENRIETTA, NY, US, 4 décembre 2008 (2008-12-04), XP013127272 ISSN: 1533-0001**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 358 345 B1

## Description

L'invention concerne un procédé cosmétique de maquillage et/ou de soin des matières kératiniques comprenant l'application sur lesdites matières kératiniques, notamment la peau, les lèvres, et de préférence la peau, d'une composition cosmétique, comprenant des résines de siloxane et une matière colorante pulvérulente traitée en surface par un agent hydrophobe. L'invention concerne en particulier des compositions de soin ou de maquillage desdites matières kératiniques.
Selon l'invention, on entend désigner par matières kératiniques, la peau du corps et les muqueuses, par exemple le visage ou les lèvres.

Il est connu d'utiliser des produits de maquillage afin d'apporter de la couleur sur la peau. Ces produits contiennent le plus souvent des pigments (en particulier les oxydes de fer et de titane) qui permettent d'obtenir une certaine couvrance. Une fois appliquée sur la peau, après application, ce produit doit avoir de bonnes propriétés de tenue en particulier une bonne tenue de la couleur, de la matité, de l'homogénéité et le maquillage ne doit pas être sensible aux frottements. Après maquillage, le sébum sécrété par la peau au cours du temps modifie les propriétés cosmétiques du rendu de maquillage. En particulier, le sébum a tendance à modifier la couleur du maquillage déposé sur la peau ; cette modification de la couleur va à l'encontre de l'effet esthétique coloré recherché, puisque l'aspect visuel du maquillage ne correspond plus à la couleur initiale de la composition choisie. Cette mauvaise tenue de la couleur du maquillage au cours de la journée nécessite donc de renouveler son application pour conserver un maquillage homogène.
Pour améliorer cette tenue, on utilise des polymères apportant de la tenue au cours de la journée. Ces polymères sont de nature chimique très différente et sont véhiculés soit dans une phase grasse, soit dans une phase aqueuse (par exemple, les résines de silicones, les polyacrylates, les latex, etc.)
Si ces polymères apportent effectivement des propriétés de tenue, en particulier de non transfert, ils ne permettent pas toujours d'avoir une tenue de la couleur tout au long de la journée (problème de tenue à la sécrétion de sébum et d'eau).
Il est donc nécessaire de trouver une solution technique permettant d'obtenir ces propriétés de tenue de la couleur.

Le but de la présente invention est donc de proposer un procédé cosmétique de soin et en particulier de maquillage des matières kératiniques permettant d'obtenir des propriétés cosmétiques satisfaisantes de manière immédiate et au cours du temps, en particulier permettant d'obtenir une tenue dans le temps de la couleur initiale, de la matité et/ou de l'homogénéité du maquillage.

Ce but, ainsi que d'autres, sont atteints par la présente invention qui décrit notamment un procédé cosmétique de maquillage et/ou de soin des matières kératiniques comprenant l'application sur lesdites matières kératiniques, d'une composition comprenant, dans un milieu physiologiquement acceptable :

a) une résine de siloxane comprenant au moins 80% en moles d'unités :

(i) $(R'_3SiO_{1/2})_a$ (ci-après unités "M") et
(ii) $(SiO_{4/2})_b$ (ci-après unités « Q »)

dans lesquelles

- R' représente indépendamment un groupement alkyle ayant de 1 à 8 atomes de carbone, un groupement aryle, un groupement carbinol ou un groupement amino,

avec la condition qu'au moins 95% en moles des groupes R' sont des groupes alkyles,

- a et b ont des valeurs strictement supérieures à 0 ;
- et le rapport a/b est compris entre 0,5 et 1,5,

b) une résine propyl silsesquioxane comprenant au moins 80% en moles d'unités $(R''SiO_{3/2})$ (ci-après unités "T") dans lesquelles R" représente indépendamment un groupement alkyle ayant de 1 à 8 atomes de carbone, un groupement aryle, un groupement carbinol ou un groupement amino, avec la condition qu'au moins 80% en moles des groupes R" sont des groupes propyles,
le rapport pondéral entre les résines a) et b) étant compris entre 1/99 et 99/1, en particulier entre 85/15 et 15/85, les résines a) et b) n'étant pas liées l'une avec l'autre par des liaisons covalentes,
et le nombre d'unités M du mélange final étant strictement inférieur au nombre d'unités (T+Q),
et
c) au moins une matière colorante pulvérulente traitée en surface par un agent hydrophobe choisi parmi un composé de nature fluorée, un acide aminé les acides aminés N-acylés ou leurs sels et un de leurs mélanges.

La présente invention a également pour objet un procédé cosmétique de maquillage et/ou de soin des matières kératiniques comprenant l'application sur lesdites matières kératiniques, d'une composition comprenant, dans un milieu physiologiquement acceptable :

a) une résine de siloxane comprenant au moins 80% en moles d'unités :

(i) $(R'_3SiO_{1/2})_a$ (ci-après unités "M") et
(ii) $(SiO_{4/2})_b$ (ci-après unités « Q »)

dans lesquelles

- R' représente indépendamment un groupement alkyle ayant de 1 à 8 atomes de carbone, un groupement aryle, un groupement carbinol ou un groupement amino,

avec la condition qu'au moins 95% en moles des groupes R' sont des groupes alkyles,

- a et b ont des valeurs strictement supérieures à 0 ;
- et le rapport a/b est compris entre 0,5 et 1,5,

b) une résine propyl silsesquioxane filmogène comprenant au moins 80% en moles d'unités $(R''SiO_{3/2})$ (ci-après unités "T") dans lesquelles R" représente indépendamment un groupement alkyle ayant de 1 à 8 atomes de carbone, un groupement aryle, un groupement carbinol ou un groupement amino, avec la condition qu'au moins 40% en moles des groupes R" sont des groupes propyles,
le rapport pondéral entre les résines a) et b) étant compris entre 1/99 et 99/1, en particulier entre 85/15 et 15/85, les résines a) et b) n'étant pas liées l'une avec l'autre par des liaisons covalentes,
et le nombre d'unités M du mélange final étant strictement inférieur au nombre d'unités (T+Q),
et
c) au moins une matière colorante pulvérulente traitée en surface par un agent hydrophobe choisi parmi un composé de nature fluorée, un acide aminé, les acides aminés N-acylés ou leurs sels et un de leurs mélanges.

Le procédé selon l'invention permet d'obtenir de manière avantageuse des dépôts ayant une bonne tenue de la couleur tout le long de la journée, et/ou une bonne homogénéité du maquillage.
La composition selon l'invention peut se présenter sous diverses formes, notamment sous forme de poudres (libres ou compactes), de dispersion anhydre, émulsion eau/huile ou eau/cire, huile/eau, multiples ou cire/eau, ou encore sous forme de gel.
La composition selon l'invention est en particulier destinée au maquillage et/ou au soin de la peau, des lèvres, des ongles, en particulier de la peau.

## RESINES DE SILOXANE

La résine de siloxane a), appelée « résine MQ » par la suite, comprend de préférence des groupements silanols (-SiOH) résiduels. Dans ce cas, de préférence la quantité de groupements -OH est comprise entre 2 et 10% en poids de la résine MQ, de préférence entre 2 et 5% en poids de la résine MQ.
De préférence, les groupes R' de la résine MQ sont des groupes Méthyle.
La résine b), appelée ci-après « résine T propyle », comprend de préférence des groupes résiduels silanols (-SiOH) et/ou des groupes alcoxy. Dans ce cas, de préférence la quantité de groupements -OH est comprise entre 2 et 10% en poids de la résine T propyle, et/ou la quantité de groupes alcoxy est inférieure ou égale à 20% en poids de la résine T propyle. De préférence, la quantité de groupements-OH est comprise entre 6 et 8% en poids de la résine T propyle, et/ou la quantité de groupes alcoxy est inférieure ou égale à 10% en poids de la résine T propyle.
La résine T propyle selon l'invention est telle qu'au moins 40% en moles des groupes R" sont des groupes propyles, de préférence au moins 50% en moles, et plus préférentiellement au moins 90% en moles.
Par liaison covalente, on entend une liaison chimique entre au moins 2 atomes (carbone, silicium, oxygène, etc..) dans laquelle chacun des atomes liés met en commun un électron d'une de ses couches externes afin de former un doublet d'éléctrons liant les deux atomes.
La résine MQ selon l'invention comprend au moins 80% en moles d'unités :

(i) $(R'_3SiO_{1/2})_a$ (ci-après unités "M") et
(ii) $(SiO_{4/2})_b$ (ci-après unités « Q »),

dans lesquelles

- R' représente indépendamment un groupement alkyle ayant de 1 à 8 atomes de carbone, un groupement aryle, un groupement carbinol ou un groupement amino, avec la condition qu'au moins 95% en moles des groupes R' sont des groupes alkyles,
- a et b ont des valeurs strictement supérieures à 0 ;
- et le rapport a/b est compris entre 0,5 et 1,5.

Le radical R' de la résine MQ représente indépendamment un groupement alkyle ayant de 1 à 8 atomes de carbone, un groupement aryle, un groupement carbinol ou un groupement amino.
Les groupements alkyle peuvent notamment être choisis parmi les groupements méthyle, éthyle, propyle, butyle, pentyle, hexyle et octyle. De préférence, le groupe alkyle est un groupe méthyle ou propyle.
Les groupements aryle peuvent être choisis parmi les groupements phényle, naphthyle, benzyle, tolyle, xylyle, xényle, méthylphényle, 2-phényléthyle, 2-phényl-2-méthyléthyle, chlorophényle, bromophényle et fluorophényle, le groupement aryle étant préférentiellement un groupement phényle.
Dans la présente invention, par « groupement carbinol », on entend tout groupement contenant au moins un radical hydroxyle lié à un carbone (COH). Les groupements carbinol peuvent ainsi contenir plus d'un radical COH, tel que par exemple

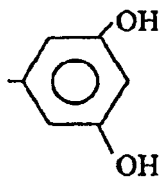

Si le groupement carbinol est exempt de groupements aryle, il comporte au moins 3 atomes de carbone. Si le groupement carbinol comprend au moins un groupement aryle, il comporte au moins 6 atomes de carbone.
Comme exemples de groupement carbinol exempt de groupements aryle comportant au moins 3 atomes de carbone, on peut citer les groupements de formule $R^1OH$ dans laquelle $R^1$ représente un radical hydrocarboné bivalent comportant au moins 3 atomes de carbone ou un radical hydrocarbonoxy bivalent comportant au moins 3 atomes de carbone. Comme exemples de groupement $R^1$, on peut citer des radicaux alkylène tels que $-(CH_2)_x-$, la valeur de x étant comprise entre 3 et 10, $-CH_2CH(CH_3)-$, $-CH_2CH(CH_3)CH_2-$, $-CH_2CH_2CH(CH_2CH_3)CH_2CH_2CH_2-$ et-$OCH(CH_3)(CH_2)_x-$, la valeur de x étant comprise entre 1 et 10.
Comme exemples de groupement carbinol comportant des groupements aryle présentant au moins 6 atomes de carbone, on peut citer les groupements de formule $R^2OH$ dans laquelle $R^2$ représente un radical arylène tel que $-(CH_2)_xC_6H_4-$, x ayant une valeur comprise entre 0 et 10,$-CH_2CH(CH_3)(CH_2)_xC_6H_4-$, x ayant une valeur comprise entre 0 et 10, $-(CH_2)xC_6H_4(CH_2)_x-$, x ayant une valeur comprise entre 1 et 10. Les groupements carbinol comportant des groupements aryle comportent généralement de 6 à 14 atomes.
Par groupement amino selon l'invention, on entend notamment des groupements de formule $-R^3NH_2$ ou $-R^3NHR^4NNH_2$, $R^3$ représentant un radical hydrocarboné bivalent ayant au moins 2 atomes de carbone et $R^4$ représentant un radical hydrocarboné bivalent ayant au moins 2 atomes de carbone. Le groupement $R^3$ représente généralement un radical alkylène ayant de 2 à 20 atomes de carbone. Comme exemples de groupement $R^3$, on peut citer les groupements éthylène, propylène, $-CH_2CHCH_3-$, butylène, $-CH_2CH(CH_3)CH_2-$, pentaméthylène, hexaméthylène, 3-éthyl-hexaméthylène, octaméthylène et décaméthylène.
Le groupement $R^4$ représente généralement un radical alkylène ayant de 2 à 20 atomes de carbone. Comme exemples de groupement $R^4$, on peut citer les groupements éthylène, propylène, $-CH_2CHCH_3-$, butylène, $-CH_2CH(CH_3)CH_2-$, pentaméthylène, hexaméthylène, 3-éthyl-hexaméthylène, octaméthylène et décaméthylène.
Les groupements amino sont généralement $-CH_2CH_2CH_2NH_2$ et $-CH_2(CH_3)CHCH_2(H)NCH_3$,$-CH_2CH_2NHCH_2CH_2NH_2$, $-CH_2CH_2NH_2$, $-CH_2CH_2NHCH_3$, $-CH_2CH_2CH_2CH_2NH_2$, $-(CH_2CH_2NH)_3H$ et $-CH_2CH_2NHCH_2CH_2NHC_4H_9$.
Des résines MQ convenant à une utilisation en tant que composant a), ainsi que leurs procédés de fabrication, sont connus dans l'état de la technique. Le brevet US 2 814 601, appartenant à Currie et al., daté du 26 novembre 1957 décrit un procédé de fabrication de résines MQ par transformation d'un silicate hydrosoluble en un monomère d'acide silicique ou un oligomère d'acide silicique en utilisant un acide. Une fois la polymérisation adéquate réalisée, des extrémités triméthylchlorosilane sont introduites pour obtenir la résine MQ. Un autre procédé de préparation de résines MQ est décrit dans le brevet US 2 857 356 appartenant à Goodwin, daté du 21 octobre 1958. Goodwin décrit un procédé de fabrication d'une résine MQ par cohydrolyse d'un mélange d'un silicate d'alkyle et d'un organopolysiloxane trialkylsilane hydrolysable avec de l'eau.
Les résines MQ convenant en tant que composant a) dans la présente invention peuvent contenir des unités D et T, à condition d'au moins 80 % en moles, voire 90 % en moles des unités de siloxane totales soient des unités M et Q. Les résines MQ peuvent également contenir des groupements hydroxy résiduels comme cela est mentionné ci-dessus. Les résines MQ peuvent également comporter des extrémités supplémentaires, des groupements hydroxy résiduels étant pour cela mis en réaction avec des groupements M appropriés.

La résine T propyle b) selon l'invention comprend au moins 80% en moles d'unités ($R''SiO_{3/2}$) dans lesquelles R" représente indépendamment un groupement alkyle ayant de 1 à 8 atomes de carbone, un groupement aryle, un groupement carbinol ou un groupement amino, avec la condition qu'au moins 40% en moles des groupes R" sont des groupes propyles.
De préférence, la résine T propyle selon l'invention est telle qu'au moins 50% en moles des groupes R" sont des groupes propyles, de préférence au moins 90% en moles.

De préférence, la résine T propyle b) est filmogène. Par « résine filmogène », on entend une résine apte à former à elle seule ou en présence d'un agent auxiliaire de filmification, un film macroscopiquement continu et adhérent sur les matières kératiniques, et de préférence un film cohésif, et mieux encore un film dont la cohésion et les propriétés mécaniques sont telles que ledit film peut être isolable et manipulable isolément, par exemple lorsque ledit film est réalisé par coulage sur une surface antiadhérente comme une surface téflonnée ou siliconnée.
La définition du radical R" est la même que celle du radical R'. Les définitions mentionnées ci-dessus applicables à R' sont donc applicables à R".

La résine T propyle b) selon l'invention est une résine de silsesquioxane. Les résines de silsesquioxane sont bien connues dans l'état de la technique et sont généralement obtenues par hydrolyse d'un organosilane comportant trois groupements hydrolysables, tels que des groupements halogène ou alcoxy, présents dans la molécule. La résine T propyle b) peut ainsi être obtenue par hydrolyse de propyltriméthoxysilane, propyltriéthoxysilane, propyltripropoxysilane, ou par cohydrolyse des propylalcoxysilanes susmentionnés avec divers alcoxysilanes. Comme exemples de ces alcoxysilanes, on peut citer le méthyltriméthoxysilane, le méthyltriéthoxysilane, le méthyltriisopropoxysilane, le diméthyldiméthoxysilane et le phényltriméthoxysilane. Le propyltrichlorosilane peut également être hydrolysé seul, ou en présence d'alcool. Dans ce cas, la cohydrolyse peut être réalisée en ajoutant du méthyltrichlorosilane, du diméthyldichlorosilane, du phényltrichlorosilane ou des chlorosilanes similaires et du méthyltriméthoxysilane, du méthyltriéthoxysilane, du méthyltriisopropoxysilane ou des méthylalcoxysilanes similaires. Comme alcools convenant en ce but, on peut citer le méthanol, l'éthanol, l'alcool n-propylique, l'alcool isopropylique, le butanol, le méthoxy éthanol, l'éthoxy éthanol ou des alcools similaires. Comme exemples de solvants de type hydrocarbures pouvant être utilisés simultanément, on peut citer le toluène, le xylène ou des hydrocarbures aromatiques similaires, l'hexane, l'heptane, l'isooctane ou des hydrocarbures saturés linéaires ou en partie ramifiés similaires ; ainsi que le cyclohexane ou des hydrocarbures aliphatiques similaires.

Les résines de T propyle b) selon l'invention peuvent contenir des unités M, D et Q, à condition qu'au moins 80 % en moles, voire 90 % en moles des unités de siloxane totales soient des unités T. Les résines de propyle T peuvent également contenir des groupements hydroxy et/ou alcoxy résiduels, comme cela est mentionné précédemment.

La composition selon l'invention comprend également un milieu physiologiquement acceptable. Par milieu physiologiquement acceptable, on entend un milieu compatible avec la peau, les muqueuses et les phanères.

Ce milieu peut comprendre au moins un solvant volatil siliconé ou organique, ce solvant étant de préférence compatible avec les résines a/ et b/ et compatible avec une utilisation cosmétique.
Comme solvant volatil siliconé, on peut citer les polysiloxanes cycliques, les polysiloxanes linéaires et leurs mélanges. Comme polysiloxanes volatiles linéaires, on peut citer l'hexamethyldisiloxane, l'octamethyltrisiloxane, le decamethyltetrasiloxane, le tetradecamethylhexasiloxane et l'hexadecamethylheptasiloxane. Comme polysiloxanes volatiles cycliques, on peut citer l'hexamethylcyclotrisiloxane, l'octamethylcylotetrasiloxane, le decamethylcyclopentasiloxane et le dodecamethylcyclohexasiloxane. Le solvant organique peut également être un alcool comme, l'éthanol, l'isopropanol, le butanol, le n-propanol ; une cétone comme l'acétone, la méthyléthylcétone, ou la méthyl isobutyl cétone ; un hydrocarbure aliphatique comme l'heptane, l'hexane, l'octane ou l'isododécane ; un éther de glycol comme le propylène glycol méthyl éther, le dipropylène glycol méthyl éther, le propylène glycol n-butyl éther, le propylène glycol n-propyl éther, l'éthylène glycol n-butyl éther.

Le mélange de résines a/ et b/ peut être obtenu à partir de chacune des résines en solution dans un solvant. En général, en fin de synthèse de la résine MQ selon l'invention, on obtient directement cette résine en solution dans du xylène.
De même, en fin de synthèse de la résine T propyle b) selon l'invention, on obtient cette résine en solution dans du toluène.

Chacune de ces résines en solution est mélangée à l'autre selon le protocole suivant :

1) Mélange sous agitation des deux solutions de résines, puis chauffage, notamment dans un réacteur ou dans un autoclave (pour pouvoir opérer éventuellement sous pression ou, au contraire, en établissant un vide partiel), voire

dans un extrudeur équipé ou non d'un système de « dévolatilisation » des solvants, sous les conditions spécifiques suivantes :

- on chauffe de façon homogène : la température de chauffage doit être supérieure à 90°C, et inférieure ou égale à 250°C, et de préférence comprise entre 90°C et 190°C.
  Soit on peut chauffer à une seule température, comprise entre 90°C et 250°C, Soit on peut chauffer en faisant des paliers de températures successifs : d'abord entre 90°C et T1°C,
  T1°C étant une température de valeur intermédiaire entre 90°C et T2°C qui est la température finale,
  pendant une durée comprise entre 10 minutes à 2 heures, puis entre T1°C et T2°C, pendant une durée comprise entre 10 minutes à 4 heures,
  la température T2°C correspondant à la température maximale choisie pour la réaction.
  Cette valeur de T2°C est variable selon le mode opératoire choisi et le type de réacteur choisi : réacteur classique ou autoclave ou extrudeur,
  mais T2°C reste inférieure ou égale à 250°C. On peut également faire des paliers de températures intermédiaires entre T1°C et T2°C ;
- la durée de chauffage est d'au moins une heure en réacteur ou en autoclave et au moins 10 minutes en extrudeur, de préférence entre 1h et 5h en réacteur ou en autoclave, et de préférence entre 10 minutes et 2 heures en extrudeur ;
- à la condition que ces traitements thermiques se fassent sans présence d'un catalyseur de condensation chimique entre les 2 résines MQ et T propyle. Un tel catalyseur est notamment une base minérale, en particulier NaOH, KOH ou ammoniaque.

2) De manière optionnelle, après, ou même pendant, l'étape 1) de traitement thermique des 2 résines dans la plage de température indiquée, on effectue une distillation partielle ou totale des solvants aromatiques, en les remplaçant par un solvant volatil cosmétiquement acceptable. Un tel solvant volatil peut notamment être une silicone volatile ou non, de préférence la décaméthylcyclopentasiloxane, ou un solvant organique volatil ou non, de préférence isododécane.

3) De manière optionnelle encore, après mélange des 2 solutions initiales de chaque résine dans un solvant volatil, on traite le mélange des solutions dans un malaxeur à vis ou double vis de type extrudeur à « dévolatilisation », dans une plage de températures entre 90° et 250°C, permettant de volatiliser les solvants volatils en établissant un vide partiel, en travaillant en continu, puis de passer dans une filière le mélange fondu et sans solvant. Le mélange fondu est alors refroidi en sortie de filière et découpé en granulés solides ou sous forme de poudre. Dans ce cas le mélange est directement sous forme solide et sera remis en solution dans les solvants choisis au moment de la formulation.

Aussi la présente invention a également pour objet un procédé utilisant une composition telle que décrite ci-dessus comprenant, dans un milieu physiologiquement acceptable :

1) le mélange entre une résine de siloxane a) et une résine propyl silsesquioxane b), le mélange étant tel que décrit ci-dessus, et
2) au moins une matière colorante pulvérulente traitée en surface par un agent hydrophobe choisi parmi un composé de nature fluorée, un acide gras, un acide aminé et un de leurs mélanges,
les résines de siloxane a) et propyl silesquioxane b) étant formulées dans la composition via un mélange susceptible d'être obtenu selon le procédé suivant :

- Mélange, de préférence sous agitation, d'une solution de résine de siloxane avec une solution de résine propyl silsesquoxane, le solvant présent dans chacune des solutions étant de préférence volatil, puis
- Chauffage, notamment dans un réacteur ou dans un autoclave ou dans un extrudeur, sous les conditions spécifiques suivantes :

  - on chauffe de façon homogène à une température supérieure à 90°C, et inférieure ou égale à 250°C, de préférence comprise entre 90°C et 190°C ; le chauffage peut se faire à une seule température, ou à des paliers de température, comme indiqué ci-dessus ;
  - la durée de chauffage est d'au moins 1 heure en réacteur ou en autoclave et au moins 10 minutes en extrudeur, de préférence entre 1h et 5h en réacteur ou en autoclave et de préférence entre 10 minutes et 2 heures en extrudeur ;
  - à la condition que ces traitements thermiques se fassent sans présence d'un catalyseur de condensation

chimique entre les 2 résines MQ et T propyle. Un tel catalyseur est notamment une base minérale, en particulier NaOH, KOH ou ammoniaque.

Ce procédé peut comprendre, après ou même pendant l'étape de mélange, une étape supplémentaire de distillation partielle ou totale des solvants aromatiques, en les remplaçant par un solvant volatil cosmétiquement acceptable.

Dans le cas où on utilise un extrudeur, ce procédé peut comprendre, après ou même pendant l'étape de mélange, une étape supplémentaire de distillation partielle ou totale des solvants aromatiques, en sortant directement le mélange à l'état solide.

L'étape finale du traitement thermique, ou même le traitement thermique lui-même peuvent être réalisés dans un malaxeur prévu pour l'agitation de milieux très visqueux tels que :

- un malaxeur de type « bras en Z » (« Zigma blender »), en particulier un malaxeur Brabender,
- un malaxeur à vis type extrudeur, en particulier un extrudeur mono-vis ou un extrudeur double vis (avec ou non étape de « dévolatilisation » des solvants volatils de départ) ou dans un knider qui permet de dévolatiliser en établissant un film mince sur les parois.

Les mélanges de résines 1) utilisables selon l'invention sont notamment ceux décrits dans la demande WO 2005/075567, en particulier ceux décrits dans les tableaux 1 et 3 de ladite demande. On peut également utiliser les mélanges de résines 1) décrits dans la demande WO2007/145765, en particulier ceux décrits dans les exemples 12 à 14 de cette demande, dans lesquels le rapport pondéral entre les résines a) et b) sont respectivement de 50/50, 60/40 et 71/29 (70/30)..

Selon un mode particulier, on utilise le mélange de résines 1) décrit à l'exemple 1-f dans le tableau 1 des exemples, dans lequel le rapport pondéral entre les résines a) et b) est de 50/50.

Selon un mode particulier, on utilise le mélange de résines 1) décrit à l'exemple 22 de ladite demande WO2005/075567, dans lequel le rapport pondéral entre les résines a) et b) est de 85/15.

Selon un mode particulier, on utilise le mélange de résines 1) décrit à l'exemple 13 de ladite demande WO2007/145765, dans lequel le rapport pondéral entre les résines a) et b) est de 60/40.

Le rapport pondéral entre les résines a) et b) (i.e. a/b) est compris entre 1/99 et 99/1, alternativement entre 85/15 et 15/85.

La composition selon l'invention comprend une quantité de résines a) et b) de siloxane, en poids de matière active (matière sèche), allant de 0,5 à 60% en poids, par rapport au poids total de la composition, de préférence de 3 à 60 % en poids, et mieux de 4 à 60% en poids par rapport au poids total de ladite composition.
Selon un mode particulier, la quantité de résines a) et b) de siloxane, en poids de matière active (matière sèche) varie avantageusement de 3 à 60 % en poids, et mieux de 6 à 60% en poids par rapport au poids total de ladite composition. Ces teneurs sont notamment adaptées aux compositions sous forme anhydre et en particulier aux compositions sous forme de stick, telles que les rouges à lèvres. Selon un autre mode particulier, la quantité de résines a) et b) de siloxane, en poids de matière active (matière sèche) varie avantageusement de 3 à 30 % en poids, et mieux de 4 à 20% en poids par rapport au poids total de ladite composition. Ces teneurs sont notamment adaptées aux compositions sous forme d'émulsions, et en particulier aux compositions sous forme d'émulsions E/H, telles que les fonds de teint liquides.

## MATIERES COLORANTES PULVERULENTES TRAITEES EN SURFACE PAR UN

## AGENT HYDROPHOBE

La matière colorante pulvérulente traitée en surface par un agent hydrophobe peut être choisie parmi les pigments et/ou les nacres.

Par pigments, il faut comprendre des particules de toute forme, blanches ou colorées, minérales ou organiques, insolubles dans le milieu physiologique, destinées à colorer la composition.

Par nacres, il faut comprendre des particules de toute forme irisées, notamment produites par certains mollusques dans leur coquille ou bien synthétisées.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.
Parmi les pigments organiques, on peut citer le noir de carbone, les pigments de type D & C, et les laques à base de carmin de cochenille, de baryum, strontium, calcium, aluminium.

On peut également citer les pigments à effet, tels les particules comportant un substrat organique ou minéral,

naturel ou synthétique, par exemple le verre, les résines acrylique, le polyester, le polyuréthane, le polyéthylène téréphtalate, les céramiques ou les alumines, ledit substrat étant recouvert ou non de substances métalliques comme l'aluminium, l'or, l'argent, le platine, le cuivre, le bronze, ou d'oxydes métalliques comme le dioxyde de titane, l'oxyde de fer, l'oxyde de chrome et leurs mélanges.

Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth. On peut également utiliser les pigments interférentiels, notamment à cristaux liquides ou multicouches.

Les matières colorantes, en particulier les pigments traités avec un agent hydrophobe, peuvent être présents dans la composition en une teneur allant de 0,1 % à 50 % en poids, par rapport au poids total de la composition, de préférence allant de 0,5 % à 30 % en poids, et préférentiellement allant de 1 % à 20 % en poids. Les matières colorantes pulvérulentes traitées en surface avec un agent hydrophobe peuvent constituer de 10 à 100% en poids de l'ensemble des matières colorantes.

A titre illustratif des nacres pouvant être mises en oeuvre dans le cadre de la présente invention, on peut notamment citer les nacres de couleur or, notamment commercialisées par la société ENGELHARD sous le nom de Brillant gold 212G (Timica), Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona) et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica) ; les nacres à reflet rouge notamment commercialisées par la société MERCK sous la dénomination Sienna fine (17386) (Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres roses notamment commercialisées par la société ENGELHARD sous la dénomination Tan opale G005 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), les nacres blanches à reflet argenté notamment commercialisées par la société MERCK sous la dénomination Xirona Silver et les nacres orangées rosées vert doré notamment commercialisées par la société MERCK sous la dénomination Indian summer (Xirona) et leurs mélanges.

Les nacres traitées en surface par au moins un agent hydrophobe peuvent être présentes dans la composition en une teneur allant de 1 à 50% en poids, par rapport au poids total de la composition, mieux de 5 à 30 % en poids.

Les matières colorantes pulvérulentes telles que décrites précédemment sont traitées en surface, totalement ou partiellement, avec un agent hydrophobe; un composé de nature fluorée, ou acide aminé ou acide aminé N-acylé ou son sel ou un de leurs mélanges.

La présente invention a ainsi également pour objet une composition, susceptible d'être mise en oeuvre dans le procédé selon l'invention, comprenant, dans un milieu physiologiquement acceptable, les résines de siloxane a) et b) telles que définies ci-dessus et au moins une matière colorante pulvérulente traitée en surface par un agent hydrophobe choisi parmi un composé de nature fluorée, un acide aminé ou acide aminé N-acylé ou son sel et un de leurs mélanges.

Par composé de nature fluoré, on entend un composé comprenant au moins un atome de fluor.

A titre d'exemple, l'agent de traitement hydrophobe peut être choisi parmi les perfluoroalkyl phosphates, les polyoxydes d'hexafluoropropylène ; les perfluoropolyéthers ; les acides aminés ; les acides aminés N-acylés ou leurs sels et leurs mélanges. De préférence, l'agent de traitement hydrophobe est choisi parmi les perfluoroalkyl phosphates, les polyoxydes d'hexafluoropropylène, les perfluoropolyéthers, les acides aminés, les acides aminés N-acylés ou leurs sels, et leurs mélanges.

Les matières colorantes pulvérulentes traitées en surface peuvent être préparées selon des techniques de traitement de surface de nature chimique, électronique, mécano-chimique ou mécanique bien connues de l'homme de l'art. On peut également utiliser des produits commerciaux.

L'agent de surface peut être absorbé ou adsorbé sur les matières colorantes pulvérulentes par évaporation de solvant, réaction chimique et création d'une liaison covalente.

Selon une variante, le traitement de surface consiste en un enrobage des matières colorantes pulvérulentes.

L'enrobage peut représenter de 1 à 300% en poids du poids des matières colorantes pulvérulentes non traitées, par exemple de 5 à 200%, en particulier de 10 à 100% en poids du poids de matières colorantes pulvérulentes non traitées. L'enrobage peut représente de 0,1 à 10% en poids, et en particulier de 1 à 5% en poids du poids total de la matière

colorante pulvérulente enrobée.

L'enrobage peut être réalisé par exemple par adsorption d'un agent de surface liquide à la surface des matières colorantes pulvérulentes par simple mélange sous agitation matières colorantes pulvérulentes et dudit agent de surface, éventuellement à chaud, préalablement à l'incorporation des particules dans les autres ingrédients de la composition de maquillage ou de soin.

L'enrobage peut être réalisé par exemple par réaction chimique d'un agent de surface avec la surface des matières colorantes pulvérulentes et création d'une liaison covalente entre l'agent de surface et les matières colorantes pulvérulentes. Cette méthode est notamment décrite dans le brevet US 4 578 266.

Le traitement de surface chimique peut consister à diluer l'agent de surface dans un solvant volatile, à disperser les matières colorantes pulvérulentes dans ce mélange, puis à évaporer lentement le solvant volatile, de manière à ce que l'agent de surface se dépose à la surface des matières colorantes pulvérulentes.

***Agent de surface fluoré***

Les particules solides peuvent être traitées en surface totalement ou partiellement avec un composé de nature fluorée.

Les agents de surface fluorés peuvent être choisis parmi les phosphates de perfluoroalkyle, les perfluoropolyéthers, les polytétrafluoropolyéthylène (PTFE) et les perfluoroalcanes.

Les perfluoropolyéthers sont notamment décrits dans la demande de brevet EP-A-486135, et vendus sous les dénominations commerciales FOMBLIN par la société MONTEFLUOS.

Des phosphates de perfluoroalkyle sont en particulier décrits dans la demande JP H05-86984. Les phosphate-diéthanol amine de perfluoroalkyle commercialisés par Asahi Glass sous la référence AsahiGuard AG530 peuvent être utilisés.

Les perfluoroalcanes peuvent être des perfluoroalcanes linéaires ou cycliques. Parmi les perfluoroalcanes linéaires, on peut citer la série des alcanes linéaires, tels que le perfluorooctane, le perfluorononane ou le perfluorodécane. Parmi les perfluoroalcanes cycliques, on peut citer les perfluorocycloalcanes, les perfluoro(alkylcycloalcanes), les perfluoropolycycloalcanes, les hydrocarbures perfluorés aromatiques (les perfluoroarènes). Parmi les perfluoroalcanes, on peut aussi citer les composés organo perfluorés hydrocarbonés comportant au moins un hétéroatome.

Parmi les perfluorocycloalcanes et les perfluoro(alkylcycloalcanes), on peut citer la perfluorodécaline vendue sous la dénomination de "FLUTEC PP5 GMP" par la Société RHODIA, la perfluoro(méthyldécaline), les perfluoro(C3-C5 alkyl-cyclohexanes) tels que le perfluoro(butylcyclohexane).

Parmi les perfluoropolycycloalcanes, on peut citer les dérivés de bicyclo [3.3.1] nonane tel que le perfluorotriméthylbicyclo [3.3.1] nonane, les dérivés de l'adamantane, tels que le perfluorodiméthyladamantane, et les dérivés perfluorés de phénanthrène hydrogéné, tels que le tétracosafluoro-tétradécahydrophénanthrène.

Parmi les perfluoroarènes, on peut citer les dérivés perfluorés du naphtalène comme le perfluoronaphtalène et le perfluorométhyl-1-napthtalène.

A titre d'exemple de références commerciales de pigments et de charges traités avec un composé fluoré, on peut citer :

- L'oxyde de fer jaune/phosphate de perfluoroalkyle, vendu en particulier sous la référence PF 5 Yellow 601 par la société Daito Kasei,
- L'oxyde de fer rouge/phosphate de perfluoroalkyle, vendu en particulier sous la référence PF 5 Red R 516L par la société Daito Kasei,
- L'oxyde de fer noir/phosphate de perfluoroalkyle, vendu en particulier sous la référence PF 5 Black BL 100 par la société Daito Kasei,
- Le dioxyde de titane/phosphate de perfluoroalkyle, vendu en particulier sous la référence PF 5 TiO2 CR 50 par la société Daito Kasei,
- L'oxyde de fer jaune/perfluoropolymethylisopropylether, vendu en particulier sous la référence Iron oxyde yellow BF-25-3 par la société Toshiki,
- Le DC Red 7/perfluoropolymethylisopropylether, vendu en particulier sous la référence D&C Red 7 FHC par la société Cardre Inc.,
- Le DC Red 6/PTFE, vendu en particulier sous la référence T 9506 par la société Warner - Jenkinson,

***Agent de traitement acide aminé***

L'agent de traitement hydrophobe peut être choisi parmi les acides aminés ; les acides aminés N-acylés ou leurs sels ; et leurs mélanges.

Les acides aminés N-acylés peuvent comprendre un groupe acyle ayant de 8 à 22 atomes de carbone, comme par

exemple un groupe 2-éthyl-hexanoyle, caproyle, lauroyle, myristoyle, palmitoyle, stéaroyle, cocoyle. Les sels de ces composés peuvent être les sels d'aluminium, de magnésium, de calcium, de zirconium, de zinc, de sodium, de potassium. L'acide aminé peut être par exemple la lysine, l'acide glutamique, l'alanine.

A titre d'exemple de pigments, on peut citer ceux vendus sous la référence commerciale NAI-TAO-77891, NAI - C33-8073 - 10, NAI - C33-8075, NAI - C47-051 - 10, NAI-C33-115, NAI - C33-134, NAI - C33-8001-10, NAI - C33-7001-10, NAI - C33-9001-10 de la Société MIYOSHI KASEI.

**AUTRES COMPOSANTS**

La composition selon l'invention peut comprendre un ou plusieurs autres composants, et en particulier des composants choisis parmi les huiles, les tensioactifs non ioniques, les élastomères siliconés amphiphiles, les matières colorantes pulvérulentes non traitées en surface par un agent hydrophobe, les composés pâteux d'origine non animale, les agents rhéologiques épaississants ou gélifiants de phase grasse (en particulier à l'exception des dimethicone crosspolymers), les cires (en particulier à l'exception de la cire de candelilla, l'ozokérite et les cires siliconées), les agents gélifiants hydrophiles, les charges, les matières colorantes pulvérulentes non traitées en surface par un agent hydrophobe, les polymères filmogènes, les tensioactifs ioniques (en particulier à l'exception du lauryl éther sulfate), les fibres, et leurs mélanges

En particulier, la composition selon l'invention peut comprendre un ou plusieurs autres composants choisis parmi les huiles, les tensioactifs non ioniques, les élastomères siliconés amphiphiles, les matières colorantes pulvérulentes non traitées en surface par un agent hydrophobe, les agents rhéologiques épaississants ou gélifiants de phase grasse (en particulier à l'exception des dimethicone crosspolymers), les agents gélifiants hydrophiles, les charges, les polymères filmogènes notamment lipophiles, et leurs mélanges.

**Elastomères de silicone**

Les compositions selon l'invention peuvent en outre comprendre un élastomère de silicone amphiphile, de préférence choisi parmi les élastomères siliconés polyoxyalkylénés et polyglycérolés.

Comme élastomères de silicone polyoxyalkylénés, on peut citer ceux décrits dans les brevets US5236986, US5412004, US5837793, US5811487.

Comme élastomères de silicone polyoxyalkylénés, on peut utiliser :

- ceux de nom INCI PEG-10 Dimethicone/Vinyl dimethicone crosspolymer : comme ceux commercialisées sous les dénominations "KSG-21 ", "KSG-20", par Shin Etsu ;
- ceux de nom INCI Lauryl PEG-15 Dimethicone/Vinyldimethicone Crosspolymer : comme ceux commercialisés sous les dénominations "KSG-30" et "KSG-31", KSG-32" (dans l'isododécane), "KSG-33" (dans la trioctanoine), "KSG-210", "KSG-310" (dans une huile minérale), "KSG-320" (dans l'isododécane), "KSG-330", "KSG-340" par la société Shin Etsu.

Comme élastomères de silicone polyglycérolés, on peut utiliser :

- ceux de nom INCI Dimethicone (and) Dimethicone/Polyglycerin-3 crosspolymer : comme ceux commercialisés sous les dénominations "KSG-710" par Shin Etsu ;
- ceux de nom INCI Lauryl Dimethicone/Polyglycerin-3 crosspolymer: comme ceux commercialisés sous les dénominations "KSG-840" (dans du squalène) par la société Shin Etsu.

Ces élastomères particuliers, lorsqu'ils sont en association avec les résines selon l'invention, peuvent permettre d'améliorer les propriétés de non transfert et de confort (souplesse) des compositions les comprenant.

**Huiles**

La composition selon l'invention peut comprendre en outre au moins une huile.

L'huile peut être choisie parmi les huiles hydrocarbonées, les huiles siliconées, les huiles fluorées. Ces huiles peuvent être d'origine animale, végétale, minérale ou synthétique.

L'huile peut être choisie parmi les huiles volatiles, les huiles non volatiles, et leurs mélanges.

Par huile hydrocarbonée, on entend une huile formée essentiellement, voire constituée, d'atomes de carbone et d'hydrogène, et éventuellement d'atomes d'oxygène, d'azote, et ne contenant pas d'atome de silicium ou de fluor; elle peut contenir des groupes ester, éther, amine, amide.

Par huile siliconée, on entend une huile contenant au moins un atome de silicium, et notamment contenant

des groupes Si-O.

Par huile fluorée, on entend une huile contenant au moins un atome de fluor.

La composition selon l'invention peut comprendre au moins une huile volatile.

Par "huile volatile", on entend une huile (ou milieu non aqueux) susceptible de s'évaporer au contact de la peau en moins d'une heure, à température ambiante et pression atmosphérique. L'huile volatile est une huile cosmétique volatile, liquide à température ambiante, ayant notamment une pression de vapeur non nulle, à température ambiante et pression atmosphérique, en particulier ayant une pression de vapeur allant de 0,13 Pa à 40 000 Pa (10-3 à 300 mm de Hg), et de préférence allant de 1,3 Pa à 13 000 Pa (0,01 à 100 mm de Hg), et préférentiellement allant de 1,3 Pa à 1300 Pa (0,01 à 10 mm de Hg).

En outre, l'huile volatile a généralement un point d'ébullition, mesuré à pression atmosphérique, allant de 150 °C à 260 °C, et de préférence allant de 170 °C à 250 °C.

La composition selon l'invention peut comprendre une huile volatile hydrocarbonée notamment choisie parmi les huiles hydrocarbonées ayant un point éclair allant de 40 °C à 102 °C, de préférence allant de 40 °C à 55 °C, et préférentiellement allant de 40 °C à 50 °C.

Comme huile volatile hydrocarbonée, on peut citer les huiles volatiles hydrocarbonées ayant de 8 à 16 atomes de carbone et leurs mélanges, et notamment les alcanes ramifiés en C8-C16 comme les iso-alcanes (appelées aussi isoparaffines) en C8-C16, l'isododécane, l'isodécane, l'isohexadécane et par exemple les huiles vendues sous les noms commerciaux d'Isopars ou de Permetyls, les esters ramifiés en C8-C16 comme le néopentanoate d'iso-hexyle, et leurs mélanges. De préférence, l'huile volatile hydrocarbonée est choisie parmi les huiles volatiles hydrocarbonées ayant de 8 à 16 atomes de carbone et leurs mélanges, en particulier parmi l'isododécane, l'isodécane, l'isohexadécane, et est notamment l'isododécane.

Pour de bonnes propriétés de tenue de la couleur tout en conservant un dépôt mat et confortable pour les applications teint, on préférera les solvants volatils hydrocarbonés de 8 à 16 atomes de carbone, en particulier de 9 à 13 atomes de carbone. Comme solvant volatil hydrocarboné en C8 à C16, on peut notamment citer les alcanes linéaires ou ramifiés, en particulier ramifiés, comme les iso-alcanes (appelées aussi isoparaffines) en C8-C16, l'isododécane, l'isodécane, l'isohexadécane, et par exemple les huiles vendues sous les noms commerciaux d'Isopars ou de Permetyls, et leurs mélanges. De préférence, le solvant volatil hydrocarboné ayant de 8 à 16 atomes de carbone est choisi parmi l'isododécane, l'isodécane, l'isohexadécane, et leur mélange.

Selon un mode de réalisation particulier, le solvant volatil est l'isododécane.

Selon un autre mode particulier de réalisation, le solvant volatil hydrocarboné est un alcane linéaire volatil présentant un point éclair compris dans l'intervalle variant de 70 à 120 °C, et plus particulièrement de 80 à 100 °C, et notamment d'être d'environ 89 °C.

Un alcane linéaire volatil convenant à l'invention est liquide à température ambiante (environ 25 °C). Selon un mode de réalisation, un alcane convenant à l'invention peut être un alcane linéaire volatil comprenant de 8 à 16 atomes de carbone, en particulier de 10 à 15 atomes de carbone, et plus particulièrement de 11 à 13 atomes de carbone.

Un alcane linéaire volatil convenant à l'invention peut être avantageusement d'origine végétale.

Un tel alcane peut être obtenu, directement ou en plusieurs étapes, à partir d'une matière première végétale comme une huile, un beurre, une cire, etc.

A titre d'exemple d'alcane convenant à l'invention, on peut mentionner les alcanes décrits dans la demande de brevet de la société Cognis WO 2007/068371.

Ces alcanes sont obtenus à partir d'alcools gras, eux-mêmes obtenus à partir d'huile de coprah ou de palme.

A titre d'exemple d'alcane linéaire convenant à l'invention, on peut citer le n-nonane (C9), le n-décane (C10), le n-undécane (C11), le n-dodécane (C12), len-tridécane (C13), le n-tétradecane (C14), le n-pentadécane (C15), le n-héxadécane (C16) et le n-heptadécane (C17), et leurs mélanges, et en particulier le mélange de n-undécane (C11) et de n-tridécane (C13) commercialisé sous la réference de CETIOL UT par la Société Cognis.

Selon un mode de réalisation particulier, un alcane linéaire volatil convenant à l'invention peut être choisi parmi le n-nonane, le n-undécane, le n-dodécane, le n-tridécane, le n-héptadécane, et leurs mélanges.

Plus particulièrement, un alcane linéaire volatil convenant à l'invention peut être mis en oeuvre sous la forme d'un mélange n-undécane/n-tridécane.

De préférence, dans un tel mélange, le rapport pondéral n-undécane : n-tridécane peut être 50 : 50 à 90 : 10, de préférence variant de 60 : 40 à 80 : 20, de préférence variant de 65 : 35 à 75 : 25.

En particulier, une composition selon l'invention peut comprendre un mélange n-undécane : n-tridécane dans un rapport pondéral de 70 : 30. Un tel mélange est commercialisé sous la dénomination CETIOL UT par la société COGNIS.

Pour des produits de maquillage de la peau, notamment des fonds de teint et des rouges à lèvres, on utilisera avantageusement des huiles volatiles hydrocarbonées linéaires en ayant de 8 à 16 atomes de carbone.

Comme huile volatile siliconée, on peut citer les silicones linéaires ou cycliques ayant de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthylcyclotétrasiloxane, le

décaméthylcyclopentasiloxane, le dodécaméthylcyclohexasiloxane, l'heptaméthylhexyltri-siloxane, l'heptaméthyloctyltrisiloxane, l'octaméthyltrisiloxane, le décaméthyltétrasiloxane et leurs mélanges.

L'huile volatile peut être présente dans la composition selon l'invention en une teneur allant de 0,1 % à 90 % en poids, par rapport au poids total de la composition, de préférence allant de 1 % à 70 % en poids, et préférentiellement allant de 5 % à 50 % en poids.

La composition selon l'invention peut comprendre au moins une huile non volatile.

Par "huile non volatile", on entend une huile restant sur les matières kératiniques à température ambiante et pression atmosphérique au moins plusieurs heures et ayant notamment une pression de vapeur inférieure à $10^{-3}$ mm de Hg (0,13 Pa). On peut également définir une huile non volatile comme ayant une vitesse d'évaporation telle que dans les conditions définies précédemment, la quantité évaporée au bout de 30 minutes est inférieure à 0,07mg/$cm^2$.

Comme huile non volatile hydrocarbonée, on peut utiliser l'huile de paraffine ( ou vaseline), le squalane, le polyisobutylène hydrogéné (huile de Parléam), le perhydrosqualène, l'huile de vison, de tortue, de soja, l'huile d'amande douce, de calophyllum, de palme, de pépins de raisin, de sésame, de maïs, d'arara, de colza, de tournesol, de coton, d'abricot, de ricin, d'avocat, de jojoba, d'olive ou de germes de céréales ; des esters d'acide lanolique, d'acide oléique, d'acide laurique, d'acide stéarique ; les esters gras, notamment en C12-C36, tels que le myristate d'isopropyle, le palmitate d'isopropyle, le stéarate de butyle, le laurate d'hexyle, l'adipate de diisopropyle, l'isononanoate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de di(2-éthyl hexyle), le malate de diisostéaryle, le triisostéarate de glycérine ou de diglycérine ; l'acide béhénique, l'acide oléique, l'acide linoléique, l'acide linolénique ou l'acide isostéarique ; les alcools gras supérieurs, notamment en C16- C22, tels que le cétanol, l'alcool oléique, l'alcool linoléique ou linolénique, l'alcool isostéarique ou l'octyl dodécanol ; et leurs mélanges.

En particulier, pour obtenir un maquillage matifiant le teint, tout en conservant un usage confortable, aussi bien lors de l'application qu'après maquillage, on utilisera les huiles non volatiles hydrocarbonées en C6-C22 pouvant être choisies parmi:

- les carbonates, de formule (I) suivante : R1-O-C(=O)-O-R'1, avec R1 et R'1, identiques ou différents, représentent une chaîne alkyle en C4 à C12, et préférentiellement de C5 à C10, linéaire ou ramifiée, saturée ou insaturée (de préférence saturée), présentant éventuellement au moins un cycle, saturé ou non, de préférence saturé ;
  ces huiles de formule (I) pouvant être le dicaprylyl carbonate, commercialisé sous la dénomination Cetiol CC® par la société COGNIS, le di(ethyl-2-hexyl) carbonate, commercialisé sous la dénomination TEGOSOFT DEC® par la société Goldschmidt, di-isobutyryle carbonate ; di-neopentyl carbonate ; dipentyl carbonate ; di neoheptyl carbonate ; di-heptyl carbonate ; di-isononyl carbonate ; ou di-nonyl carbonate ;
- les monoesters, de formule (II) : R2-O-C(=O)-R'2, avec R2 et R'2, identiques ou différents, représentent une chaîne alkyle en C4 à C12, et préférentiellement de C5 à C10, linéaire ou ramifiée, saturée ou insaturée (de préférence saturée), présentant éventuellement au moins un cycle, saturé ou non, de préférence saturé ;
  ces huiles de formule (II) pouvant être l'éthyl 2-hexyl isobutyrate, éthyl 2-hexyl butyrate, caprylyl butyrate, isononyl isobutyrate, éthyl 2 hexyl hexanoate, isononyl hexanoate, neopentyl hexanoate, caprylyl heptanoate, octyl octanoate, commercialisé sous la dénomination DRAGOXAT EH® par la société SYMRISE, l'isononanoate d'isononyle,
- les di-esters de formule (III) suivante: R3-O-C(=O)-R'3-C(=O)-O-R"3, avec R3 et R"3, identiques ou différents, représentent une chaîne alkyle en C4 à C12, et préférentiellement de C5 à C10, linéaire ou ramifiée, saturée ou insaturée (de préférence saturée), présentant éventuellement au moins un cycle, saturé ou non, de préférence saturé, et R'3 représente une chaîne alkylène, saturée ou insaturée, en C1 à C4, de préférence de C2 à C4, comme par exemple une chaîne alkylène dérivée de succinate (dans ce cas R'3 est une chaîne alkylène en C2 saturé), maléate (dans ce cas R'3 est une chaîne alkylène en C2 insaturée), glutarate (dans ce cas R'3 est une chaîne alkylène en C3 saturé), ou adipate (dans ce cas R'3 est une chaîne alkylène en C4 saturé) ; en particulier, R3 et R"3 sont choisis parmi isobutyle, pentyle, neopentyle, hexyle, heptyle, neoheptyle, ethyl 2-hexyle, octyle, nonyle, isononyle ; on peut citer préférentiellement le dicaprylyl maléate, notamment commercialisé par la société ALZO ; le succinate de di(2-éthyl hexyle) ;
- les éthers, de formule suivante (IV) : R4-O-R4', avec R4 et R4', identiques ou différents, représentent une chaîne alkyle en C4 à C12, et préférentiellement de C5 à C10, linéaire ou ramifiée, saturée ou insaturée (de préférence saturée), présentant éventuellement au moins un cycle, saturé ou non, de préférence saturé ; en particulier, R4 et R4' sont choisis parmi isobutyle, pentyle, neopentyle, hexyle, heptyle, neoheptyle, éthyl 2-hexyle, octyle, nonyle, isononyle ; parmi les composés de formule (IV), on peut citer préférentiellement le dicaprylyl éther, commercialisé sous le nom de Cetiol OE® par la société COGNIS ;
- les tri-esters d'alkyles de formule (V) : R5-O-C(O)-CH2-CH[-O-C(O)-R'5]-CH2-O-C(O)-R"5, avec R5, R'5 et R"5, identiques ou différents, représentent une chaîne alkyle en C4-C10, de préférence C5-C8, linéaire ou ramifiée, saturée ou insaturée (de préférence saturée), en particulier R5, R'5 et R"5 sont identiques ; de préférence, R5, R'5 et R"5 (en particulier identiques) sont des radicaux alkyles des acides gras suivants : acide caprylique, éthyl-2

hexylique, neopentanoique, ou neoheptanoique ; on peut citer préférentiellement, comme composé de formule (V), le caprylique caprique triglycéride, commercialisé notamment sous le nom MYRITOL 318® par la société COGNIS ;

- et leurs mélanges.

L'huile non volatile hydrocarbonée en C6-C22 avantageusement utilisée dans lesdites compositions de maquillage et/ou de soin destinées à matifier le teint est le dicaprylyl carbonate, notamment commercialisé sous le nom de CETIOL CC par la société COGNIS.

Avantageusement, lorsque la composition est destinée au maquillage et/ou au soin des lèvres et comprend une huile non volatile, cette huile est choisie parmi les huiles siliconées phénylées. Une telle huile est également appelée silicone phénylée.

On entend par silicone phénylée, un organopolysiloxane substitué par au moins un groupe phényle.

La silicone phénylée est de préférence non volatile. Par « non volatile », on entend une huile dont la pression de vapeur à température ambiante et pression atmosphérique, est non nulle et inférieure à 0,13 Pa.

De préférence, le poids moléculaire en poids de l'huile siliconée phénylée est compris entre 500 et 10 000 g/mol.

L'huile siliconée peut être choisie parmi les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates.

L'huile siliconée peut répondre à la formule:

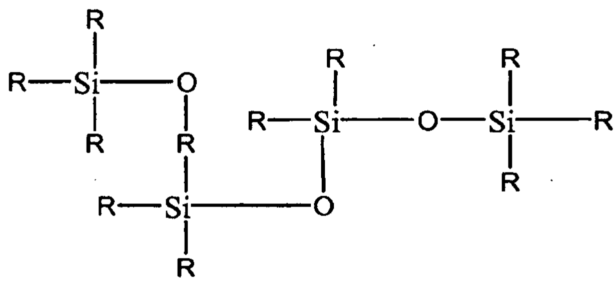

dans laquelle les groupements R représentent indépendamment les uns des autres un méthyle ou un phényle. De préférence dans cette formule, l'huile siliconée comprend au moins trois groupes phényle, par exemple au moins quatre, au moins cinq ou au moins six.

Selon un autre mode de mise en oeuvre, l'huile siliconée répond à la formule

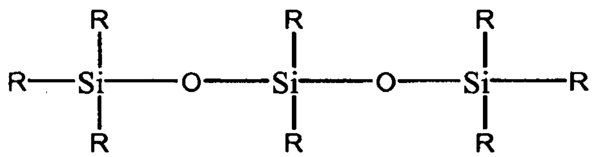

dans laquelle les groupements R représentent indépendamment les uns des autres un méthyle ou un phényle. De préférence dans cette formule, ledit organopolysiloxane comprend au moins trois groupes phenyles, par exemple au moins quatre ou au moins cinq.

Des mélanges des organopolysiloxanes phénylés décrits précédemment peuvent être utilisés.

On peut citer par exemples des mélanges d'organopolysiloxane triphénylé, tétra- ou penta-phénylé. Selon un autre mode de mise en oeuvre, l'huile siliconée répond à la formule

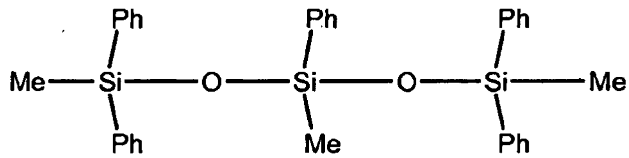

dans laquelle Me représente méthyle, Ph représente phényle. Une telle silicone phénylée est notamment fabriquée par Dow Corning sous la référence Dow Corning 555 Cosmetic Fluid (nom INCI : trimethyl pentaphenyl trisiloxane). La référence Dow Corning 554 Cosmetic Fluid peut aussi être utilisée.

Selon un autre mode de mise en oeuvre, l'huile siliconée répond à la formule

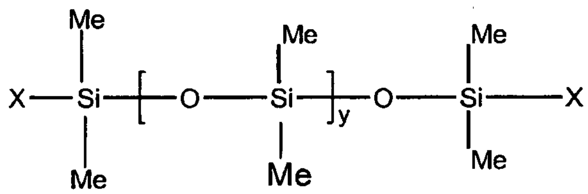

dans laquelle Me représente méthyle, y est compris entre 1 et 1 000, et X représente -CH2-CH(CH3)(Ph).

Selon un autre mode de mise en oeuvre, l'huile siliconée répond à la formule

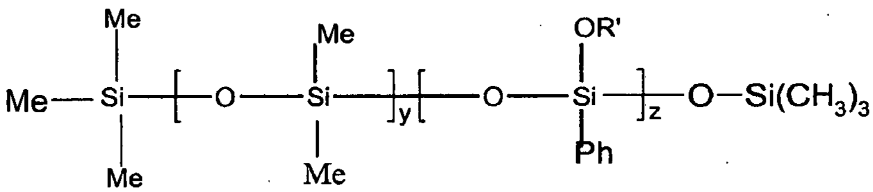

dans laquelle -OR' représente -O-SiMe3, y est compris entre 1 et 1 000 et z est compris entre 1 et 1000.

L'huile siliconée phénylée peut être choisie parmi les silicones phénylées de formule (VI) suivante :

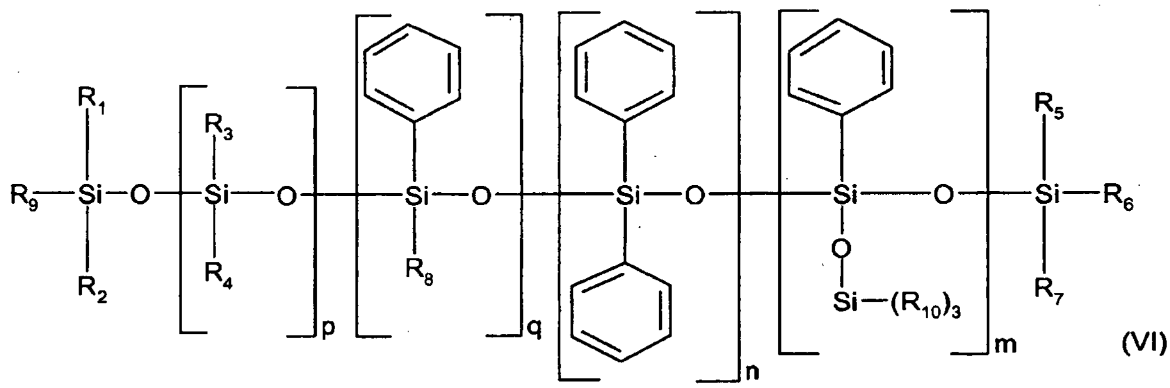

(VI)

dans laquelle

- R1 à R10, indépendamment les uns des autres, sont des radicaux hydrocarbonés, saturés ou insaturés, linéaires, cycliques ou ramifiés, en C1-C30,
- ni, n, p et q sont, indépendamment les uns des autres, des nombres entiers compris entre 0 et 900, sous réserve que la somme 'm+n+q' est différente de 0.

De préférence, la somme 'm+n+q' est comprise entre 1 et 100. De préférence, la somme 'm+n+p+q' est comprise entre 1 et 900, encore mieux entre 1 et 800. De préférence, q est égal à 0.

L'huile siliconée phénylée peut être choisie parmi les silicones phénylées de formule (VII) suivante :

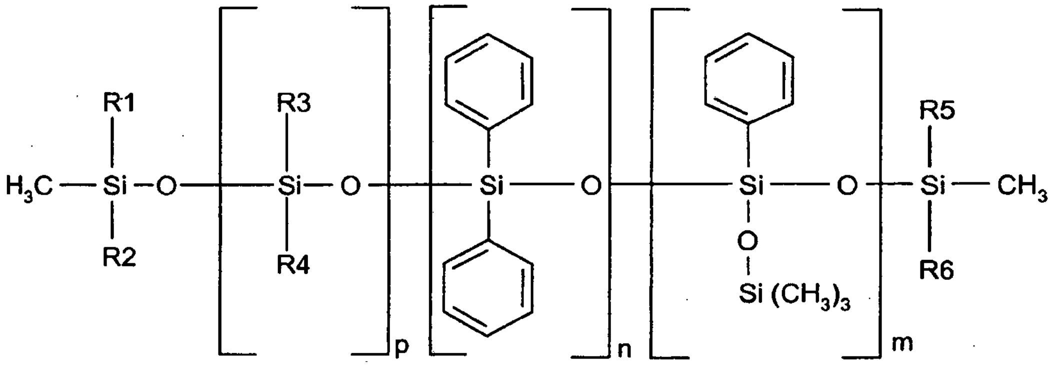

(VII)

dans laquelle :

- R1 à R6, indépendamment les uns des autres, sont des radicaux hydrocarbonés, saturés ou insaturés, linéaires cycliques ou ramifiés, en C1-C30,
- m, n et p sont, indépendamment les uns des autres, des nombres entiers compris entre 0 et 100, sous réserve que la somme 'n + m'est comprise entre 1 et 100.

De préférence, R1 à R6, indépendamment les uns des autres, représentent un radical hydrocarboné saturé, linéaire ou ramifié, en C1-C30, notamment en C1-C12, et en particulier un radical méthyle, éthyle, propyle ou butyle. Notamment, R1 à R6 peuvent être identiques, et en outre peuvent être un radical méthyle.
De préférence, on peut avoir m=1 ou 2 ou 3, et/ou n=0 et/ou p=0 ou 1., dans la formule (VII).
On peut utilise une huile siliconée phénylée de formule (VI) ayant une viscosité à 25°C comprise entre 5 et 1500 $mm^2/s$ (soit 5 à 1500 cSt), de préférence ayant une viscosité comprise entre 5 et 1000 $mm^2/s$ (soit 5 à 1000 cSt). Comme huile sliconée phénylée de formule (VII), on peut utiliser notamment les phényltriméthicones telles que la DC556 de Dow Corning (22,5 cSt), l'huile Silbione 70663V30 de Rhône Poulenc (28 cSt), ou les diphényldiméthicones telles que les huiles Belsil, notamment Belsil PDM1000 (1000cSt), Belsil PDM 200 (200 cSt) et Belsil PDM 20 (20cSt) de Wacker. Les valeurs entre parenthèses représentent les viscosités à 25°C.
L'huile siliconée non volatile peut être choisie parmi les silicones de formule :

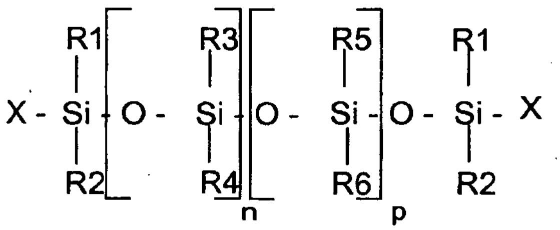

dans laquelle :

$R_1$, $R_2$, $R_5$ et $R_6$ sont, ensemble ou séparément, un radical alkyle ayant 1 à 6 atomes de carbone,
$R_3$ et $R_4$ sont, ensemble ou séparément, un radical alkyle ayant de 1 à 6 atomes de carbone, ou un radical aryle,
X est un radical alkyle ayant de 1 à 6 atomes de carbone, un radical hydroxyle ou un radical vinyle,
n et p étant choisis de manière à conférer à l'huile une masse moléculaire en poids inférieure à 200 000 g/mol, de préférence inférieure à 150 000 g/mol et de préférence encore inférieure à 100 000 g/mol.

L'huile non volatile peut être présente en une teneur allant de 0,1 % à 70 % en poids, par rapport au poids total de la phase grasse liquide non volatile, de préférence allant de 0,5 % à 60 % en poids, et préférentiellement allant de 1 % à 50 % en poids.
Pour des produits de maquillage de la peau, notamment des fonds de teint et des rouges à lèvres, on utilisera avantageusement des huiles de silicones linéaires volatiles ou non volatiles. L'association des résines selon l'invention et d'une huile siliconée linéaire peut notamment permettre d'améliorer le non transfert.
Pour des produits de maquillage de la peau, notamment des rouges à lèvres, on utilisera avantageusement des huiles de silicones phénylées. L'association des résines selon l'invention et d'une huile siliconée phénylée peut notamment permettre d'améliorer la brillance, le confort et diminuer la sensation du collant.

**Tensioactifs non ioniques**

La composition selon l'invention peut comprendre au moins un tensioactif non ionique.

En particulier, on peut utiliser un émulsionnant possédant à 25 °C une balance HLB (hydrophile-lipophile balance) au sens de GRIFFIN, spécifique de la composition que l'on souhaite obtenir.
La valeur HLB selon GRIFFIN est définie dans J. Soc. Cosm. Chem. 1954 (volume 5), pages 249-256. On peut se reporter au document "Encyclopedia of Chemical Technology, KIRK-OTHMER", volume 22, p. 333-432, 3ème édition, 1979, WILEY, pour la définition des propriétés et des fonctions (émulsionnant) des tensioactifs, en particulier p. 347-377 de cette référence, pour les tensioactifs non ioniques.

Les tensioactifs non ioniques utilisés préférentiellement dans la composition selon l'invention sont choisis parmi :

a) les tensioactifs non ioniques de HLB supérieur ou égal à 8 à 25 °C, utilisés seuls ou en mélange; on peut citer notamment :

- les esters et éthers d'oses tels que le mélange de cétylstéaryl glucoside et d'alcools cétylique et stéarylique comme le Montanov 68 de Seppic;
- les éthers oxyéthylénés et/ou oxypropylénés (pouvant comporter de 1 à 150 groupes oxyéthylénés et/ou oxypropylénés) de glycérol ;
- les éthers oxyéthylénés et/ou oxypropylénés (pouvant comporter de 1 à 150 groupes oxyéthylénés et/ou oxypropylénés) d'alcools gras (notamment d'alcool en C8-C24, et de préférence en C12-C18) tels que l'éther oxyéthyléné de l'alcool cétéarylique à 30 groupes oxyéthylénés (nom CTFA "Ceteareth-30"), l'éther oxyéthyléné de l'alcool stéarylique à 20 groupes oxyéthylénés (nom CTFA "Steareth-20"), et l'éther oxyéthyléné du mélange d'alcools gras en C12-C15 comportant 7 groupes oxyéthylénés (nom CTFA "C12-15 Pareth-7") commercialisé sous la dénomination NEODOL 25-7® par SHELL CHEMICALS,
- les esters d'acide gras (notamment d'acide en C8-C24, et de préférence en C16-C22) et de polyéthylène glycol (pouvant comprendre de 1 à 150 motifs d'éthylèneglycol) tels que le stéarate de PEG-50 et le monostéarate de PEG-40 commercialisé sous le nom MYRJ 52P® par la société ICI UNIQUEMA,
- les esters d'acide gras (notamment d'acide en C8-C24, et de préférence en C16-C22) et des éthers de glycérol oxyéthylénés et/ou oxypropylénés (pouvant comporter de 1 à 150 groupes oxyéthylénés et/ou oxypropylénés), comme le monostéarate de PEG-200 glycéryle vendu sous la dénomination Simulsol 220 TM® par la société SEPPIC ; le stéarate de glycéryle polyéthoxylé à 30 groupes d'oxyde d'éthylène comme le produit TAGAT S® vendu par la société GOLDSCHMIDT, l'oléate de glycéryle polyéthoxylé à 30 groupes d'oxyde d'éthylène comme le produit TAGAT O® vendu par la société GOLDSCHMIDT, le cocoate de glycéryle polyéthoxylé à 30 groupes d'oxyde d'éthylène comme le produit VARIONIC LI 13® vendu par la société SHEREX, l'isostéarate de glycéryle polyéthoxylé à 30 groupes d'oxyde d'éthylène comme le produit TAGAT L® vendu par la société GOLDSCHMIDT et le laurate de glycéryle polyéthoxylé à 30 groupes d'oxyde d'éthylène comme le produit TAGAT I® de la société GOLDSCHMIDT,
- les esters d'acide gras (notamment d'acide en C8-C24, et de préférence en C16-C22) et des éthers de sorbitol oxyéthylénés et/ou oxypropylénés (pouvant comporter de 1 à 150 groupes oxyéthylénés et/ou oxypropylénés), comme le polysorbate 20 vendu sous la dénomination Tween 20® par la société CRODA, le polysorbate 60 vendu sous la dénomination Tween 60® par la société CRODA,
- la diméthicone copolyol, telle que celle vendue sous la dénomination Q2-5220® par la société DOW CORNING,
- la diméthicone copolyol benzoate (FINSOLV SLB 101® et 201® de la société FINTEX),
- les copolymères d'oxyde propylène et d'oxyde d'éthylène, également appelés polycondensats OE/OP,
- et leurs mélanges.

Les polycondensats OE/OP sont plus particulièrement des copolymères consistant en des blocs polyéthylène glycol et polypropylène glycol, comme par exemple les polycondensats tribloc polyéthylène glycol/polypropylène glycol/polyéthylène glycol. Ces polycondensats tribloc ont par exemple la structure chimique suivante :

$$H\text{-}(O\text{-}CH_2\text{-}CH_2)_a\text{-}(O\text{-}CH(CH_3)\text{-}CH_2)_b\text{-}(O\text{-}CH_2\text{-}CH_2)_a\text{-}OH,$$

formule dans laquelle a va de 2 à 120, et b va de 1 à 100.
Le polycondensat OE/OP a de préférence un poids moléculaire moyen en poids allant de 1000 à 15000, et de mieux allant de 2000 à 13000. Avantageusement, ledit polycondensat OE/OP a une température de trouble, à 10 g/l en eau distillée, supérieure ou égale à 20 °C, de préférence supérieure ou égale à 60 °C. La température de trouble est mesurée selon la norme ISO 1065. Comme polycondensat OE/OP utilisable selon l'invention, on peut citer les polycondensats tribloc polyéthylène glycol / polypropylène glycol / polyéthylène glycol vendus sous les dénominations SYNPERONIC® comme les SYNPERONIC PE/ L44® et SYNPERONIC PE/F127® par la société ICI.

b) les tensioactifs non ioniques de HLB inférieur à 8 à 25 °C, éventuellement associés à un ou plusieurs agents tensioactif non ioniques de HLB supérieur à 8 à 25 °C, tels que cités ci-dessus tels que :

- les esters et éthers d'oses tels que les stéarate de sucrose, cocoate de sucrose, stéarate de sorbitan et leurs mélanges comme l'Arlatone 2121® commercialisé par la société ICI ;
- les éthers oxyéthylénés et/ou oxypropylénés (pouvant comporter de 1 à 150 groupes oxyéthylénés et/ou oxypropylénés) d'alcools gras (notamment d'alcool en C8-C24, et de préférence en C12-C18) tels que l'éther oxyéthyléné de l'alcool stéarylique à 2 groupes oxyéthylénés (nom CTFA "Steareth-2") ;
- les esters d'acides gras (notamment d'acide en C8-C24, et de préférence en C16-C22) et de polyol, notamment de glycérol ou de sorbitol, tels que stéarate de glycéryle, stéarate de glycéryle tel que le produit vendu sous la dénomination TEGIN M® par la société GOLDSCHMIDT, laurate de glycéryle tel que le produit vendu sous la dénomination IMWITOR 312® par la société HULS, stéarate de polyglycéryl-2, tristéarate de sorbitan, ricinoléate de glycéryle ;
- les lécithines, telles que les lécithines de soja (comme Emulmetik 100 J de Cargill, ou Biophilic H de Lucas Meyer) ;
- le mélange de cyclométhicone/diméthicone copolyol vendu sous la dénomination Q2-3225C® par la société DOW CORNING.

Le tensioactif non ionique peut aussi être choisi parmi un alkyl C8-C22 diméthicone copolyol, c'est-à-dire un poly méthyl alkyl(C8-C22) diméthyl méthyl siloxane oxypropyléné et/ou oxyéthyléné.
L'alkyl C8-C22 diméthicone copolyol est avantageusement un composé de formule (I) suivante :

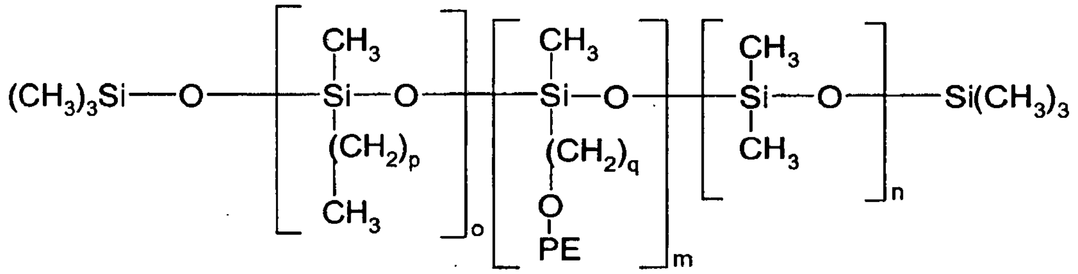

**(I)**

dans laquelle :

- PE représente (-C2H4O)x-(C3H60)y-R, R étant choisi parmi un atome d'hydrogène et un radical alkyle de 1 à 4 atomes de carbone, x allant de 0 à 100 et y allant de 0 à 80, x et y n'étant pas simultanément 0
- m allant de 1 à 40
- n allant de 10 à 200
- o allant de 1 à 100
- p allant de 7 et 21
- q allant de 0 à 4

et de préférence R = H, m = 1 à 10 ; n = 10 à 100 ; o = 1 à 30 ; p = 15 ; et q = 3.
Comme alkyl C8-C22 diméthicone copolyol, on peut citer le cétyl diméthicone copolyol comme le produit commercialisé sous la dénomination Abil EM-90 par la société Goldschmidt.
Selon un mode particulier destiné à formuler des émulsions stables, présentant une viscosité faible permettant une application aisée, tout en conférant au produit de maquillage une tenue dans le temps du maquillage une fois appliqué, on utilisera avantageusement au moins un tensioactif non ionique siliconé, éventuellement associé à au moins un tensioactif hydrocarboné et éventuellement en outre au moins une cire:

Comme tensioactif siliconé non ionique, on peut citer par exemple :

a) les polydialkyl silicones à groupes latéraux et/ou terminaux hydrophiles polyoxyalkylénés (polyoxyethylénés (ou POE) et/ou polyoxypropylénés (ou PPO). De plus, ces tensio-actifs siliconés comportent, de préférence, des groupes latéraux alkyles en C1 à C20 linéaires ou ramifiés, de préférence des groupes alkyles linéaires,

tels que lauryle ou cétyle. Ces tensio-actifs peuvent encore porter des groupes latéraux organosiloxanes.
En particulier, dans cette première catégorie, on peut citer :

- les polydimethyl siloxanes à groupes latéraux POE, tels que notamment les KF-6011, KF-6012, KF-6013, KF-6015, KF-6016 et KF-6017 de la société Shin Etsu. ;
- les polydiméthyl siloxanes à groupements latéraux POE et à groupes latéraux alkyles, tels que notammment le Cetyl PEG-PPG 10/1 dimethicone, commercialisé sous la dénomination ABIL EM90 par la société Evonik GOLDSCHMIDT ;
- les polydimethylsiloxanes ramifiées à groupes latéraux POE, tels que notamment le PEG-9 polydimethyl siloxyethyl dimethicone, commercialisé sous la dénomination KF-6028 par la société Shin Etsu ;
- les polydimethyl siloxanes ramifiées à groupes latéraux alkyles, tels que notamment le lauryl PEG-9 polydimethylsiloxyethyl dimethicone, commercialisé sous la dénomination KF-6038 par la société Shin Etsu.

b) les polydialkyl silicones à groupements latéraux polyglycérolés ou glycérolés. Ces tensio-actifs siliconés comportent en plus, de préférence, des groupes latéraux alkyles en C1 à C20 linéaires ou ramifiés, et de préférence aussi des groupes alkyles linéaires tels que lauryl ou cetyl. De même, ces tensio-actifs siliconés et glycérolés peuvent, en plus, porter des groupes latéraux organosiloxanes.
En particulier, dans cette catégorie, on peut citer :

- les polydimethyl siloxanes à groupes latéraux polyglycérolés, tel que le polyglycéryl -3 disiloxane diméthicone, commercialisé sous la dénomination KF-6100 par la société Shin Etsu ;
- les polydiméthyl siloxanes ramifiés et à groupes latéraux polyglycérolés, tel que le polyglycéryl -3 polydiméthylsiloxyethyl diméthicone, commercialisé sous la dénomination KF-6104 par la société Shin Etsu ;
- les polydimethyl siloxanes ramifiés, à groupes latéraux polyglycérolés et à groupe latéraux alkyles, tel que le lauryl polyglycéryl -3 polydimethyl siloxyethyl dimethicone, commercialisé sous la dénomination KF-6105 par la société Shin Etsu.

Parmi les tensioactifs non ioniques siliconés, on préfère le Cetyl PEG/PPG - 10/1 dimethicone, commercialisé sous la dénomination ABIL EM90 par la société EVONIK GOLDSCHMIDT.
Le tensioactif siliconé non ionique est avantageusement en association avec au moins un tensioactif organique non ionique.
Comme tensioactif organique non ionique, on peut citer les esters d'acides gras de polyols comme les mono-, di-, tri- ou sesqui-oléates ou stéarates de sorbitol ou de glycérol, les laurates de glycérol ou de polyéthylène glycol ; les esters d'acides gras de polyéthylène glycol (monostéarate ou monolaurate de polyéthylène glycol) ; les esters d'acides gras (stéarate, oléate) de sorbitol polyoxyéthylénés ; les alkyl (lauryl, cétyl, stéaryl, octyl) éthers polyoxyéthylénés.
Parmi les tensio-actifs organiques non ioniques, on préfère :

- les esters d'acides gras polyglycérolés comportant au moins 3 motifs glycérol éther, tel que le polyglycéryl 3 ;
- les esters d'acides gras polyoxyalkylénés (polyoxyethylénés et/ou polyoxypropylénés), comportant de préférence au moins 3 groupes oxyéthylène ;
- les éthers d'alcools gras et de polyglycérols avec au moins 3 motifs éther de glycéryle ;
- les éthers d'alcools gras et de polyoxyalkylène (POE et/ou POE/PPO) avec au moins 3 groupes POE.

Parmi les tensioactifs organiques non ioniques, on préfère le polyglyceryl-4 isostéarate commercialisé sous la dénomination ISOLAN GI34® par la société EVONIK GOLDSCHMIDT.
En association avec le tensioactif siliconé non ionique et le tensioactif organique non ionique, on pourra utiliser avantageusement au moins une cire.
Parmi les cires, on préfère le mélange de stéarate d'éthylène glycol acétyl / tri-stéarate de glycéryl, notamment commercialisé sous la dénomination UNITWIX par a société UNITED GARDIAN.

**Agent rhéolosique épaississant ou gélifiant de phase grasse**

La composition selon l'invention peut comprendre en outre un agent rhéologique épaississant ou gélifiant de phase grasse.
On entend par « agent rhéologique épaississant ou gélifiant de phase grasse » un composé apte à augmenter la viscosité de la phase grasse de la composition. L'agent rhéologique épaississant ou gélifiant de phase grasse permet notamment d'obtenir une composition pouvant présenter une texture allant des textures fluides à solides.
De préférence, les agents rhéologiques épaississants ou gélifiants de phase grasse sont choisis parmi les

polymères cristallins, les agents structurants lipophiles minéraux, les polyamides lipophiles, les polyurées et polyuréthanes lipophiles, les polymères siliconés comprenant le cas échéant au moins un motif hydrocarboné comportant deux groupes capables d'établir des interactions hydrogènes choisis parmi les groupes ester, amide, sulfonamide, carbamate, thiocarbamate, urée, uréthane, thiourée, oxamido, guanidino, biguanidino et leurs combinaisons, les organogélateurs, les polymères blocs, les agents cristaux liquides cholestériques, les copolymères dimethicone/vinyldimethicone, et les copolymères vinyldimethicone/alkyl dimethicone, tels que les copolymères vinyldimethicone/lauryl dimethicone.

L'agent rhéologique épaississant ou gélifiant de phase grasse peut être choisi parmi :

- les polymères cristallins, de préférence choisis parmi les polymères semi-cristallins, les esters de dextrine et d'acide gras, les polysaccharides modifiés hydrophobes, les copolymères d'oléfines cristallins et les polycondensats cristallins;
- les agents structurants lipophiles minéraux, comme les argiles lipophiles et les silices hydrophobes, comme la silice pyrogénée traitée hydrophobe,
- les polymères de type polyamide lipophiles,
- les polyurées et les polyuréthanes lipophiles,
- les polymères siliconés comprenant le cas échéant au moins un motif hydrocarboné comportant deux groupes capables d'établir des interactions hydrogènes choisis parmi les groupes ester, amide, sulfonamide, carbamate, thiocarbamate, urée, uréthane, thiourée, oxamido, guanidino, biguanidino et leurs combinaisons, de préférence des groupes amides,
- les organogélateurs ;
- les polymères blocs ;
- les agents cristaux liquides cholestériques ;
- les élastomères de silicone ;
- et leurs mélanges.

De préférence, l'agent rhéologique de phase grasse est choisi parmi les polymères semi-cristallins, les polymères blocs, les polymères de type polyamide lipophiles, et les polymères siliconés comprenant au moins un motif hydrocarboné comportant deux groupes capables d'établir des interactions hydrogènes choisis parmi les groupes amides, les agents structurants lipohiles minéraux, en particulier les argiles lipophiles et les silices hydrophobes, et les élastomères de silicone.

On précise que, selon l'invention, dans le cas des associations d'un agent rhéologique de phase grasse avec une huile, on entend par « huile », un corps gras liquide à température ambiante. Les huiles peuvent être celles décrites précédemment.

*<u>Agents structurants lipophiles minéraux</u>*

L'agent rhéologique épaississant ou gélifiant de phase grasse peut être un agent structurant lipophile minéral. On peut notamment citer les argiles lipophiles, comme les argiles éventuellement modifiées telles que les hectorites modifiées par un chlorure d'ammonium d'acide gras en $C_{10}$ à $C_{22}$, comme l'hectorite modifiée par du chlorure de distéaryl di-méthyl ammonium.

Les argiles sont des silicates contenant un cation pouvant être choisi parmi les cations de calcium, de magnésium, d'aluminium, de sodium, de potassium, de lithium, et leurs mélanges.
A titre d'exemples de tels produits, on peut citer les argiles de la famille des smectites telles que les montmorillonites, les hectorites, les bentonites, les beidellites, les saponites, ainsi que de la famille des vermiculites, de la stévensite, des chlorites. Ces argiles peuvent être d'origine naturelle ou synthétique.
L'argile peut être choisie parmi la montmorrillonite, la bentonite, l'hectorite, l'attapulgite, la sépiolite, et leurs mélanges. L'argile est de préférence une bentonite ou une hectorite.
Les argiles organophiles sont des argiles modifiées avec un composé chimique choisi parmi les aminés quaternaires, les aminés tertiaires, les acétates aminés, les imidazolines, les savons aminés, les sulfates gras, les alkyl aryl sulfonates, les oxides amines, et leurs mélanges.
Comme argiles organophiles, on peut citer les quaternium-18 bentonites telles que celles vendues sous les dénominations Bentone 3, Bentone 38, Bentone 38V par la société Elementis, Tixogel VP par la société United catalyst, Claytone 34, Claytone 40, Claytone XL par la société Southern Clay ; les stéaralkonium bentonites telles que celles vendues sous les dénominations Bentone 27V par la société Elementis, Tixogel LG par la société United Catalyst, Claytone AF, Claytone APA par la société Southern Clay ; les quaternium-18/benzalkonium bentonite telles que celles vendues sous les dénominations Claytone HT, Claytone PS par la société Southern Clay.
Selon un mode de réalisation préféré, l'agent épaississant est choisi parmi les argiles modifiées organophiles, telles que l'hectorite modifiée par le stéarate de benzyldiméthylammonium.

On peut également citer les silices hydrophobes, comme la silice pyrogénée éventuellement traitée hydrophobe en surface dont la taille des particules est inférieure à 1 μm Il est en effet possible de modifier chimiquement la surface de la silice, par réaction chimique générant une diminution du nombre de groupes silanol présents à la surface de la silice. On peut notamment substituer des groupes silanol par des groupements hydrophobes : on obtient alors une silice hydrophobe. Les groupements hydrophobes peuvent être :

- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6ème édition, 1995). Elles sont par exemple commercialisées sous les références "AEROSIL R812®" par la société Degussa, "CAB-O-SIL TS-530®" par la société Cabot.
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénommées "Silica diméthyl silylate" selon le CTFA (6ème édition, 1995). Elles sont par exemple commercialisées sous les références "AEROSIL R972®", "AEROSIL R974®" par la société Degussa, "CAB-O-SIL TS-610®", "CAB-O-SIL TS-720®" par la société Cabot.

La silice pyrogénée hydrophobe présente de préférence une taille de particules pouvant être nanométrique à micrométrique, par exemple allant d'environ de 5 à 200 nm.

*Polymères polyamides lipophiles*

Par polymère, on entend au sens de l'invention un composé ayant au moins 2 motifs de répétition, de préférence au moins 3 motifs de répétition et mieux encore 10 motifs de répétition.

Comme polymères polyamides structurants lipophiles préférés utilisables dans l'invention, on peut citer les polyamides ramifiés par des chaînes grasses pendantes et/ou des chaînes grasses terminales ayant de 12 à 120 atomes de carbone et notamment de 12 à 68 atomes de carbone, les chaînes grasses terminales étant liées au squelette polyamide par des groupes ester. Ces polymères sont plus spécialement ceux décrits dans le document US-A-5783657 de la société Union Camp. En particulier on peut citer les polymères dont le nom INCI est « éthylènediamine/stéaryl dimère dilinoléate copolymère » et « éthylène diamine/stéaryl dimère tallate copolymère ».

A titre d'exemple de polymères structurants utilisables dans la composition selon l'invention, on peut citer les produits commerciaux vendu par la société Bush Boake Allen sous les noms Uniclear 80, Uniclear 100 Uniclear 80 V, Uniclear 100 V et Uniclear 100 VG. Ils sont vendus respectivement sous forme de gel à 80 % (en matière active) dans une huile minérale et à 100 % (en matière active). Ils ont un point de ramollissement de 88 à 94°C. Ces produits commerciaux sont un mélange de copolymère d'un diacide en $C_{36}$ condensé sur l'éthylène diamine, de masse moléculaire moyenne d'environ 6000. Les terminaisons d'acide restantes sont, en outre, estérifiées par l'alcool cétylique, stéarylique ou leurs mélanges (appelés aussi alcool cétylstéarylique).

*Polymères polyurées ou polyuréthanes lipophiles*

Comme agent rhéologique de phase grasse, on peut également citer les polyuréthanes et les polyurées solubles ou dispersibles dans la (ou les) huile(s) hydrocarbonée(s), et comportant:

- au moins deux groupes uréthanes, ou au moins deux groupes urées ou au moins un groupe uréthane et un groupe urée dans la chaîne,
- au moins une séquence ou un greffon hydrocarboné ou polyester aliphatique à longue chaîne hydrocarbonée, de préférence ramifiée.

Par longue chaîne hydrocarbonée, on entend une chaîne hydrocarbonée, linéaire ou ramifiée, comportant au moins 8 atomes de carbone et de préférence 10 à 500 atomes de carbone.

*Polymères silïconés lipophiles :*

Les agents structurants lipophiles polymériques siliconés sont par exemple des polymères du type polyorganosiloxane comme ceux décrits dans les documents US-A-5 874 069, US-A-5,919,441, US-A-6,051,216 et US-A-5,981,680. Selon l'invention, les polymères utilisés comme agent structurant peuvent appartenir aux deux familles suivantes :

1) des polyorganosiloxanes comportant au moins deux groupes capables d'établir des interactions hydrogène, ces deux groupes étant situés dans la chaîne du polymère, et/ou

2) des polyorganosiloxanes comportant au moins deux groupes capables d'établir des interactions hydrogène, ces deux groupes étant situés sur des greffons ou ramifications.

Les groupes capables d'établir des interactions hydrogène peuvent être choisis parmi les groupes ester, amide, sulfonamide, carbamate, thiocarbamate, urée, uréthane, thiourée, oxamido, guanidino, biguanidino et leurs combinaisons.

Selon une première variante, les polymères siliconés sont des polyorganosiloxanes tels que définis ci-dessus et dont les motifs capables d'établir des interactions hydrogènes sont disposés dans la chaîne du polymère.

Selon une variante de l'invention, on peut encore utiliser un polymère comprenant de plus au moins un motif hydrocarboné comportant deux groupes capables d'établir des interactions hydrogènes choisis parmi les groupes ester, amide, sulfonamide, carbamate, thiocarbamate, urée, uréthane, thiourée, oxamido, guanidino, biguanidino et leurs combinaisons.

Ces copolymères peuvent être des polymères blocs, des polymères séquencés ou des polymères greffés.

A titre d'exemple de polymère siliconé utilisable, on peut citer un des polyamides siliconés, obtenus conformément aux exemples 1 à 3 du document US-A-5 981 680.

Selon une variante de réalisation de l'invention, le polymère est constitué par un homopolymère ou copolymère comportant des groupes uréthane ou urée. Ces polymères sont décrits en détail dans la demande WO 2003/106614 publiée le 24/12/2003 dont le contenu est incorporé dans la présente demande par référence

Les polymères et copolymères utilisés dans la composition de l'invention ont avantageusement une température de transition solide-liquide de 45°C à 190°C. De préférence, ils présentent une température de transition solide-liquide allant de 70 à 130°C et mieux de 80°C à 105°C.

<u>Organogélateurs</u> :

Le structurant huileux peut également être choisi parmi les gélifiants organiques moléculaires non polymériques, également appelés organogélateurs, qui sont des composés dont les molécules sont capables d'établir entre elles des interactions physiques conduisant à une auto-agrégation des molécules avec formation d'un réseau supra-moléculaire 3D qui est responsable de la gélification de la (des) huile(s) (appelée également phase grasse liquide).

Le réseau supra-moléculaire peut résulter de la formation d'un réseau de fibrilles (dues aux empilements ou agrégations de molécules d'organogélateur), immobilisant les molécules de la phase grasse liquide.

L'aptitude à former ce réseau de fibrilles, et donc à gélifier, dépend de la nature (ou classe chimique) de l'organogélateur, de la nature des substituants portés par ses molécules pour une classe chimique donnée et de la nature de la phase grasse liquide.

Les interactions physiques sont diverses mais excluent la co-cristallisation. Ces interactions physiques sont en particulier des interactions du type interactions hydrogènes auto-complémentaires, interactions $\pi$ entre cycles insaturés, interactions dipolaires, liaisons de coordination avec des dérivés organométalliques et leurs associations. En général, chaque molécule d'un organogélateur peut établir plusieurs types d'interactions physiques avec une molécule voisine. Aussi, avantageusement, les molécules des organogélateurs selon l'invention comportent au moins un groupement capable d'établir des liaisons hydrogènes et mieux au moins deux groupements capables d'établir des liaisons hydrogène, au moins un cycle aromatique et mieux aux moins deux cycles aromatiques, au moins une ou plusieurs liaisons à insaturation éthylénique et/ou au moins un ou plusieurs carbones asymétriques. De préférence, les groupements capables de faire des liaisons hydrogènes sont choisis parmi les groupements hydroxyle, carbonyle, amine, acide carboxylique, amide, urée, benzyle et leurs associations.

Le ou les organogélateurs selon l'invention sont solubles dans la phase grasse liquide après chauffage jusqu'à obtention d'une phase liquide homogène transparente. Ils peuvent être solides ou liquides à température ambiante et pression atmosphérique.

Le ou les organogélateurs moléculaires utilisables dans la composition selon l'invention sont notamment ceux décrits dans le document "Specialist Surfactants", édité par D. Robb de 1997, p.209-263, chapitre 8 de P. Terech, les demandes européennes EP-A-1068854 et EP-A-1086945 ou encore dans la demande WO-A-02/47031.

On peut notamment citer parmi ces organogélateurs, les amides d'acides carboxyliques en particulier les acides tri-carboxyliques comme les cyclohexanetricarboxamides (voir la demande de brevet européen EP-A-1068854), les diamides ayant des chaînes hydrocarbonées contenant chacune de 1 à 22 atomes de carbone, par exemple de 6 à 18 atomes de carbone, lesdites chaînes étant non substituées ou substituées avec au moins un substituant choisi parmi les groupes ester, urée et fluoro (voir la demande EP-A-1086945) et notamment les diamides résultant de la réaction du diaminocyclohexane, en particulier du diaminocyclohexane sous forme trans, et d'un chlorure d'acide comme par exemple le N,N'-bis (dodécanoyl)-1,2-diaminocyclohexane, les amides de N-acylamino acides comme les diamides

résultant de l'action d'un N-acylamino acide avec des amines comportant de 1 à 22 atomes de carbone, comme par exemple ceux décrits dans le document WO-93/23008 et notamment les amides de l'acide N-acylglutamique où le groupe acyle représente une chaîne alkyle en $C_8$ à $C_{22}$ tels que le dibutylamide de l'acide N-Lauroyl-L-glutamique, fabriqué ou commercialisé par la société Ajinomoto sous la dénomination GP-1 et leurs mélanges.

On peut également utiliser comme organogélateurs des composés de type bis-urée de formule générale suivante :

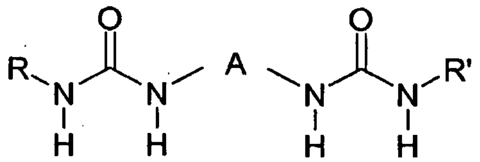

dans laquelle :

- A est un groupement de formule (II) :

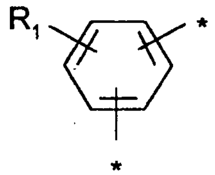

avec R1 étant un radical alkyle $C_1$ à $C_4$ linéaire ou ramifié, et les * symbolisant les points de rattachement du groupement A à chacun des deux atomes d'azote du reste du composé de formule générale (I), et

- R et R', identiques ou différents, sont choisis parmi :
  - i) les radicaux de formule (III) :

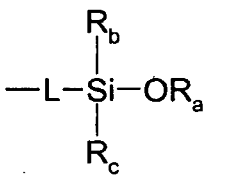

(III)

dans laquelle :

- L est une liaison simple ou un radical divalent carboné, notamment hydrocarboné (alkylène), linéaire, ramifié et/ou cyclique, saturé ou insaturé, comprenant 1 à 18 atomes de carbone, et pouvant comprendre 1 à 4 hétéroatomes choisis parmi N, O et S;
- Ra est :

a) un radical carboné, notamment hydrocarboné (alkyle), linéaire, ramifié et/ou cyclique, saturé ou insaturé, comprenant 1 à 18 atomes de carbone, et pouvant comprendre 1 à 8 hétéroatomes choisis parmi N, O, Si et S; ou bien
b) un radical siliconé de formule :

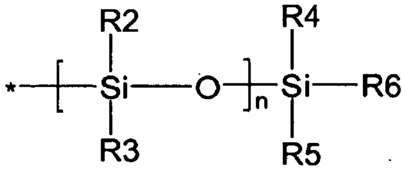

avec n étant compris entre 0 et 100, notamment entre 1 et 80, voire 2 à 20;
et R2 à R6 étant, indépendamment les uns des autres, des radicaux carbonés, notamment hydrocarbonés (alkyles) linéaires ou ramifiés, ayant 1 à 12, notamment 1 à 6 atomes de carbone, et pouvant comprendre 1 à 4 hétéroatomes, notamment O;

- Rb et Rc sont, indépendamment l'un de l'autre, choisis parmi:

  a) les radicaux carbonés, notamment hydrocarbonés (alkyles), linéaires, ramifiés et/ou cycliques, saturés ou insaturés, comprenant 1 à 18 atomes de carbone, et pouvant comprendre 1 à 4 hétéroatomes choisis parmi N, O, Si et S;
  b) les radicaux de formule :

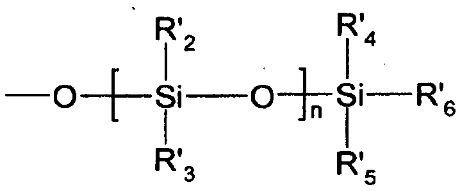

avec n étant compris entre 0 et 100, notamment entre 1 et 80, voire 2 à 20;
et R'2 à R'6 étant, indépendamment les uns des autres, des radicaux carbonés, notamment hydrocarbonés (alkyles) linéaires ou ramifiés, ayant 1 à 12, notamment 1 à 6 atomes de carbone, et pouvant comprendre 1 à 4 hétéroatomes, notamment O.
et

- ii) les radicaux alkyle en $C_1$ à $C_{30}$, linéaire, ramifié et/ou cyclique, saturé ou insaturé, et comprenant éventuellement 1 à 3 hétéroatomes choisis parmi O, S, F et N; étant entendu que l'un au moins des radicaux R et/ou R' est de formule (III).

Notamment le groupement A peut être de formule :

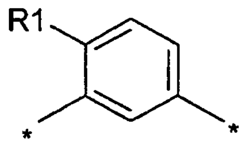

ou

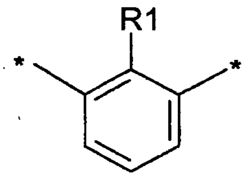

avec R1 et les * étant tels que définis précédemment.
En particulier, R1 peut être un groupement méthyle, ce qui conduit à un groupement A de formule :

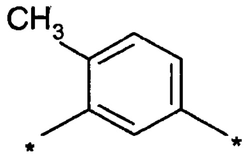

ou

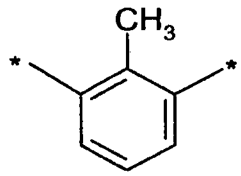

dans laquelle les * sont tels que définis précédemment.
En particulier, les composés selon l'invention peuvent être sous forme de mélange lié au fait que A peut être un mélange de 2,4-tolylène et 2,6-tolylène, notamment dans des proportions (isomère 2,4)/(isomère 2,6) variant de 95/5 à 80/20.
Selon l'invention, l'un au moins des radicaux R et/ou R' doit être de formule (III):

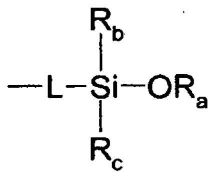

**(III)**

Dans cette formule, L est de préférence un radical divalent carboné, notamment hydrocarboné (alkylène), linéaire, ramifié et/ou cyclique, saturé ou insaturé, comprenant 1 à 18 atomes de carbone, et pouvant comprendre 1 à 4 hétéroatomes choisis parmi N, O et S. Dans le radical L, la chaîne carbonée peut être interrompue par le/les hétéroatomes et/ou peut comprendre un substituant comprenant ledit/lesdits hétéroatomes.
En particulier, L peut être de structure $-(CH_2)n-$ avec n= 1 à 18, notamment 2 à 12, voire 3 à 8. De préférence, L est choisi parmi les radicaux méthylène, éthylène, propylène, butylène et notamment n-butylène ou octylène.
Le radical L peut également être ramifié, par exemple du type $-CH_2-CH(CH_3)-$, ce qui conduit au radical de formule (III) suivant :

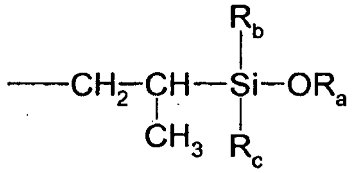

Le radical Ra peut être un radical carboné, notamment hydrocarboné (allyle), linéaire, ramifié et/ou cyclique, saturé ou insaturé, comprenant 1 à 18 atomes de carbone, et pouvant comprendre 1 à 8 hétéroatomes choisis parmi N, O, Si et S. La chaîne carbonée peut être interrompue par le/les hétéroatomes et/ou peut comprendre un substituant comprenant ledit/lesdits hétéroatomes; les hétéroatomes peuvent notamment former un ou plusieurs groupements -SiO- (ou -OSi)-.

Ainsi, le radical Ra peut être de structure $-(CH_2)n'-CH_3$ avec n'= 0 à 17, notamment 1 à 12, voire de 1 à 6. En particulier, Ra peut être méthyle, éthyle, propyle ou butyle.
Il peut également être de structure $-(CH_2)x-O-(CH_2)z-CH_3$ ou bien $-(CH_2)x-O-(CH_2)y-O-(CH_2)z-CH_3$, avec x = 1 à 10, de préférence 2; y=1 à 10 de préférence 2, et z= 0 à 10, de préférence 0 ou 1.
Le radical Ra peut encore être de structure $-SiR_4R_5R_6$ (cas où n=0), dans laquelle R4, R5 et R6 sont, indépendamment les uns des autres, de préférence, des radicaux alkyle, ayant 1 à 12 atomes de carbone, notamment 1 à 6 atomes de carbone; en particulier R4, R5 et/ou R6 peuvent être choisis parmi méthyle, éthyle, propyle, butyle.
Le radical Ra peut également être un radical siliconé de formule :

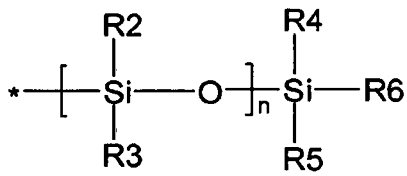

dans laquelle R2 à R6 sont, indépendamment les uns des autres, de préférence, des radicaux alkyle, ayant 1 à 12 atomes de carbone, notamment 1 à 6 atomes de carbone; en particulier R2 à R6 peuvent être choisis parmi méthyle, éthyle, propyle, butyle;
et en particulier un radical :

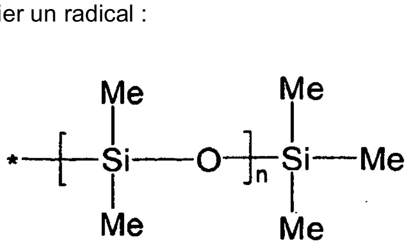

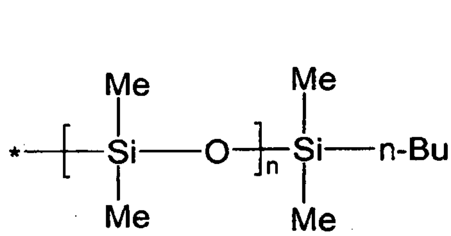

avec n=1 à 100; et encore plus particulièrement un radical :

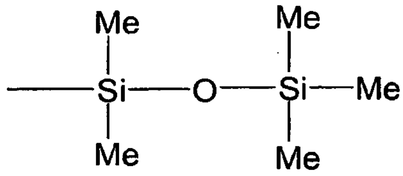

Les radicaux Rb et Rc, identiques ou différents, peuvent être des radicaux carbonés, notamment hydrocarbonés (alkyles), linéaires, ramifiés et/ou cycliques, saturés ou insaturés, comprenant 1 à 18 atomes de carbone, et pouvant comprendre 1 à 8 hétéroatomes choisis parmi N, O, Si et S. Dans ces radicaux, la chaîne carbonée peut être interrompue par le/les hétéroatomes et/ou peut comprendre un substituant comprenant ledit/lesdits hétéroatomes; les hétéroatomes peuvent notamment former un ou plusieurs groupements -SiO- (ou -OSi)-.
Ainsi, ils peuvent être de structure $-(CH_2)m-CH_3$ avec m= 0 à 17, notamment 1 à 12, voire 2 à 5; en particulier, Rb et/ou Rc peuvent être méthyl, éthyle, propyle ou butyle;
Ils peuvent également être de structure $-O-(CH_2)m'-CH_3$ avec m'= 0 à 5, notamment 1 à 4, et en particulier méthoxy ou éthoxy.
Ils peuvent aussi être de structure $-O-(CH_2)x-O-(CH_2)z-CH_3$ ou $-O-(CH_2)x-O-(CH_2)y-O-(CH_2)z-CH_3$, avec x = 1 à 10, de préférence 2; y=1 à 10 de préférence 2, et z= 0 à 10, de préférence 0 ou 1.
Ils peuvent encore être de structure :

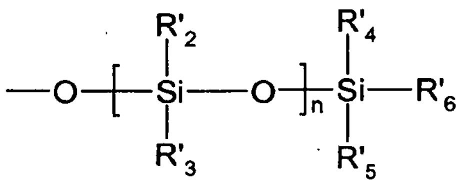

avec n étant compris entre 0 et 100, notamment entre 1 et 80, voire 2 à 20;
et R'2 à R'6 étant, indépendamment les uns des autres, de préférence, des radicaux alkyle, ayant 1 à 12 atomes de carbone, notamment 1 à 6 atomes de carbone; en particulier R'2 à R'6 peuvent être choisis parmi méthyle, éthyle, propyle, butyle.

Lorsqu'ils sont de formule (III), les radicaux R et/ou R' sont de préférence choisis parmi les radicaux suivants :

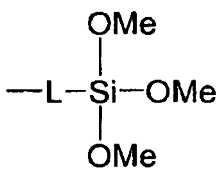

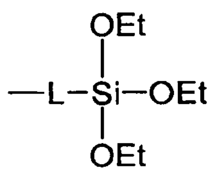

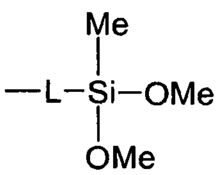

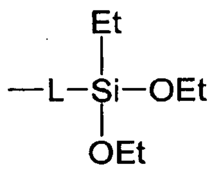

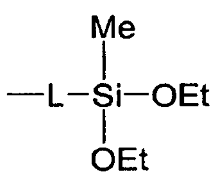

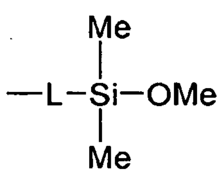

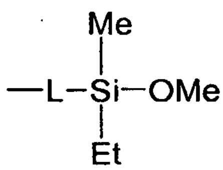

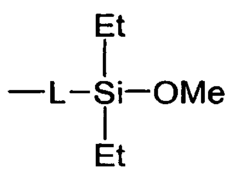

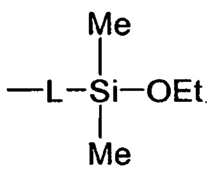

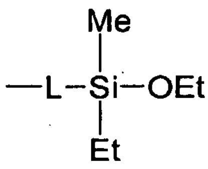

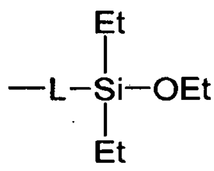

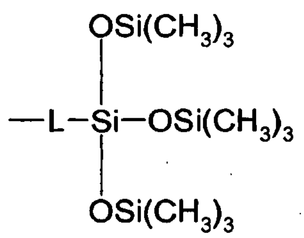

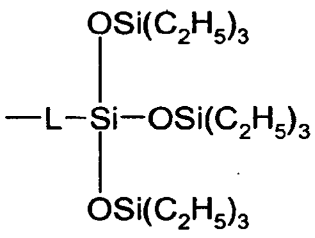

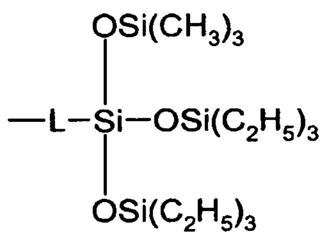

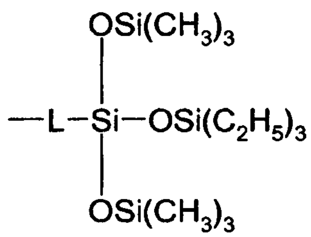

et également ceux de formule :

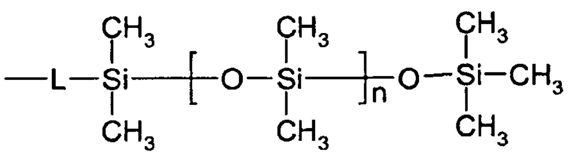

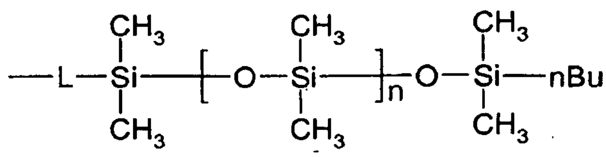

avec n variant de 0 à 100 et en particulier

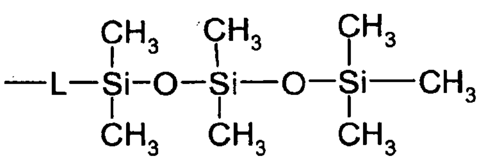

et

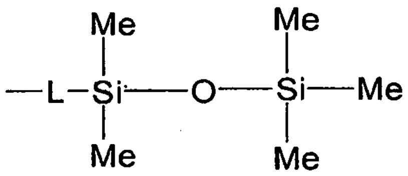

et

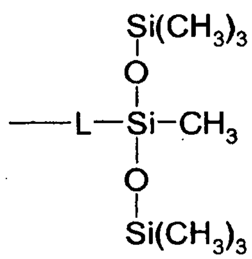

ou encore

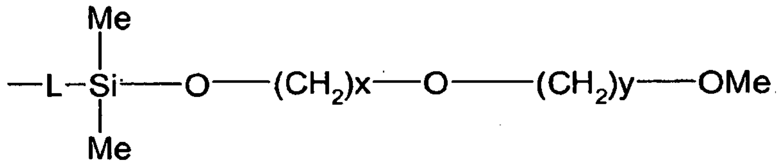

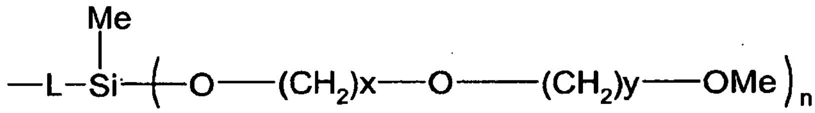 avec n=2

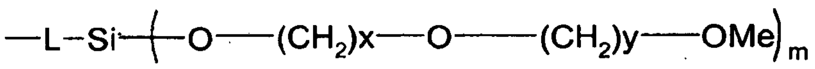 avec m=3

dans lesquelles x = 1 à 10, de préférence 2; et y = 1 à 10, de préférence 2;
et L étant tel que défini ci-dessus.
De préférence, dans ces formules, L est un radical alkylène en C1-C8, linéaire ou ramifié, notamment méthylène, éthylène, propylène, butylène et notamment n-butylène, octylène ou de formule $-CH_2-CH(CH_3)-$.

Dans un mode de réalisation particulier, R et R', identiques ou différents, sont tous les deux de formule (III).

Dans un autre mode de réalisation, l'un des radicaux R ou R' représente un radical alkyle en $C_1$ à $C_{30}$, linéaire, ramifié et/ou cyclique, saturé ou insaturé, et comprenant éventuellement 1 à 3 hétéroatomes choisis parmi O, S, F et N. Ceci s'avère particulièrement avantageux pour conférer un caractère universel aux composés de formule (I), c'est-à-dire leur permettre de texturer à la fois des milieux carbonés polaires ou apolaires, des milieux siliconés linéaires ou cycliques, des huiles mixtes c'est-à-dire carbonées partiellement siliconées, ainsi que leurs mélanges.
La chaîne carbonée peut être interrompue par le/les hétéroatomes et/ou peut comprendre un substituant comprenant ledit/lesdits hétéroatomes, notamment sous forme de groupement carbonyle (-CO-), d'un ou plusieurs radicaux hydroxy (-OH), et/ou d'un radical ester -COOR" avec R" = radical alkyle, linéaire ou ramifié, ayant 1 à 8 atomes de carbone.

Ainsi, ledit radical R ou R' peut être un groupement choisi parmi :

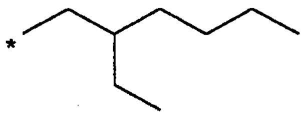

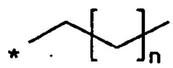

n = 16

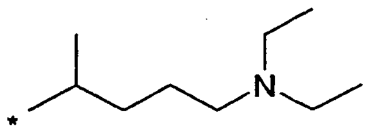

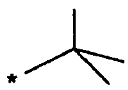

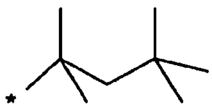

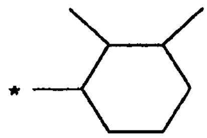

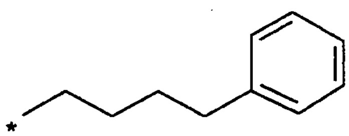

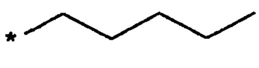

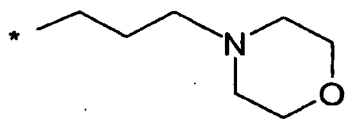

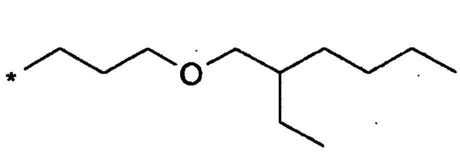

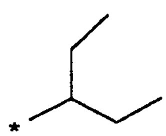

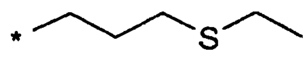

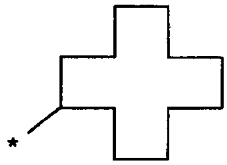
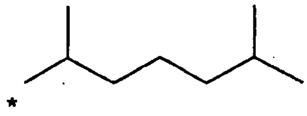
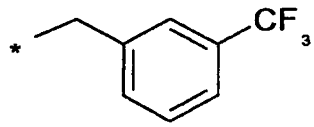

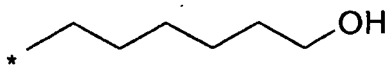
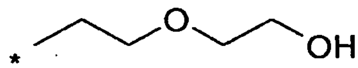

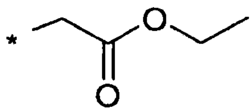
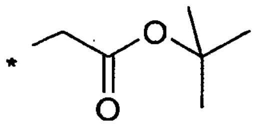
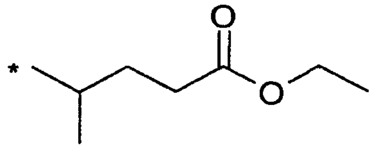

avec * ayant la définition donnée ci-dessus.

Dans un mode de réalisation préféré, R ou R' représente un radical alkyle ramifié, notamment monoramifié, de préférence non cyclique, saturé ou insaturé, comprenant 3 à 16 atomes de carbone, notamment 4 à 12, voire 4 à 8 atomes de carbone, et comprenant éventuellement 1 à 3 hétéroatomes choisis parmi O, S, F et/ou N, de préférence O et/ou N.

En particulier, R ou R' peuvent être des radicaux tertio-butyle ou 2-éthylhexyle ou de formule :

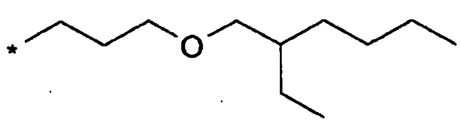

Lorsque le composé de formule (I) comprend un radical R qui est un radical alkyle, et donc un radical R' qui est de formule (III), le rapport entre $n_R$ et $n_{R'}$ est de préférence compris entre 5/95 et 95/5, par exemple entre 10/90 et 90/10, en particulier entre 40/60 et 85/15, notamment entre 50/50 et 80/20, voire entre 60/40 et 75/25;
avec $n_R$ étant le nombre de moles d'amine $NH_2$-R et $n_{R'}$ étant le nombre de moles d'amine $NH_2$-R' utilisées pour préparer le composé de formule (I).

Les composés selon l'invention peuvent se présenter sous forme de sels et/ou d'isomères de composés de formule (I).

D'une manière générale, les composés de formule générale (I) selon l'invention peuvent être préparés comme décrit dans la demande FR2910809.

Les composés de type bis-urée siliconés décrits précédemment peuvent être en mélange avec d'autres composés bis-urées non siliconés. Les composés de bis-urée non siliconés peuvent, selon un premier aspect, répondre à la formule générale (II) suivante :

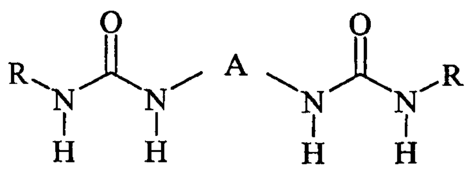
(II)

dans laquelle :

- A est un groupement de formule :

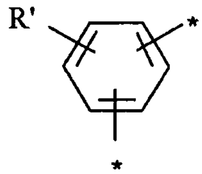

avec R' étant un radical alkyle $C_1$ à $C_4$ linéaire ou ramifié et les * symbolisant les points de rattachement du groupement A à chacun des deux atomes d'azote du reste du composé de formule générale (II), et

- R est un radical alkyle en $C_6$ à $C_{15}$, mono-ramifié, non cyclique, saturé ou insaturé et dont la chaîne hydrocarbonée est éventuellement interrompue par 1 à 3 hétéroatomes choisis parmi O, S et N, ou l'un de ses sels ou isomères.

Selon un mode de réalisation préféré de l'invention, le groupement figuré par A est un groupement de formule :

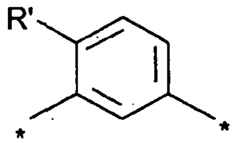

ou

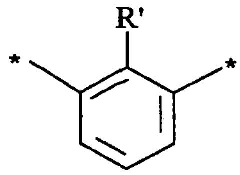

avec R' et les * étant tels que définis précédemment.

En particulier, R' peut être un groupement méthyle, et le groupement A est alors plus particulièrement un groupement de formule :

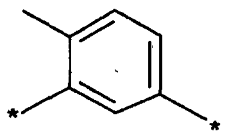

ou

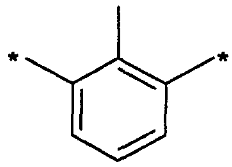

avec les * étant tel que défini précédemment.

Selon un premier mode de réalisation de l'invention, R peut être choisi parmi les radicaux mono-ramifiés de formule générale $C_nH_{2n+1}$, n étant un entier variant de 6 à 15, en particulier de 7 à 9 voire égal à 8.

Ainsi, les deux groupements R du composé de formule (II) peuvent figurer respectivement un groupement :

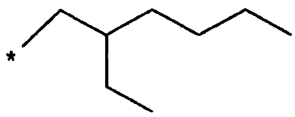

avec * symbolisant le point de rattachement de chacun des groupements R à chacun des atomes d'azote du reste du composé de formule générale (II).

Selon un deuxième mode de réalisation de l'invention, R peut être choisi parmi les radicaux mono-ramifiés de formule générale $C_{m-p}H_{2m+1-2}X_p$, p étant égal à 1, 2 ou 3, de préférence égal à 1, m étant un entier variant de 6 à 15, de préférence de 10 à 14, en particulier de 10 à 12, voire égal à 11 et X représentant les atomes de soufre et/ou d'oxygène, en particulier les atomes d'oxygène.

Plus particulièrement, R peut être un radical de formule $C_{m'}H_{2m'}X-(C_{p'}H_{2p'}X')_r-C_xH_{2x+1}$, dans laquelle X et X' sont indépendamment l'un de l'autre un atome d'oxygène ou de soufre, de préférence d'oxygène, r vaut 0 ou 1, m', p' et x sont des entiers tels que leur somme varie de 6 à 15, en particulier de 10 à 12, voire est égale à 11 et étant entendu qu'au moins une des chaînes carbonées $C_{m'}H_{2m'}$, $C_{p'}H_{2p'}$, ou $C_xH_{2x+1}$ est ramifiée.

De préférence c'est la chaîne $C_xH2_{x+1}$ qui est ramifiée, de préférence r égal 0, de préférence m' est un entier variant de 1 à 10, notamment de 2 à 6, en particulier égal à 3, et/ou de préférence x est un entier variant de 4 à 16, notamment de 6 à 12, en particulier égal à 8.

Ainsi, les deux groupements R du composé de formule (I) peuvent figurer respectivement un groupement :

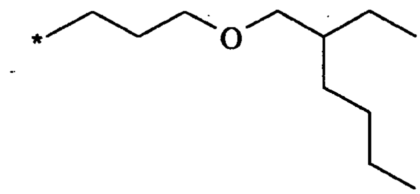

avec * symbolisant le point de rattachement de chacun des groupements R à chacun des atomes d'azote du reste du composé de formule générale (I).

De tels composés peuvent être présents dans les compositions selon l'invention en mélanges avec des isomères, notamment des isomères de position sur le groupement A, notamment dans des proportions 95/5 ou 80/20.

Comme il ressort des exemples ci-après, la présence de l'un ou l'autre de ses radicaux dans la molécule de formule générale (II) s'avère particulièrement avantageuse pour conférer un caractère universel, au sens de l'invention, aux dérivés bis-urée non siliconés correspondants.

A titre représentatif et non limitatif des composés convenant tout particulièrement à l'invention, on peut plus particulièrement citer les composés suivants, utilisés purs ou en mélange :

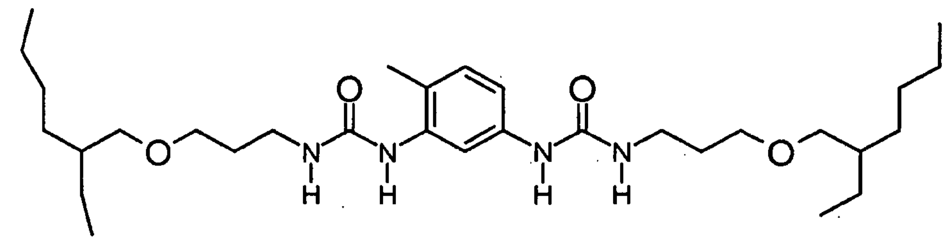

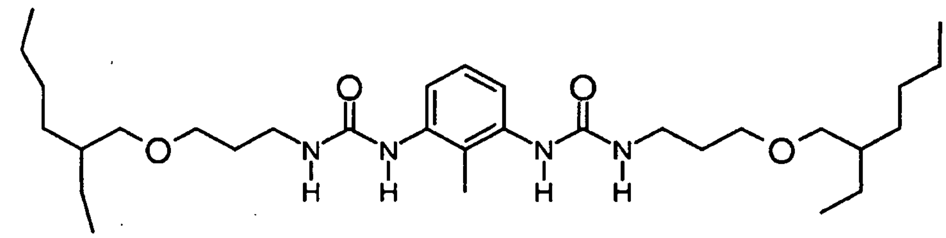

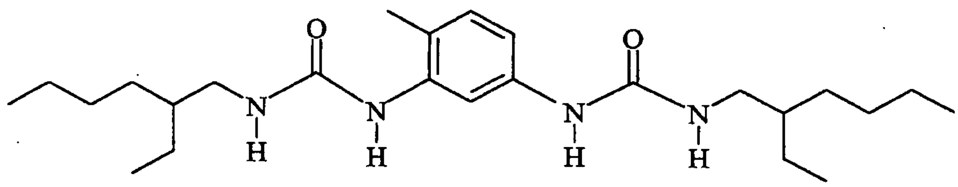

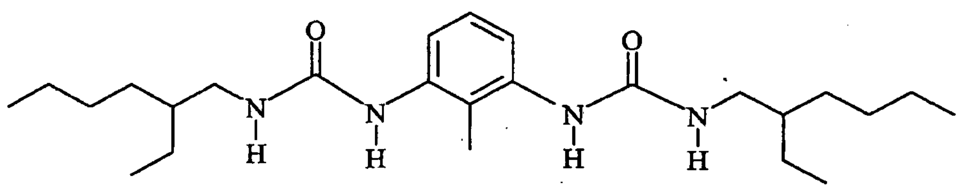

et leurs sels.

Selon un autre aspect de l'invention, les dérivés bis-urée non siliconés formule (III) suivante :

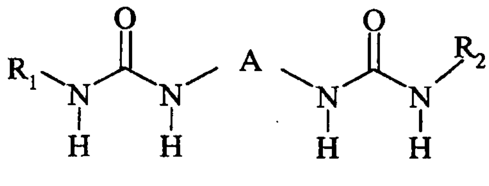

(III)

dans laquelle :

- A est un groupement de formule :

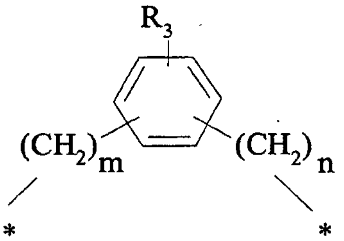

avec

- $R_3$ étant un atome d'hydrogène ou un radical alkyle en $C_1$ à $C_4$ linéaire ou ramifié,
- n et m étant, indépendamment l'un de l'autre, égaux à 0 ou 1, et
- * symbolisant le point de rattachement du groupement A aux deux atomes d'azote du reste du composé de formule générale (III),
- $R_1$ est un radical carboné en $C_3$ à $C_{15}$, ramifié, non cyclique, saturé ou insaturé et contenant éventuellement de 1 à 3 hétéroatomes choisis parmi O, S, F et N et/ou un carbonyle, et leurs combinaisons,
- $R_2$ est différent de $R_1$ et est choisi parmi les radicaux alkyles en $C_1$-$C_{24}$, saturé ou insaturé, linéaire, ramifié ou cyclique, contenant éventuellement de 1 à 3 hétéroatomes choisis parmi O, S, F et N, et éventuellement substitué par :
- 1, 2 ou 3 radicaux hydroxy,
- un radical ester (-$COOR_4$), avec $R_4$ étant un radical alkyle, linéaire ou ramifié, ayant de 1 à 8, notamment 1 à 6, voire 2 à 4 atomes de carbone ;
- un radical cyclique saturé, insaturé ou aromatique ayant de 5 à 12 atomes de carbone, en particulier un radical phényle éventuellement substitué par un ou plusieurs radicaux identiques ou différents choisis parmi les radicaux alkyle en $C_1$-$C_4$, trifluorométhyle, ou un dérivé morpholine, et/ou
- un ou plusieurs radicaux alkyles linéaires ou ramifiés en $C_1$-$C_4$,

ou l'un de ses sels ou isomères.

En particulier n et m sont égaux, et plus particulièrement égaux à zéro et $R_3$ est un radical $R'_3$, tel que défini ci-après. Ainsi, de manière préférée, A représente un groupement

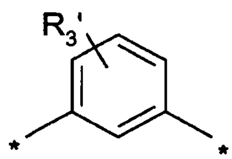

Avec $R_3$' étant un radical alkyle en $C_1$ à $C_4$ linéaire ou ramifié et * symbolisant les point de rattachement du groupement A aux deux atomes d'azote du reste du composé de formule générale (III).

Selon une variante de l'invention, le composé de formule générale (III) comprend à titre de A, au moins un groupement choisi parmi :

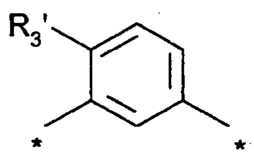

ou

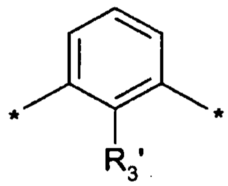

avec $R_3$' et étant tels que défini précédemment.
En particulier, $R_3$' peut être un groupement méthyle, et dans ce cas le groupement A représente un groupement

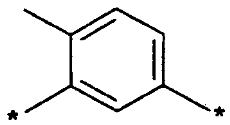

ou

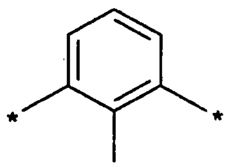

* étant tel que défini précédemment.
En particulier, les composés sont tels que A est un mélange de 2,4-tolylène et 2,6-tolylène notamment dans des proportions (isomère 2,4)/(isomère 2,6) variant de 95/5 à 80/20.

Selon un mode de réalisation de l'invention, le composé de formule générale (III) comprend à titre de $R_1$, un radical en $C_6$-$C_{15}$ ramifié.
Selon un mode de réalisation de l'invention, le composé de formule générale (III) comprend à titre de $R_1$, un groupement choisi parmi :

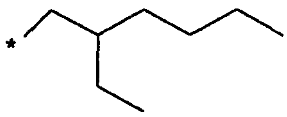

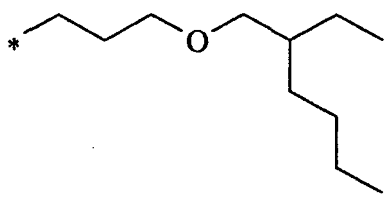

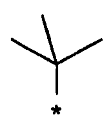

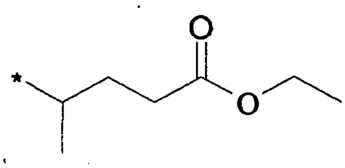

avec * symbolisant le point de rattachement du groupement $R_1$ à l'azote du reste du composé de formule générale (III). Comme il ressort des exemples ci-après, la présence de l'un et/ou l'autre de ses deux radicaux dans la molécule de formule générale (III) s'avère particulièrement avantageuse pour conférer un caractère universel au sens de l'invention, aux dérivés bis-urée asymétriques correspondants.

En ce qui concerne $R_2$ qui est différent de $R_1$, il peut être avantageusement choisi parmi les groupements suivants :

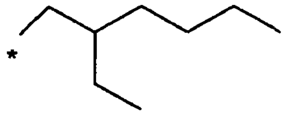

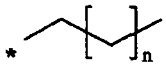

n = 16

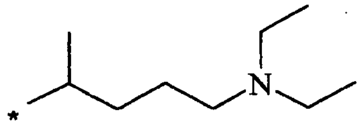

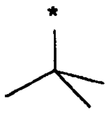

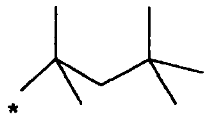

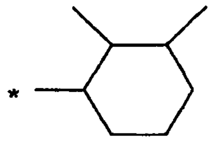

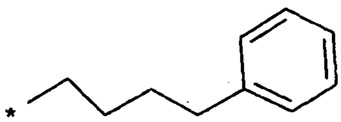

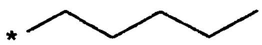

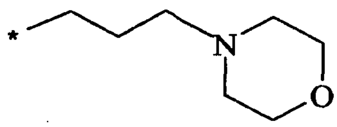

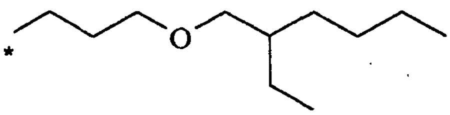

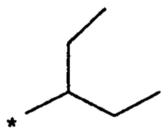

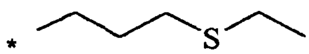

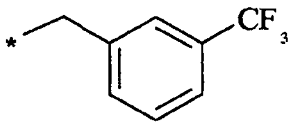

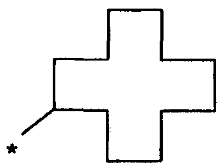

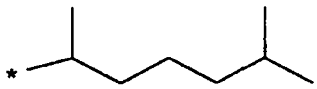

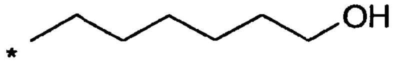

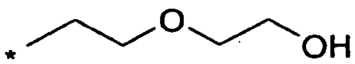

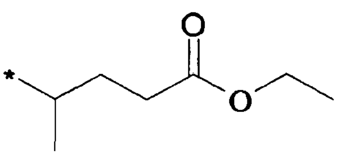

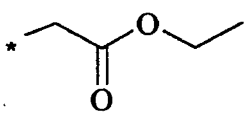

et

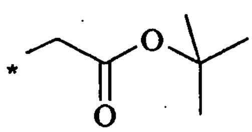

avec * symbolisant le point de rattachement du groupement $R_2$ à l'azote du reste du composé de formule générale (III).

D'une manière générale, les composés décrits peuvent être préparés comme décrit dans la demande FR2910809.

*Polymères blocs :*

On peut aussi utiliser comme agent rhéologique de phase grasse des polymères blocs greffés ou séquencés.

On peut notamment utiliser des copolymères blocs greffés ou séquencés comprenant au moins un bloc de type polyorganosiloxane et au moins un bloc d'un polymère radicalaire, comme les copolymères greffés de type acrylique/silicone qui peuvent être employés notamment lorsque le milieu non aqueux est siliconé.

On peut aussi utiliser des copolymères blocs greffés ou séquencés comprenant au moins un bloc de type polyorganosiloxane et au moins d'un polyéther. Le bloc polyorganopolysiloxane peut être notamment un polydiméthylsiloxane ou bien encore un poly alkyl($C_2$-$C_{18}$) méthyl siloxane ; le bloc polyéther peut être un poly alkylène en $C_2$-$C_{18}$, en particulier polyoxyéthylène et/ou polyoxypropylène. En particulier, on peut utiliser les diméthicones copolyol ou des alkyl ($C_2$-$C_{18}$) diméthicones copolyol tels que ceux vendu sous la dénomination "Dow Corning 3225C" par la société Dow Corning, les lauryl méthicones tels que ceux vendu sous la dénomination "Dow Corning Q2-5200 par la société "Dow Corning".

Comme copolymères blocs greffés ou séquencés, on peut citer aussi ceux comprenant au moins un bloc résultant de la polymérisation d'au moins un monomère éthylénique, à une ou plusieurs liaisons éthyléniques éventuellement conjuguées, comme l'éthylène ou les diènes tels que le butadiène et l'isoprène, et d'au moins un bloc d'un polymère vinylique et mieux styrénique. Lorsque le monomère éthylénique comporte plusieurs liaisons éthyléniques éventuellement conjuguées, les insaturations éthyléniques résiduelles après la polymérisation sont généralement hydrogénées. Ainsi, de façon connue, la polymérisation de l'isoprène conduit, après hydrogénation, à la formation de bloc éthylène-propylène, et la polymérisation de butadiène conduit, après hydrogénation, à la formation de bloc éthylène-butylène. Parmi ces polymères, on peut citer les copolymères séquencés, notamment de type "dibloc" ou "tribloc" du type polystyrène/polyisoprène (SI), polystyrène/polybutadiène (SB) tels que ceux vendus sous le nom de 'LUVITOL HSB' par BASF, du type polystyrène/copoly(éthylène-propylène) (SEP) tels que ceux vendus sous le nom de 'Kraton' par Shell Chemical Co ou encore du type polystyrène/copoly(éthylène-butylène) (SEB). En particulier, on peut utiliser le Kraton G1650 (SEBS), le Kraton G1651 (SEBS), le Kraton G1652 (SEBS), le Kraton G1657X (SEBS), le Kraton G1701X (SEP), le Kraton G1702X (SEP), le Kraton G1726X (SEB), le Kraton D-1101 (SBS), le Kraton D-1102 (SBS), le Kraton D-1107 (SIS). Les polymères sont généralement appelés des copolymères de diènes hydrogénés ou non.

On peut aussi utiliser les Gelled Permethyl 99A-750, 99A-753-59 et 99A-753-58 (mélange de tribloc et de polymère en étoile), Versagel 5960 de chez Penreco (tribloc + polymère en étoile) ; OS129880, OS129881 et OS84383 de chez Lubrizol (copolymère styrène/méthacrylate).

Comme copolymères blocs greffés ou séquencés comprenant au moins un bloc résultant de la polymérisation d'au moins un monomère éthylénique à une ou plusieurs liaisons éthyléniques et d'au moins un bloc d'un polymère acrylique, on peut citer les copolymères bi- ou triséquencés poly(méthylacrylate de méthyle)/polyisobutylène ou les copolymères greffés à squelette poly(méthylacrylate de méthyle) et à greffons polyisobutylène.

Comme copolymères blocs greffés ou séquencés comprenant au moins un bloc résultant de la polymérisation d'au moins un monomère éthylénique à une ou plusieurs liaisons éthyléniques et d'au moins un bloc d'un polyéther tel qu'un polylkylène en $C_2$-$C_{18}$ (polyéthyléné et/ou polyoxypropyléné notamment), on peut citer les copolymères bi- ou triséquencés polyoxyéthylène/polybutadiène ou polyoxyéthylène/polyisobutylène.

*Elastomères de silicone gélifiants de phase grasse*

On entend par « élastomère » un matériau solide souple, déformable ayant des propriétés viscoélastiques et notamment la consistance d'une éponge. Cet élastomère est formé de chaînes polymériques de haut poids moléculaire dont la mobilité est limitée par un réseau uniforme de points de réticulation.

Les organopolysiloxanes élastomères utilisés dans la composition selon l'invention sont de préférence partiellement ou totalement réticulés. Ils se présentent sous forme de particules. En particulier, les particules d'organopolysiloxane élastomère ont une taille allant de 0,1 à 500 µm, de préférence de 3 à 200 µm et mieux de 3 à 50 µm. Ces particules peuvent avoir toute forme et par exemple être sphériques, plates ou amorphes.

Quand ils sont inclus dans une phase huileuse, ces organopolysiloxanes élastomères se transforment, selon le taux de phase huileuse utilisé, en un produit d'aspect spongieux lorsqu'ils sont utilisés en présence de faibles teneurs en phase huileuse, ou en un gel homogène en présence de quantités de phase huileuse plus élevées. La gélification de la phase huileuse par ces élastomères peut être totale ou partielle.

Ainsi les élastomères de l'invention peuvent être véhiculés sous forme de gel anhydre constitué d'un organopolysiloxane élastomère et d'une phase huileuse. La phase huileuse utilisée lors de la fabrication du gel anhydre d'organopolysiloxane élastomère contient une ou plusieurs huiles liquides à températures ambiante (25°C) choisies parmi les huiles hydrocarbonées et/ou les huiles de silicone. Avantageusement, la phase huileuse est une phase liquide siliconée, contenant une ou plusieurs huiles choisies parmi les polydiméthylsiloxanes à chaîne linéaire ou cyclique, liquides à température ambiante comportant éventuellement une chaîne alkyle ou aryle pendante ou en bout de chaîne,

la chaîne alkyle ayant de 1 à 6 atomes de carbone.

Selon un mode de mise en oeuvre, les organopolysiloxanes élastomères utilisés selon l'invention peuvent être obtenus par réaction d'addition et de réticulation, en présence d'un catalyseur, de préférence un catalyseur du type platine, d'au moins :

- (i) un organopolysiloxane ayant deux groupements vinyliques en position $\alpha$-$\omega$ de la chaîne siliconée par molécule; et
- (ii) un organopolysiloxane ayant au moins deux atomes d'hydrogène liés à un atome de silicium par molécule.

Le premier organopolysiloxane (i) est choisi parmi les polydiméthylsiloxanes ; il s'agit de préférence d'un $\alpha$-$\omega$-diméthylvinyl polydiméthylsiloxane.

L'organopolysiloxane est de préférence dans un gel obtenu selon les étapes suivantes :

- (a) mélange du premier et second organopolysiloxanes (i) et (ii) ;
- (b) ajout d'une phase huileuse au mélange de l'étape (a) ;
- (c) polymérisation du premier et second organopolysiloxanes (i) et (ii) en phase huileuse en présence d'un catalyseur, de préférence d'un catalyseur de platine.

Selon un mode de mise en oeuvre, l'organopolysiloxane réticulé peut être obtenu par une réaction polymérique d'addition d'un organohydrogénopolysiloxane de formule (I) avec un organopolysiloxane de formule (II) et/ou une chaîne hydrocarbonée insaturée de formule (III).

Selon une variante, l'organopolysiloxane réticulé est obtenu par une réaction polymérique d'un organohydrogénopolysiloxane de formule (I) avec un organopolysiloxane de formule (II).

<u>Organohydrogénopolysiloxane de formule (I)</u>

L'organohydrogénopolysiloxane de formule (I) comprend au moins une unité structurale choisie dans le groupe composé d'une unité $SiO_2$, une unité $HSiO_{1,5}$, une unité $RSiO_{1,5}$, une unité RHSiO, une unité $R_2SiO$, une unité $R_3SiO_{0,5}$ et une unité $R_2HSiO_{0,5}$, le groupe R étant dans ces unités une chaîne hydrocarbonée monovalente comportant de 1 à 16 atomes de carbone pouvant être substitué ou non mais étant distinct d'un groupe aliphatique insaturé, et possédant en moyenne au moins 1,5 atomes d'hydrogène liés à un atome de silicium.

Le groupe R dans l'organohydrogénopolysiloxane de formule (I) peut être un groupe alkyle comportant de 1 à 16, de préférence de 10 à 16 atomes de carbone. Ce groupe R peut par exemple être un groupe méthyle, un groupe éthyle, un groupe propyle, un groupe lauryle, un groupe myristyle et un groupe palmityle.

Le groupe R dans l'organohydrogénopolysiloxane de formule (I) peut également être un groupe aryle tel qu'un groupe phényle ou tolyle.

Le groupe R toujours dans l'organohydrogénopolysiloxane de formule (I) peut également être une chaîne hydrocarbonée monovalente comprenant un groupe cycloalkyle tel que le cyclohexyle ou bien une chaîne hydrocarbonée substituée par un, deux ou plusieurs groupes choisis parmi un atome d'halogène tel que le chlore, le brome, le fluor et un groupe cyano, par exemple un groupe $\alpha$-trifluoropropyle ou chlorométhyle.

En particulier, on préfère que le groupe R représente au moins 30 % molaire de groupe méthyle et de 5 à 50 % molaire, de préférence de 10 à 40 % molaire en chaîne hydrocarbonée comportant de 10 à 16 atomes de carbone.

La chaîne hydrocarbonée peut alors avantageusement comporter au moins un groupe lauryle, voire la majorité des groupes R peuvent être des groupes lauryle.

L'organohydrogénopolysiloxane de formule (I) peut être linéaire, branché ou cyclique.

L'organohydrogénopolysiloxane de formule (I) contient de préférence de 2 à 50 et de façon encore plus préférée de 2 à 10 atomes d'hydrogène liés à un atome de silicium (Si-H). La teneur en atome d'hydrogène liés à un atome de silicium dans ce composé de formule (I) varie classiquement de 0,5 à 50 % molaire, et de façon encore plus préférée de 1 à 20 % molaire par rapport à la somme totale des atomes d'hydrogène et de tous les groupes organiques liés à un atome de silicium.

<u>Organopolysiloxane de formule (II)</u>

L'organopolysiloxane de formule (II) comprend au moins une unité structurale choisie dans le groupe composé d'une unité $SiO_2$, d'une unité $(CH_2{=}CH)SiO_{1,5}$, une unité $RSiO_{1,5}$, une unité $R(CH_2{=}CH)SiO$, une unité $R_2SiO$, une unité $R_3SiO_{0,5}$ et une unité $R_2(CH_2{=}CH)SiO_{0,5}$, le groupe R étant tel que défini dans la formule (I) et possédant en moyenne au moins 1,5 groupes vinyliques liés à un atome de silicium.

Ce composé contient de préférence de 2 à 50 groupes vinyliques liés à un atome de silicium. Le nombre moyen de groupes vinyliques liés à un atome de silicium varie de préférence de 2 à 10, et de façon encore plus préférée de 2 à 5.

De préférence, au moins 30 % molaire des groupes R sont des groupes méthyle et 5 à 50 % molaire, de préférence 10 à 40 % molaire des groupes R sont une chaîne hydrocarbonée comportant de 10 à 16 atomes de carbone.
L'organopolysiloxane de formule (II) peut être linéaire, branché ou cyclique.
La teneur en groupe vinylique dans le composé de formule (II) varie de préférence entre 0,5 et 50 % molaire, de façon encore plus préférée de 1 à 20 % molaire par rapport à tous les groups organiques liés à un atome de silicium.

<u>Chaîne hydrocarbonée insaturée de formule (III) optionnelle</u>

La chaîne hydrocarbonée insaturée de formule (III) répond à la formule suivante :

$$C_mH_{2m-1}(CH_2)_xC_mH_{2m-1}$$

dans laquelle

m est un entier variant de 2 à 6 et
x est un entier au moins égal à 1.
x est de préférence un entier variant de 1 à 20.

A titre d'exemple de ce composé de formule (III), on peut citer le pentadiène, l'héxadiène, l'heptadiène, l'octadiène, le pentadécadiène, l'héptadécadiène et le pentatriacontadiène.
Les réactions polymériques d'addition sont décrites en détail dans le document US 2004/0234477.
Parmi les organopolysiloxanes réticulés, on préfère les polyalkyl diméthylsiloxanes réticulés. Par polyalkyl diméthylsiloxane, on entend un organopolysiloxane linéaire de formule (IV)

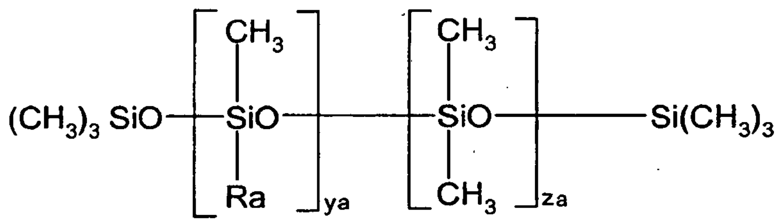

(IV)

comportant des greffons liés de façons monovalente ou divalente de formule (V)

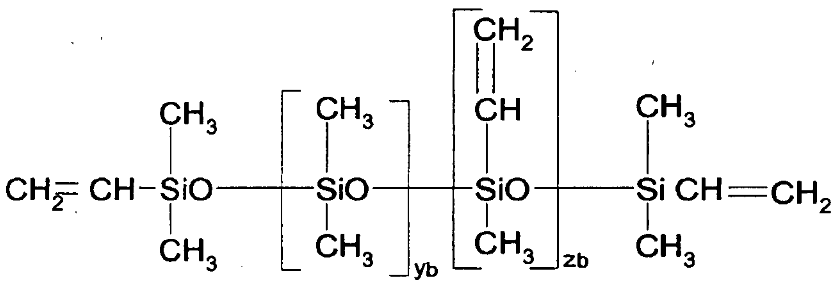

(V)

dans lesquelles :

Ra est un groupe alkyle comportant de 10 à 16 atomes de carbone, et peut être de façon préférée un groupe lauryle,
ya est un entier compris de 1 à 100;
za est un entier compris de 1 à 100,
yb est un entier compris de 1 à 100,
zb est un entier compris de 1 à 100.

Par "lié de façon divalente", on entend lié à deux organopolysiloxane de formule (IV) distincts. Il s'agit autrement dit d'un pont entre deux chaînes linéaires telles que définies par la formule (IV).

Comme élastomères non-émulsionnants utilisables selon l'invention, on utilise de préférence les copolymères dimethicone/vinyldimethicone (nom INCI : Dimethicone/Vinyldimethicone crosspolymer), et les copolymères vinyldimethicone/alkyl dimethicone, comme les copolymères vinyldimethicone/lauryl dimethicone (nom INCI: Vinyl Dimethicone/Lauryl Dimethicone Crosspolymer).

Comme élastomères non-émulsionnants utilisables selon l'invention, on peut citer :

- ceux de nom INCI Dimethicone/Vinyldimethicone Crosspolymer (and) C12-14 Pareth-12 : comme ceux vendus sous le nom "DC 9509" par la société Dow Corning,
- ceux de nom INCI Dimethicone/Vinyldimethicone Crosspolymer : comme ceux vendus sous le nom "DC9505", ou "DC 9506" par la société Dow Corning, ceux de nom INCI Cyclomethicone (and) Dimethicone/Vinyldimethicone Crosspolymer : comme ceux vendus sous KSG-15® par Shin-Etsu, Methyl Trimethicone (and) Dimethicone/Vinyldimethicone Crosspolymer : comme ceux vendus par Shin-Etsu sous KSG-1610®,
- ceux de nom INCI Dimethicone (and) Dimethicone/Vinyldimethicone Crosspolymer : comme ceux vendus sous KSG-16® par Shin-Etsu, Isododecane (and) Dimethicone/Vinyldimethicone Crosspolymer : comme ceux vendus sous USG-106® par Shin-Etsu,
- ceux de nom INCI : Vinyl Dimethicone/ Lauryl Dimethicone Crosspolymer : KSG-41® (dans une huile minérale), KSG-42® (dans l'isododecane), KSG-43® (dans la triethylhexanoine) et KSG-44® (dans le squalane), vendus par Shin-Etsu.

Comme élastomère non émulsionnant, on peut aussi citer des élastomères de silicone non émulsionnants sphériques sous forme de poudre d'organopolysiloxane réticulé élastomère enrobée de résine de silicone, notamment de résine silsesquioxane, comme décrit par exemple dans le brevet US5538793. De tels élastomères sont vendus sous les dénominations "KSP-100", "KSP-101", "KSP-102", "KSP-103", KSP-104", "KSP-105" par la société Shin Etsu.

D'autres organopolysiloxanes réticulés élastomères sous forme de poudres sphériques peuvent être des poudres de silicone hybride fonctionnalisé par des groupes fluoroalkyle, notamment vendues sous la dénomination "KSP-200" par la société Shin Etsu ; des poudres de silicones hybrides fonctionnalisées par des groupes phényl, notamment vendues sous la dénomination "KSP-300" par la société Shin Etsu.

On peut également utiliser dans les compositions selon l'invention des élastomères de silicones avec groupement MQ, tels que ceux vendus par la Société Wacker sous les dénominations Belsil RG100, Belsil RPG33 et préférenciellement RG80. Ces élastomères particuliers, lorsqu'ils sont en association avec les résines selon l'invention, peuvent permettre d'améliorer les propriétés de non transfert des compositions les comprenant.

Peuvent également être cités à titre d'agents épaississants lipophiles, encore appelés agents gélifiants,utilisables dans une composition de l'invention, l'éthylcellulose comme celle vendue sous la dénomination Ethocel® par la société DOW CHEMICAL ; les polycondensats de type polyamide résultant de la condensation entre un acide dicarboxylique comprenant au moins 32 atomes de carbone et un alkylène diamine et en particulier l'éthylène diamine, dans lequel le polymère comprend au moins un groupe acide carboxylique terminal estérifié ou amidifié avec au moins un mono alcool ou une mono amine comprenant de 12 à 30 atomes de carbone linéaires et saturés, et en particulier, les copolymères d'éthylène diamine/dilinoléate de stéaryle tel que celui commercialisé sous la dénomination Uniclear 100 VG® par la société ARIZONA CHEMICAL ; les polymères et les copolymères réticulés d'acrylamide ; es galactommananes comportant de un à six, et en particulier de deux à quatre, groupes hydroxyle par ose, substitués par une chaîne alkyle saturée ou non, comme la gomme de guar alkylée par des chaînes alkyle en $C_1$ à $C_6$, et en particulier en $C_1$ à $C_3$, et leurs mélanges.

Comme agents épaississants lipophiles convenant à l'invention peuvent également être cités les copolymères du type polystyrène/polyalkylène, et plus particulièrement les copolymères séquencés de type « dibloc », « tribloc » ou « radial » du type polystyrène/polyisoprène, polystyrène/polybutadiène tels que ceux commercialisés sous la dénomination Luvitol HSB® par la société BASF, du type polystyrène/copoly(éthylène-propylène) tels que ceux commercialisés sous la dénomination de Kraton® par la société KRATON POLYMERS ou encore du type polystyrène/copoly(éthylène-butylène), les mélanges de copolymères tribloc et radial (en étoile) dans l'isododécane tels que ceux commercialisé par la société PENRECO sous la dénomination Versagel® comme par exemple le mélange de copolymère tribloc butylène/éthylène/styrène et de copolymère étoile éthylène/propylène/styrène dans l'isododécane (Versagel M 5960).

Parmi les épaississants lipophiles pouvant être utilisés dans une composition cosmétique de l'invention, on peut encore citer les esters de dextrine et d'acide gras, tels que les palmitates de dextrine, notamment tels que ceux commercialisés sous les dénominations Rheopearl TL® ou Rheopearl KL® par la société CHIBA FLOUR, les huiles végétales hydrogénées, telles que l'huile de ricin hydrogénée, les alcools gras, en particulier de $C_8$ à $C_{26}$, et plus particulièrement de $C_{12}$ à $C_{22}$, comme par exemple, l'alcool mysritylique, l'alcool cétylique, l'alcool stéarylique, l'alcool béhénylique, ou encore l'alcool polyvinylique.

Peuvent encore être cités comme agents épaississants, les polymères poly(métha)crylates de glycéryle ; la polyvinylpyrrolidone ; les polymères associatifs, et notamment les polyuréthanes associatifs ; les alkyléther de polysaccharides (notamment dont le groupe alkyle comporte de 1 à 24 atomes de carbones, de préférence de 1 à 10, mieux de 1 à 6, et plus spécialement de 1 à 3) tels que ceux décrits dans le document EP-A-898958.

Cires

La composition selon l'invention peut comprendre en outre au moins une cire.
Par cire au sens de la présente invention, on entend un composé lipophile, solide à température ambiante (25 °C), à changement d'état solide/liquide réversible, ayant un point de fusion supérieur ou égal à 30 °C pouvant aller jusqu'à 120 °C.
Le point de fusion de la cire peut être mesuré à l'aide d'un calorimètre à balayage différentiel (D.S.C.), par exemple le calorimètre vendu sous la dénomination DSC 30 par la société METLER.
Les cires peuvent être hydrocarbonées, fluorées et/ou siliconées et être d'origine végétale, minérale, animale et/ou synthétique. En particulier, les cires présentent une température de fusion supérieure à 25 °C et mieux supérieure à 45 °C.
La cire ou le mélange de cires est présente en une teneur au moins égale à 7% en poids. De préférence, elle est présente en une teneur allant de 10 à 40 % en poids par rapport au poids total de la composition, mieux de 15 à 35 % et encore mieux de 16 à 30% en poids.
De préférence, les cires sont choisies parmi la cire d'abeilles, la cire de lanoline, et les cires d'insectes de Chine; la cire de riz, la cire de Carnauba, la cire d'Ouricurry, la cire d'Alfa, la cire de fibres de liège, la cire de canne à sucre, la cire du Japon et la cire de sumac; la cire de montan, les cires microcristallines, les paraffines; les cires de polyéthylène, les cires obtenues par la synthèse de Fisher-Tropsch, les cires obtenues par hydrogénation catalytique d'huiles animales ou végétales ayant des chaînes grasses, linéaires ou ramifiées, en C8-C32, les cires fluorées, la cire obtenue par hydrogénation d'huile d'olive estérifiée avec l'alcool stéarylique, les cires obtenues par hydrogénation d'huile de ricin estérifiée avec l'alcool cétylique et les cires collantes.
On peut notamment utiliser les cires hydrocarbonées comme la cire d'abeilles, la cire de lanoline, et les cires d'insectes de Chine; la cire de riz, la cire de Carnauba, la cire d'Ouricurry, la cire d'Alfa, la cire de fibres de liège, la cire de canne à sucre, la cire du Japon et la cire de sumac; la cire de montan, les cires microcristallines, les paraffines; les cires de polyéthylène, les cires obtenues par la synthèse de Fisher-Tropsch et les copolymères cireux ainsi que leurs esters.
On peut aussi citer les cires obtenues par hydrogénation catalytique d'huiles animales ou végétales ayant des chaînes grasses, linéaires ou ramifiées, en C8-C32.
Parmi celles-ci, on peut notamment citer l'huile de jojoba hydrogénée, l'huile de tournesol hydrogénée, l'huile de ricin hydrogénée, l'huile de coprah hydrogénée et l'huile de lanoline hydrogénée, le tétrastéarate de di-(triméthylol-1,1,1 propane) vendu sous la dénomination "HEST 2T-4S" par la société HETERENE, le tétrabéhénate de di-(triméthylol-1,1,1 propane) vendue sous la dénomination HEST 2T-4B par la société HETERENE.
On peut encore citer les cires fluorées.
On peut également utiliser la cire obtenue par hydrogénation d'huile d'olive estérifiée avec l'alcool stéarylique vendue sous la dénomination "PHYTOWAX Olive 18 L 57" ou bien encore les cires obtenues par hydrogénation d'huile de ricin estérifiée avec l'alcool cétylique vendus sous la dénomination "PHYTOWAX ricin 16L64 et 22L73", par la société SOPHIM. De telles cires sont décrites dans la demande FR-A- 2792190.
On peut également citer :

- les cires de silicone, en particulier les polysiloxanes linéaires substitués; on peut citer, par exemple, les cires de silicone polyéther, les alkyl ou alkoxy-diméthicones ayant de 16 à 45 atomes de carbone, les alkyl méthicones comme la $C_{30}$-$C_{45}$ alkyl méthicone vendue sous la dénomination commerciale « AMS C 30 » par DOW CORNING,
- les huiles hydrogénées concrètes à 25 °C telles que l'huile de ricin hydrogénée, l'huile de jojoba hydrogénée, l'huile de palme hydrogénée, le suif hydrogéné, l'huile de coco hydrogénée et les esters gras concrets à 25 °C comme le stéarate d'alkyle en $C_{20}$-$C_{40}$ vendu sous la dénomination commerciale « KESTER WAX K82H » par la société KOSTER KEUNEN,
- et/ou leurs mélanges.

Selon un mode de réalisation, la cire présente dans la composition selon l'invention peut être totalement ou partiellement sous forme de poudre, notamment micronisée, pour faciliter sa mise en oeuvre dans la préparation de la composition cosmétique.
Parmi les cires utilisables sous forme de poudre, on peut notamment citer les microbilles de cire de Carnauba vendues sous la dénomination Microcare 350® par la société Micro Powders et les microbilles de cire de paraffine vendues sous la dénomination Microease 1145® par la société Micro Powders. De telles cires additionnelles micronisées permettent notamment d'améliorer les propriétés lors de l'application de la composition sur la peau.

**Agents gélifiants hydrophiles :**

La composition selon l'invention peut comprendre en outre au moins un agent gélifiant hydrophile, appelé aussi épaississant hydrophile par la suite.

Ces épaississants peuvent être utilisés seuls ou en association. Ces épaississants peuvent être notamment choisis parmi les gommes et les polymères cellulosiques.

Par épaississant hydrophile, on entend un agent épaississant soluble ou dispersible dans l'eau.

Comme épaississants hydrophiles, on peut citer en particulier les polymères épaississants hydrosolubles ou hydrodispersibles. Ceux-ci peuvent notamment être choisis parmi :

- la polyvinylpyrrolidone,
- l'alcool polyvinylique,
- les polymères carboxyvinyliques modifiés ou non, tels que les produits commercialisés sous les dénominations Carbopol (nom CTFA : carbomer) par la société Goodrich ;
- les homo- ou copolymères d'acides acrylique ou méthacrylique ou leurs sels et leurs esters et en particulier les produits vendus sous les dénominations VERSICOL F® ou VERSICOL K® ou Salcare SC95 par la société ALLIED COLLOID, UTRAHOLD 8® par la société CIBA-GEIGY, les polyacrylates et polyméthacrylates tels que les produits vendus sous les dénominations de Lubrajel et Norgel par la société GUARDIAN ou sous la dénomination Hispagel par la société HISPANO CHIMICA, les acides polyacryliques de type SYNTHALEN K ;
- les polyacrylamides ;
- les copolymères d'acide acrylique et d'acrylamide vendus sous la forme de leur sel de sodium sous les dénominations RETEN® par la société HERCULES, le polyméthacrylate de sodium vendu sous la dénomination DARVAN N°7® par la société VANDERBILT, les sels de sodium d'acides polyhydroxycarboxyliques vendus sous la dénomination HYDAGEN F® par la société HENKEL;
- les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société CLARIANT sous la dénomination « Hostacerin AMPS » (nom CTFA : ammonium polyacryldimethyltauramide) ;
- les copolymères anioniques réticulés d'acrylamide et d'AMPS, se présentant sous la forme d'une émulsion E/H, tels ceux commercialisés sous le nom de SEPIGEL 305 (nom C.T.F.A. : Polyacrylamide / C13-14 Isoparaffin / Laureth-7) et sous le nom de SIMULGEL 600 (nom C.T.F.A. : Acrylamide / Sodium acryloyldimethyltaurate copolymer / Isohexadecane / Polysorbate 80) par la société SEPPIC ;
- les copolymères acide polyacryliques/acrylates d'alkyle de type PEMULEN ;
- les biopolymères polysaccharidiques comme la gomme de xanthane, la gomme de guar, la gomme arabique, la gomme de caroube, la gomme d'acacia, les scléroglucanes, les dérivés de chitine et de chitosane, les carraghénanes, les gellanes, les alginates, les celluloses telles que la cellulose microcristalline, la carboxyméthylcellulose, l'hydroxyméthylcellullose, l'hydroéthylcellulose et l'hydroxypropylcellulose ;
- les silices pyrogénées hydrophiles obtenues par hydrolyse à haute température d'un composé volatil du silicium dans une flamme oxhydrique, produisant une silice finement divisée. Les silices hydrophiles présentent un nombre important de groupements silanol à leur surface. De telles silices hydrophiles sont par exemple commercialisées sous les dénominations "AEROSIL 130®", "AEROSIL 200®", "AEROSIL 255®", "AEROSIL 300®", "AEROSIL 380®" par la société Degussa; "CAB-O-SIL HS-5®", "CAB-O-SIL EH-5®", "CAB-O-SIL LM-130®", "CAB-O-SIL MS-55®", "CAB-O-SIL M-5®" par la société Cabot. Elles présentent de préférence une taille de particules pouvant être nanométrique à micrométrique, par exemple allant d'environ de 5 à 200 nm;
- les argiles hydrophiles ;
- les polymères associatifs comme le PEG-150/STEARYL ALCOHOL/SMDI COPOLYMER vendu sous le nom Aculyn 46 par Rohm & Haas, ou le STEARETH-100/PEG-136/HDI COPOLYMER vendu sous le nom Rhéolate FX 1100 par Elementis) ;
- et leurs mélanges.

L'épaississant hydrophile peut être choisi parmi les polymères associatifs. Par "polymère associatif" au sens de la présente invention, on entend tout polymère amphiphile comportant dans sa structure au moins une chaîne grasse et au moins une portion hydrophile. Les polymères associatifs conformes à la présente invention peuvent être anioniques, cationiques, non-ioniques ou amphotères.

Parmi les polymères anioniques associatifs, on peut citer ceux comportant au moins un motif hydrophile, et au moins un motif éther d'allyle à chaîne grasse, plus particulièrement parmi ceux dont le motif hydrophile est constitué par un monomère anionique insaturé éthylénique, plus particulièrement par un acide carboxylique vinylique et tout particulièrement par un acide acrylique, un acide méthacrylique ou leurs mélanges, et dont le motif éther d'allyle à chaîne grasse correspond au monomère de formule (I) suivante :

$$CH_2 = C(R')CH_2OB_nR \qquad (I)$$

dans laquelle R' désigne H ou $CH_3$, B désigne le radical éthylèneoxy, n est nul ou désigne un entier allant de 1 à 100, R désigne un radical hydrocarboné choisi parmi les radicaux alkyl, arylalkyle, aryle, alkylaryle, cycloalkyle, comprenant 8 à 30 atomes de carbone, de préférence 10 à 24, et plus particulièrement encore de 12 à 18 atomes de carbone.
Des polymères amphiphiles anioniques de ce type sont décrits et préparés, selon un procédé de polymérisation en émulsion, dans le brevet EP-0 216 479.
Comme polymères anioniques associatifs, on peut citer également les polymères anioniques comportant au moins un motif hydrophile de type acide carboxylique insaturé oléfinique, et au moins un motif hydrophobe exclusivement de type ester d'alkyl ($C_{10}$-$C_{30}$) d'acide carboxylique insaturé. On peut citer à titre d'exemple les polymères anioniques décrits et préparés, selon les brevets US-3 915 921 et 4 509 949.
Comme polymères associatifs cationiques, on peut citer les dérivés de cellulose quaternisée et les polyacrylates à groupements latéraux aminés.
Les polymères associatifs non-ioniques peuvent être choisis parmi :

- les celluloses modifiées par des groupements comportant au moins une chaîne grasse comme par exemple les hydroxyéthylcelluloses modifiées par des groupements comportant au moins une chaîne grasse tels que des groupes alkyle, notamment en $C_8$-$C_{22}$ , arylalkyle, alkylaryle, telles que le NATROSOL PLUS GRADE 330 CS (alkyles en $C_{16}$) vendu par la société AQUALON,
- les celluloses modifiées par dés groupes polyalkylène glycol éther d'alkyl phénol,
- les guars tels que l'hydroxypropyl guar, modifiés par des groupements comportant au moins une chaîne grasse telle qu'une chaîne alkyle,
- les copolymères de vinyl pyrrolidone et de monomères hydrophobes à chaîne grasse ;
- les copolymères de méthacrylates ou d'acrylates d'alkyles en $C_1$-$C_6$ et de monomères amphiphiles comportant au moins une chaîne grasse,
- les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle,
- les polyuréthanes associatifs,
- leurs mélanges.

De préférence, le polymère associatif est choisi parmi les polyuréthanes associatifs. Les polyuréthannes associatifs sont des copolymères séquencés non ioniques comportant dans la chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.
En particulier, ces polymères comportent au moins deux chaînes lipophiles hydrocarbonées, ayant de $C_6$ à $C_{30}$ atomes de carbone, séparées par une séquence hydrophile, les chaînes hydrocarbonées peuvent être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile. Les polyuréthanes associatifs peuvent être séquencés sous forme de tribloc ou multibloc. Les séquences hydrophobes peuvent donc être à chaque extrémité de la chaîne (par exemple : copolymère tribloc à séquence centrale hydrophile) ou réparties à la fois aux extrémités et dans la chaîne (copolymère multiséquencé par exemple). Ces polymères peuvent être également en greffons ou en étoile. De préférence, les polyuréthanes associatifs sont des copolymères triblocs dont la séquence hydrophile est une chaîne polyoxyéthylénée comportant de 50 à 1 000 groupements oxyéthylénés. En général les polyuréthannes associatifs comportent une liaison uréthane entre les séquences hydrophiles, d'où l'origine du nom.
A titre d'exemple, des polymères associatifs utilisables dans l'invention, on peut citer le polymère $C_{16}$-$OE_{120}$-$C_{16}$ de la société SERVO DELDEN (sous le nom SER AD FX1100, molécule à fonction uréthanne et poids moléculaire moyen en poids de 1300), OE étant un motif oxyéthyléné. Comme polymère associatif, on peut aussi utiliser aussi le Rhéolate 205 à fonction urée vendu par la société RHEOX ou encore le Rhéolate 208 ou 204 ou encore le Rhéolate FX 1100 par Elementis. Ces polyuréthannes associatifs sont vendus sous forme pure. Le produit DW 1206B de chez RHOM & HAAS à chaîne alkyle en $C_{20}$ et à liaison uréthane, vendu à 20 % en matière sèche dans l'eau, peut aussi être utilisé.
On peut aussi utiliser des solutions ou dispersions de ces polymères notamment dans l'eau ou en milieu hydroalcoolique. A titre d'exemple, de tels polymères on peut citer, le SER AD FX1010, le SER AD FX1035 et le SER AD 1070 de la société SERVO DELDEN, le Rhéolate 255, le Rhéolate 278 et le Rhéolate 244 vendus par la société RHEOX. On peut aussi utiliser le produit Aculyn 46, DW 1206F et le DW 1206J, ainsi que l'Acrysol RM 184 ou l'Acrysol 44 de la société ROHM & HAAS, ou bien encore le Borchigel LW 44 de la société BORCHERS.

**Charges :**

La composition selon l'invention peut comprendre en outre au moins une charge.
La (ou les) charge(s) peuvent être présentes en une teneur allant de 0,01 % à 50 % en poids, par rapport au poids total de la composition, de préférence allant de 0,01 % à 30 % en poids.
Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.
Les charges peuvent être minérales ou organiques de toutes formes, plaquettaires, sphériques ou oblongues. Lorsqu'elles sont minérales, elles peuvent présenter toute forme cristallographique (par exemple feuillet, cubique, hexagonale, orthorombique, etc.). On peut citer le talc, le mica, la silice, le kaolin, les poudres de polyamide (Nylon®) (Orgasol® de chez Atochem), les poudres de polyméthyl méthacrylate, les poudres de polymères acryliques, de poly-β-alanine et de polyéthylène, les poudres de polymères de tétrafluoroéthylène (Téflon®), les poudres de lauroyl-lysine, les poudres d'amidon, les poudres de cellulose, le nitrure de bore, les microsphères creuses en polymères organiques, notamment les microsphères creuses polymériques de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydrocarbonate de magnésium, l'hydroxyapatite, les microsphères de silice creuses (Silica Beads® de Maprecos), les microcapsules de verre ou de céramique, l'argile, le quartz, la poudre de diamant naturel, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.
Selon un mode particulier, l'association des résines selon l'invention avec au moins une charge de nature minérale, éventuellement en association avec au moins une charge organique, permet d'obtenir un produit dont le fini est doux et dont les propriétés cosmétiques sont stables dans le temps, en particulier tout au long de la journée. Le produit est également glissant sur la peau, lors de l'application, sans sensation de frottement, et confère un toucher doux sur ladite peau.
Par charges minérales, on peut notamment citer le talc, le mica, la silice, le kaolin, le nitrure de bore, le carbonate de calcium précipité, le carbonate et l'hydro-carbonate de magnésium, l'hydroxyapatite, les microsphères de silice creuses (Silica Beads® de Maprecos), ou les microcapsules de verre ou de céramique, l'argile, le quartz, la poudre de diamant naturel ou leur mélange.
Comme poudre de silice, on peut citer :

- les microsphères de silice poreuses vendues sous la dénomination SILICA BEADS SB-700 par la société MYOSHI ; "SUNSPHERE® H51", "SUNSPHERE® H33" par la société ASAHI GLASS ;
- les microsphères de silice amorphe enrobées de polydiméthylsiloxane vendues sous la dénomination "SA SUNSPHERE® H 33", "SA SUNSPHERE® H53" par la société ASAHI GLASS.

De préférence, la charge minérale est la silice, le talc ou leur mélange.
Parmi les charges sphériques, on préfère les silices, comme les microsphères de silice creuses, en particulier les SB700® de Miyoshi Kasei.
Selon un mode préféré, la composition selon l'invention comprend en outre au moins une autre charge. Ladite au moins une autre charge peut être minérale ou organique. Il peut ainsi s'agir d'un mélange de charges minérales et organiques.
Selon une alternative, la composition selon l'invention peut contenir une charge minérale et une autre charge minérale, lesdites charges minérales étant telles que définies ci-dessus, et éventuellement au moins une charge organique, telle que définie ci-dessous.
Selon une autre alternative, la composition selon l'invention peut contenir une charge minérale et une charge organique.
Par charges organiques, on peut notamment citer les poudres de polyamide (Nylon® ou Orgasol® de chez Arkema), les poudres de polymères acryliques, notamment les poudres de polyméthyl méthacrylate, de poly méthacrylate de méthyle/diméthacrylate d'éthylène glycol, de polyméthacrylate d'allyle/diméthacrylate d'éthylène glycol, de copolymère diméthacrylate d'éthylène glycol/méthacrylate de lauryle, les poudres de cellulose, de poly-β-alanine et de polyéthylène, les poudres de polymères de tétrafluoroéthylène (Téflon®), la lauroyl-lysine, l'amidon, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, notamment obtenues par polymérisation d'organopolysiloxane ayant au moins deux atomes d'hydrogène liés chacun à un atome de silicium et d'un organopolysiloxane comprenant au moins deux groupes à insaturation éthylénique (notamment deux groupes vinyles) en présence

de catalyseur platine, ou encore les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Comme poudre de polymères acryliques, on peut citer :

- les poudres de polyméthacrylate de méthyle vendues sous la dénomination COVABEAD® LH85 par la société WACKHERR ;
- les poudres de poly méthacrylate de méthyle/diméthacrylate d'éthylène glycol vendues sous la dénomination DOW CORNING 5640 MICROSPONGE® SKIN OIL ADSORBER par la société DOW CORNING ; GANZPEARL® GMP-0820 par la société GANZ CHEMICAL ;
- les poudres de polyméthacrylate d'allyle/diméthacrylate d'éthylène glycol vendues sous la dénomination POLY-PORE® L200, POLY-PORE® E200 par la société AMCOL ;
- les poudres de copolymère diméthacrylate d'éthylène glycol/méthacrylate de lauryle vendues sous la dénomination POLYTRAP® 6603 de la société DOW CORNING.

Comme poudre de silicone élastomère, on peut citer les poudres vendues sous les dénominations "Trefil® Powder E-505C", "Trefil® Powder E-506C" par la société DOW CORNING.

De préférence, la charge organique correspond aux poudres de polyamide.

Dans le cas où la composition selon l'invention comprend des charges minérales et d'autres charges de type organiques, elles pourront être avantageusement dans ladite composition dans un rapport charges minérales/ charges organiques supérieur ou égal à 1.

Pour améliorer encore la matité et la tenue dans le temps de la matité obtenue avec les compositions comprenant l'association de résines selon l'invention avec une charge, la charge peut être choisie parmi les charges dites « absorbant le sébum ». La charge absorbant le sébum peut être une poudre minérale ou une poudre organique ; elle peut être choisie parmi la silice, les poudres de polyamides (nylon®), les poudres de polymères acryliques, notamment de polyméthacrylate de méthyle, de poly méthacrylate de méthyle/diméthacrylate d'éthylène glycol, de polyméthacrylate d'allyle/diméthacrylate d'éthylène glycol, de copolymère diméthacrylate d'éthylène glycol/méthacrylate de lauryle ; microsphères creuses polymériques de chlorure de polyvinylidène/acrylonitrile, les poudres de silicone élastomère, notamment obtenues par polymérisation d'organopolysiloxane ayant au moins deux atomes d'hydrogène liés chacun à un atome de silicium et d'un organopolysiloxane comprenant au moins deux groupes à insaturation éthylénique (notamment deux groupes vinyles) en présence de catalyseur platine.

Comme microsphères creuses polymériques de chlorure de polyvinylidène/acrylonitrile, on peut citer celles vendues sous la dénomination Expancel® par la société Nobel Industrie.

## <u>Matières colorantes non traitées en surface:</u>

La composition selon l'invention peut comprendre en outre au moins une matière colorante non traitée en surface.

La matière colorante peut être choisie parmi les matières colorantes pulvérulentes (notamment les pigments et les nacres) telles que décrites ci-dessus, les matières colorantes hydrosolubles ou liposolubles.

Les pigments et les nacres peuvent être définis tels que précédemment, mais sont ici non traités en surface par un agent hydrophobe.

Par ailleurs, les colorants hydrosolubles sont par exemple le jus de betterave, le bleu de méthylène.

Les colorants liposolubles, synthétiques ou naturels sont, par exemple, le DC Red 17, le DC Red 21, le DC Red 27, le DC Green 6, le DC Yellow 11, le DC Violet 2, le DC Orange 5, le rouge Soudan, les carotènes (le β-carotène, le lycopène), les xanthophylles (capsanthine, capsorubine, lutéine), l'huile de palme, le brun Soudan, le jaune quinoléine, le rocou, le curcumin.

Les matières colorantes, en particulier les pigments, non traitées en surface peuvent être présents dans la composition en une teneur allant de 0,1 % à 50 % en poids, par rapport au poids total de la composition, de préférence allant de 0,5 % à 30 % en poids, et préférentiellement allant de 1 % à 20 % en poids.

## <u>Polymères filmogènes</u>

La composition selon l'invention peut comprendre en outre au moins un polymère filmogène.

Dans la présente invention, on entend par « polymère filmogène », un polymère apte à former à lui seul ou en présence d'un agent auxiliaire de filmification, un film macroscopiquement continu et adhérent sur les matières kératiniques, et de préférence un film cohésif, et mieux encore un film dont la cohésion et les propriétés mécaniques sont telles que ledit film peut être isolable et manipulable isolément, par exemple lorsque ledit film est réalisé par coulage

sur une surface antiadhérente comme une surface téflonnée ou siliconnée.

Le ou les polymères filmogènes utilisés, en association avec les mélanges de résines MQ et T propyle, peuvent être véhiculés dans la phase huileuse (polymères liposolubles ou lipo-dispersibles) ou véhiculés dans une phase aqueuse (polymères hydrosolubles ou latex).

La composition peut comporter une phase aqueuse et le polymère filmogène peut être présent dans cette phase aqueuse. Dans ce cas celui-ci sera de préférence un polymère en dispersion aqueuse (latex) ou hydrosoluble.

Parmi les polymères filmogènes utilisables dans la composition de la présente invention, on peut citer les polymères synthétiques, de type radicalaire ou de type polycondensat, les polymères d'origine naturelle, et leurs mélanges.

Comme exemples de polymères filmogènes hydrosolubles, on peut citer :

- les protéines comme les protéines d'origine végétale telles que les protéines de blé, de soja ; les protéines d'origine animale tels que les kératines, par exemples les hydrolysats de kératine et les kératines sulfoniques ;
- les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
- les polymères ou copolymères acryliques, tels que les polyacrylates ou les polyméthacrylates ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;
- les polymères de chitine ou de chitosane anioniques, cationiques, amphotères ou non-ioniques,
- les gommes arabiques, la gomme de guar, les dérivés du xanthane, la gomme de karaya ;
- les alginates et les carraghénanes ;
- les glycoaminoglycanes, l'acide hyaluronique et ses dérivés ;
- la résine shellac, la gomme de sandaraque, les dammars, les élémis, les copals ;
- l'acide désoxyribonucléïque ;
- les muccopolysaccharides tels les chondroïtines sulfate,
- et leurs mélanges.

Le polymère filmogène peut être également présent dans la composition sous la forme de particules en dispersion dans une phase aqueuse, connue généralement sous le nom de latex ou pseudolatex. Les techniques de préparation de ces dispersions sont bien connues de l'homme du métier.

Comme dispersion aqueuse de polymère filmogène, on peut utiliser les dispersions acryliques vendues sous les dénominations Neocryl XK-90®, Neocryl A-1070®, Neocryl A-1090®, Neocryl BT-62®, Neocryl A-1079® et Neocryl A-523® par la société AVECIA-NEORESINS, Dow Latex 432® par la société DOW CHEMICAL, Daitosol 5000 AD® ou Daitosol 5000 SJ® par la société DAITO KASEY KOGYO; Syntran 5760® par la société Interpolymer Allianz Opt® par la société Rohm and Haas, les dispersions aqueuses de polymères acryliques ou styrene/acrylique vendues sous le nom de JONCRYL® par la société JOHNSON POLYMER ou encore les dispersions aqueuses de polyuréthane vendues sous les dénominations Neorez R-981® et Neorez R-974® par la société AVECIA-NEORESINS, les Avalure UR-405®, Avalure UR-410®, Avalure UR-425®, Avalure UR-450®, Sancure 875®, Avalure UR-445® et Sancure 2060® par la société NOVEON, Impranil 85® par la société BAYER, Aquamere H-1511® par la société HYDROMER ; les sulfopolyesters vendus sous le nom de marque Eastman AQ® par la société EASTMAN CHEMICAL PRODUCTS, les dispersions vinyliques comme le Mexomère PAM® de la société CHIMEX, les dispersions aqueuses de polyvinyl acétate comme le "Vinybran®" de la société Nisshin Chemical ou celles commercialisées par la société UNION CARBIDE, les dispersions aqueuses de terpolymère vinyl pyrrolidone, diméthylaminopropyl méthacrylamide et chlorure de lauryldiméthylpropylméthacrylamidoammonium telles que le Styleze W-d'ISP, les dispersions aqueuses de polymères hybrides polyuréthane/polyacryliques telles que celles commercialisées sous les références "Hybridur®" par la société AIR PRODUCTS ou "Duromer®" de NATIONAL STARCH, les dispersions type core/shell : par exemple celles commercialisées par la société ATOFINA sous la référence Kynar (core : fluoré- shell : acrylique) ou encore ceux décrits dans le document US 5 188 899 (core ; silice - shell : silicone) et leurs mélanges. La composition peut comporter une phase huileuse et le polymère filmogène peut être présent dans cette phase huileuse. Le polymère pourra alors être en dispersion ou en solution.

Comme exemples de dispersions non aqueuses de polymère filmogène lipodispersibles sous forme de dispersions non aqueuses de particules de polymère dans une ou plusieurs huiles de silicone et/ou hydrocarbonées et pouvant être stabilisées en leur surface par au moins un agent stabilisant, notamment un polymère séquencé, greffé ou statistique, on peut citer les dispersions acryliques dans l'isododécane comme le Mexomère PAP® de la société CHIMEX, les dispersions de particules d'un polymère éthylénique greffé, de préférence acrylique, dans une phase grasse liquide, le polymère éthylénique étant avantageusement dispersé en l'absence de stabilisant additionnel en surface des particules telles que décrite notamment dans le document WO 04/055081.

Parmi les polymères filmogènes utilisables dans la composition de la présente invention, on peut citer les

polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats).

Les polymères filmogènes de type radicalaire peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés $\alpha$, $\beta$-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en C1-C30, de préférence en C1-C20, des (méth)acrylates d'aryle, en particulier d'aryle en C6-C10, des (méth)acrylates d'hydroxyalkyle, en particulier d'hydroxyalkyle en C2-C6.

Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.

Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.

Parmi les (méth)acrylates d'aryle, on peut citer l'acrylate de benzyle et l'acrylate de phényle.

Les esters de l'acide (méth)acrylique particulièrement préférés sont les (méth)acrylates d'alkyle.
Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en C2-C12. Parmi les N-alkyl (méth)acrylamides, on peut citer le N-éthyl acrylamide, le N-t-butyl acrylamide, le N-t-octyl acrylamide et le N-undécylacrylamide.

Les polymères filmogènes vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.

Comme exemple d'esters vinyliques, on peut citer l'acétate de vinyle, le néodécanoate de vinyle, le pivalate de vinyle, le benzoate de vinyle et le t-butyl benzoate de vinyle.

Comme monomères styrèniques, on peut citer le styrène et l'alpha-méthyl styrène.

Parmi les polycondensats filmogènes, on peut citer les polyuréthanes, les polyesters, les polyesters amides, les polyamides, et les résines époxyesters, les polyurées.

Les polyuréthanes peuvent être choisis parmi les polyuréthanes anioniques, cationiques, non-ioniques ou amphotères, les polyuréthanes-acryliques, les poly-uréthanes-polyvinylpirrolidones, les polyester-polyuréthanes, les polyéther-polyuréthanes, les polyurées, les polyurée-polyuréthanes, et leurs mélanges.

Les polyesters peuvent être obtenus, de façon connue, par polycondensation d'acides dicarboxyliques avec des polyols, notamment des diols.

L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide, 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexanedicarboxylique, l'acide isophtalique, l'acide téréphtalique, l'acide 2,5-norbornane dicarboxylique, l'acide diglycolique, l'acide thiodipropionique, l'acide 2,5-naphtalènedicarboxylique, l'acide 2,6-naphtalènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

Selon un exemple de composition selon l'invention, le polymère filmogène peut être un polymère solubilisé dans une phase grasse liquide comprenant des huiles ou solvants organiques (on dit alors que le polymère filmogène est un polymère liposoluble). De préférence, la phase grasse liquide comprend une huile volatile, éventuellement en mélange avec une huile non volatile.

A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester) et d'au moins un autre monomère qui peut être un ester vinylique (différent de l'ester vinylique déjà présent), une $\alpha$-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme exemples supplémentaires de polymères filmogènes liposolubles, on peut citer les copolymères d'ester vinylique et au moins un autre monomère qui peut être un ester vinylique, notamment le néodécanoate de vinyle, le benzoate de vinyle et le t-butyl benzoate de vinyle, une $\alpha$-oléfine, un alkylvinyléther, ou un ester allylique ou méthallylique.

Comme polymères filmogènes liposolubles, on peut également citer les copolymères liposolubles, et en particulier ceux résultant de copolymérisation d'esters vinyliques ayant de 9 à 22 atomes de carbone ou d'acrylates ou de méthacrylates d'alkyle, les radicaux alkyles ayant de 10 à 20 atomes de carbone.

De tels copolymères liposolubles peuvent être choisis parmi les copolymères de polystéarate de vinyle, de polystéarate de vinyle réticulé à l'aide de divinylbenzène, de diallyléther ou de phtalate de diallyle, les copolymères de poly(méth)acrylate de stéaryle, de polylaurate de vinyle, de poly(méth)acrylate de lauryle, ces poly(méth)acrylates pouvant être réticulés à l'aide dé diméthacrylate de l'éthylène glycol ou de tétraéthylène glycol.

Les copolymères liposolubles définis précédemment sont connus et notamment décrits dans la demande FR-A-2232303 ; ils peuvent avoir un poids moléculaire moyen en poids allant de 2.000 à 500.000 et de préférence de 4.000 à 200.000.

Comme polymères filmogènes liposolubles utilisables dans l'invention, on peut également citer les polyalkylènes et notamment les copolymères d'alcènes en C2-C20, comme le polybutène, les alkylcelluloses avec un radical alkyle linéaire ou ramifié, saturé ou non en C1 à C8 comme l'éthylcellulose et la propylcellulose, les copolymères de la vinylpyrolidone (VP) et notamment les copolymères de la vinylpyrrolidone et d'alcène en C2 à C40 et mieux en C3 à C20. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

On peut également citer les résines de silicone, généralement solubles ou gonflables dans les huiles de silicone, qui sont des polymères de polyorganosiloxanes réticulés.

A titre d'exemples de résines polymethylsilsesquioxanes commercialement disponibles, on peut citer celles qui sont commercialisées par la société Wacker sous la référence Resin MK tels que la Belsil PMS MK, ou par par la société SHIN-ETSU sous les références KR-220L.

A titre d'exemples de résines polypropylsilsesquioxanes commercialement disponibles, on peut citer celles qui sont commercialisés sous la référence DC670 par la société Dow Corning.

Comme résines siloxysilicates, on peut citer les résines trimethylsiloxysilicate (TMS) telles que celle commercialisées sous la référence SR1000 par la société General Electric ou sous la référence TMS 803 par la société Wacker. On peut encore citer les résines timéthylsiloxysilicate commercialisées dans un solvant tel que la cyclomethicone, vendues sous la dénomination KF-7312J par la société Shin-Etsu, "DC 749", "DC 593" par la société Dow Corning.

On peut aussi citer des copolymères de résines de silicone telles que celles citées ci-dessus avec des polydiméthylsiloxanes, comme les copolymères adhésifs sensibles à la pression commercialisés par la société Dow Corning sous la référence BIO-PSA et décrits dans le document US 5 162 410 ou encore les copolymères siliconés issus de la

réaction d'un résine de silicone, telle que celles décrite plus haut, et d'un diorganosiloxane tels que décrits dans le document WO 2004/073626.

On peut également citer les copolymères greffés acryliques/silicone ayant un squelette polymérique vinyl, méthacrylique ou acrylique, et des greffons pendants organosiloxanes ou polyorganoxiloxanes. De tels polymères sont notamment décrits dans les brevets US 4 693 935, US 4 981 903, et US 4 981 902.

De préférence, ces polymères comprennent des monomères A, C et optionnellement B pour lesquels :

- A est au moins un monomère polymérisable vinyl, méthacrylate ou acrylate radicalaire libre ;
- B, lorsqu'il est présent, est au moins un monomère rigidifiant copolymérisable avec A;
- C est un monomère de formule suivante :

$$X(Y)_nS\ i(R)_{3-m}Z_m$$

Où X est un groupement vinyl copolymérisable avec les monomères A et B ;
Y est un linker divalent ;
n est 0 ou 1 ;
m est un entier compris entre 1 et 3 ;
R est un atome d'hydrogène, un radical alkyle ayant de 1 à 10 atomes de carbone, un radical phényle substitué ou non, un radical alcoxy ayant de 1 à 10 atomes de carbone ;
Z est un groupe polymérique siloxane monovalent.

Des exemples de monomères A sont des esters inférieurs à intermédiaires d'acide méthacrylique et d'alcools C1-12 à chaîne linéaire ou ramifiée, du styrène, des esters vinyliques, du chlorure de vinyle, du chlorure de vinylidène, ou des monomères acryloyl.

Des exemples de monomères B sont des monomères acryliques ou méthacryliques polaires ayant au moins un groupe hydroxy, amino, ester ou ionique (comme les ammoniums quaternaires, le sel carboxylate ou les acides comme les acides carboxyliques, les acides acryliques, l'acide sulfonique ou ses sels).

Le monomère C est défini ci-dessus.

Comme exemples de copolymères greffés acrylique/silicone, on peut citer ceux commercialisés par 3M sous la référence 3M Silicones "Plus" VS70 Dry Polymer®, de nom INCI : Polysilicone-6, ou encore le KP-561® commercialisé par SHIN-ETSU et de nom INCI : Acrylates/ Stearyl Acrylate/ Dimethicone Methacrylate Copolymer, le KP-562® commercialisé par SHIN-ETSU et de nom INCI : Acrylates/ Behenyl Acrylate/ Dimethicone Acrylate Copolymer.

Selon un exemple de mise en oeuvre de l'invention, le polymère filmogène est un polymère éthylénique séquencé linéaire filmogène, qui comprend de préférence au moins une première séquence et au moins une deuxième séquence ayant des températures de transition vitreuse (Tg) différentes, lesdites première et deuxième séquences étant reliées entre elles par une séquence intermédiaire comprenant au moins un monomère constitutif de la première séquence et au moins un monomère constitutif de la deuxième séquence.

Avantageusement, les première et deuxième séquences et du polymère séquencé sont incompatibles l'une avec l'autre.

De tels polymères sont décrits par exemple dans les documents EP 1411069 ou WO04/028488.

Le polymère filmogène peut être choisi parmi les polymères et/ou copolymères blocs ou statiques comportant notamment les polyuréthanes, polyacryliques, les silicones, les polymères fluorés, les gommes butyliques, les copolymères d'éthylènes, gommes naturelles et les alcools polyvinyliques et leurs mélanges. Les monomères des copolymères blocs ou statiques comprenant au moins une association de monomères dont le polymère résulte à une température de transition vitreuse inférieure à la température ambiante (25 °C) peuvent être choisis parmi notamment le butadiène, l'éthylène, le propylène, l'acrylique, le méthacrylique, l'isoprène, l'isobutène, une silicone et leurs mélanges.

La composition selon l'invention peut également comprendre au moins un polymère filmogène choisi parmi les polymères vinyliques comprenant au moins un motif dérivé de dendrimère carbosiloxane.

Le polymère vinylique peut posséder notamment un squelette et au moins une chaîne latérale, laquelle comprend une structure de dendrimère carbosiloxane. Le terme « structure de dendrimère carbosiloxane » dans le contexte de la présente invention représente une structure moléculaire possédant des groupes ramifiés ayant des masses moléculaires élevées, ladite structure ayant une régularité élevée dans la direction radiale en partant de la liaison au squelette. De telles structures de dendrimère carbosiloxane sont décrites sous la forme d'un copolymère siloxane-silylalkylène fortement ramifié dans la demande de brevet japonais mise à l'inspection publique Kokai 9-171 154.

Le polymère vinylique peut être un des polymères décrits dans les exemples de la demande EP0963751 ou par exemple le produit TIB-4-200 commercialisé par Dow Corning.

On peut également citer comme polymères filmogènes des systèmes à deux composantes telles que des

composés X et Y, définis ci-après, capables de polymériser *in situ*, à pression atmosphérique et température ambiante, et de former des films avantageusement biocompatibles, non collants, légèrement opalescents voire pelables. De tels systèmes sont notamment en partie décrits dans les brevets WO 01/96 450 et GB 2 407 496 de Dow Corning.

Selon un mode de réalisation particulier, les composés X et les composés Y sont siliconés. Les composés X et Y peuvent être aminés ou non aminés.

Selon un autre mode de réalisation, au moins un des composés X et Y est un polymère dont la chaîne principale est formée majoritairement d'unités organosiloxanes.

Parmi les composés siliconés cités ci-après, certains peuvent présenter à la fois des propriétés filmogènes et adhésives, selon par exemple leur proportion en silicone ou selon qu'on les utilise en mélange avec un additif particulier. Il est par conséquent possible de moduler les propriétés filmogènes ou les propriétés adhésives de tels composés selon l'utilisation envisagée, c'est en particulier le cas pour les silicones élastomères réactives dites " room temperature vulcanization".

Les composés X et Y peuvent réagir ensemble à une température variant entre la température ambiante et 180°C. Avantageusement les composés X et Y sont susceptibles de réagir ensemble à température ambiante (20 ± 5°C) et pression atmosphérique, ou avantageusement en présence d'un catalyseur, par une réaction d'hydrosilylation ou une réaction de condensation, ou une réaction de réticulation en présence d'un peroxyde.

Selon un mode de réalisation particulier, les composés X et Y réagissent par hydrosilylation en présence d'un catalyseur.

De façon avantageuse, les composés X et Y sont choisis parmi les composés siliconés susceptibles de réagir par hydrosilylation en présence d'un catalyseur ; en particulier le composé X est choisi parmi les polyorganosiloxanes comprenant des unités de formule (I) décrits ci-dessous et le composé Y est choisi parmi les organosiloxanes comprenant des unités alkylhydrogènosiloxanes de formule (III) décrits ci-dessous.

Selon un mode de réalisation particulier, le composé X est un polydiméthylsiloxane à groupements vinyliques terminaux, et le composé Y est un polyméthylhydrogénosiloxane.

Le composé X est donc avantageusement choisi parmi les polyorganosiloxanes comprenant des unités siloxanes de formule :

$$R_mR'SiO_{\frac{(3-m)}{2}} \quad \text{(I)}$$

dans laquelle :

- R représente un groupe hydrocarboné monovalent, linéaire ou cyclique, comprenant de 1 à 30 atomes de carbone, de préférence de 1 à 20, et mieux de 1 à 10 atomes de carbone, comme par exemple un radical alkyle à chaîne courte, comprenant par exemple de 1 à 10 atomes de carbone, en particulier un radical méthyle ou encore un groupement phényle, de préférence un radical méthyle,
- m est égal à 1 ou 2 et
- R' représente :

  ◦ un groupement hydrocarboné aliphatique insaturé comprenant de 2 à 10, de préférence de 3 à 5 atomes de carbone comme par exemple un groupe vinyle ou un groupe -R"-CH=CHR"' dans lequel R" est une chaîne hydrocarbonée aliphatique divalente, comprenant de 1 à 8 atomes de carbone, liée à l'atome de silicium et R"' est un atome d'hydrogène ou un radical alkyle comprenant de 1 à 4 atomes de carbone, de préférence un atome d'hydrogène ; on peut citer comme groupement R' les groupements vinyle, allyle et leurs mélanges ; ou
  ◦ un groupement hydrocarboné cyclique insaturé comprenant de 5 à 8 atomes de carbone comme par exemple un groupe cyclohexènyle.

De préférence R' est un groupement hydrocarboné aliphatique insaturé, de préférence un groupe vinyle.

Selon un mode de réalisation, R représente un radical alkyle comprenant de 1 à 10 atomes de carbone ou encore un groupement phényle, et de préférence un radical méthyle, et R' est un groupe vinyle.

Le composé Y peut être avantageusement choisi parmi les polyorganosiloxanes comprenant au moins une unité alkylhydrogènosiloxane de formule suivante :

$$R_pHSiO_{\frac{(3-p)}{2}} \quad \text{(III)}$$

dans laquelle :

R représente un groupe hydrocarboné monovalent, linéaire ou cyclique, comprenant de 1 à 30 atomes de carbone, comme par exemple un radical alkyle ayant de 1 à 30 atomes de carbone, de préférence de 1 à 20 et mieux de 1 à 10 atomes de carbone, en particulier un radical méthyle, ou encore un groupement phényle et p est égal à 1 ou 2. De préférence R est un groupement hydrocarboné, de préférence le méthyle.

Selon un mode de réalisation, les compositions comprenant le composé X et/ou Y peut comprendre en outre un composé réactif additionnel tels que :

- les particules organiques ou minérales comprenant à leur surface au moins 2 groupements aliphatiques insaturés, on peut citer par exemple les silices traitées en surface par exemple par des composés siliconés à groupements vinyliques tels que par exemple la silice traitée cyclotetraméthyltetravinylsiloxane,
- des composés silazanes tels que l'hexaméthyldisilazane.

La réaction d'hydrosilylation se fait en présence d'un catalyseur qui peut être présent avec l'un ou l'autre des composés X ou Y ou être présent de manière isolée. Par exemple, ce catalyseur peut être présent dans la composition sous une forme encapsulée si les deux composés X et Y, dont il doit provoquer l'interaction, sont présents dans cette même composition sous une forme non encapsulée ou à l'inverse il peut y être présent sous une forme non encapsulée si au moins l'un des composés X et Y est présent dans la composition sous une forme encapsulée. Le catalyseur est de préférence à base de platine ou d'étain.
Le catalyseur peut être présent en une teneur allant de 0,0001% à 20% en poids par rapport au poids total de la composition le comprenant.
Les composés X et/ou Y peuvent être associés à des inhibiteurs ou retardateurs de polymérisation, et plus particulièrement des inhibiteurs du catalyseur. De façon non limitative, on peut citer les polyméthylvinylsiloxanes cycliques, et en particulier le tetravinyl tétraméthyl cyclotetrasiloxane, les alcools acétyléniques, de préférence volatils, tels que le méthylisobutynol.
La présence de sels ioniques, tels que l'acétate de sodium peut avoir une influence dans la vitesse de polymérisation des composés.

A titre d'exemple d'une combinaison de composés X et Y réagissant par hydrosilylation en présence d'un catalyseur, on peut citer les références suivantes proposée par la société Dow Corning : DC 7-9800 Soft Skin Adhesive Parts A & B, ainsi que la combinaison des mélanges A et B suivants préparés par Dow Corning :

**MELANGE A :**

| **Ingrédient (Nom INCI)** | **N°CAS** | **Teneurs (%)** | **Fonction** |
|---|---|---|---|
| Dimethyl Siloxane, Dimethylvinylsiloxy-terminaux | 68083-19-2 | 55-95 | Polymère |
| Silica Silylate | 68909-20-6 | 10-40 | Charge |
| 1,3-Diethenyl-1,1,3,3-Tetramethyldisiloxane complexes | 68478-92-2 | Trace | Catalyseur |
| Tetramethyldivinyldisiloxane | 2627-95-4 | 0.1-1 | Polymère |

**MELANGE B :**

| **Ingrédient (Nom INCI)** | **N°CAS** | **Teneurs (%)** | **Fonction** |
|---|---|---|---|
| Dimethyl Siloxane, Dimethylvinylsiloxy-terminaux | 68083-19-2 | 55-95 | Polymère |
| Silica Silylate | 68909-20-6 | 10-40 | Charge |
| Dimethyl, Methylhydrogen Siloxane, trimethylsiloxy-terminaux | 68037-59-2 | 1-10 | Polymère |

Le composé X peut représenter de 0,1 % à 95% en poids par rapport au poids total de la composition le contenant, de préférence de 1% à 90%, et mieux de 5% à 80%.

Le composé Y peut représenter de 0.1 % à 95% en poids par rapport au poids total de la composition le contenant, de préférence de 1% à 90% et mieux de 5% à 80%.

La composition selon l'invention peut comprendre un agent plastifiant favorisant la formation d'un film avec le polymère filmogène. Un tel agent plastifiant peut être choisi parmi tous les composés connus de l'homme du métier

comme étant susceptibles de remplir la fonction recherchée.

**Tensioactifs ioniques**

La composition selon l'invention peut comprendre en outre au moins un tensioactif ionique.
Le tensioactif peut être lipophile et/ou hydrophile, utilisé seul ou en couplage. Le tensioactif peut être choisi parmi les tensioactifs anioniques, cationiques, amphotères.

Le tensioactif peut être présent dans la composition selon l'invention en une teneur allant de 0,1 % à 10 % en poids, par rapport au poids total de la composition, et de préférence allant de 0,5 % à 8 % en poids, et préférentiellement allant de 0,5 % à 7 % en poids.

De préférence, les tensioactifs ioniques sont choisis parmi les tensioactifs cationiques, les tensioactifs amphotères, les carboxylates, les taurates et N-acyl N-méthyltaurates, les alkylsulfoacétates, les polypeptides, les dérivés anioniques d'alkyl polyglycoside, les sels d'acides gras en C16-C30 dérivant des amines, les sels d'acides gras polyoxyéthylénés, les esters phosphoriques et leurs sels, les sulfosuccinates, les alkyl sulfates, les iséthionates et N-acyliséthionates, les acylglutamates, les dérivés de soja, les citrates, les dérivés de proline, les lactylates, les sarcosinates, les sulfonates et les glycinates.

Lorsque le tensioactif ionique est un tensioactif anionique, il est choisi parmi :

- les carboxylates, comme le 2-(2-Hydroxyalkyloxy) acétate de sodium ;
- les taurates et N-acyl N-méthyltaurates ;
- les alkylsulfoacétates ;
- les polypeptides ;
- les dérivés anioniques d'alkyl polyglycoside (acyl-D-galactoside uronate) ;
- les sels d'acides gras en C16-C30 notamment ceux dérivant des amines, comme le stéarate de triéthanolamine et/ou le stéarate d'amino-2-méthyl-2-propane di-ol-1,3 ;
- les sels d'acides gras polyoxyéthylénés notamment ceux dérivant des amines ou les sels alcalins, et leurs mélanges ;
- les esters phosphoriques et leurs sels tels que le "DEA oleth-10 phosphate" (Crodafos N 10N de la société CRODA) ou le phosphate de monocétyle monopotassique (Amphisol K de Givaudan);
- les sulfosuccinates tels que le "Disodium PEG-5 citrate lauryl sulfosuccinate" et le "Disodium ricinoleamido MEA sulfosuccinate" ;
- les alkyl sulfates ;
- les iséthionates et N-acyliséthionates ;
- les acylglutamates tels que le "Disodium hydrogenated tallow glutamate" (AMISOFT HS-21 R® commercialisé par la société AJINOMOTO) et le sodium stearoyl glutamate (AMISOFT HS-11 PF® commercialisé par la société AJINOMOTO) et leurs, mélanges ;
- les dérivés de soja comme le potassium soyate ;
- les citrates, comme le Glyceryl stearate citrate (Axol C 62 Pellets de Degussa) ;
- les dérivés de proline, comme le Sodium palmitoyl proline (Sepicalm VG de Seppic), ou le Mélange de Sodium palmitoyl sarcosinate, Magnesium palmitoyl glutamate, Palmitic acid et Palmitoyl proline (Sepifeel One de Seppic) ;
- les lactylates, comme le Sodium stearoyl lactylate (Akoline SL de Karlshamns AB) ;
- les sarcosinates, comme le sodium palmitoyl sarcosinate (Nikkol sarcosinate PN) ou le mélange de Stéaroyl sarcosine et Myristoyl sarcosine 75/25 (Crodasin SM de Croda) ;
- les sulfonates, comme le Sodium C14-17 alkyl sec sulfonate (Hostapur SAS 60 de Clariant) ;
- les glycinates, comme le sodium cocoyl glycinate (Amilite GCS-12 d'Ajinomoto).

Les compositions conformes à l'invention peuvent également contenir un ou plusieurs tensioactifs amphotériques comme les N-acyl-aminoacides tels que les N-alkyl-aminoacétates et le cocoamphodiacetate disodique et les oxydes d'amines tels que l'oxyde de stéaramine, les bétaïnes, les N-alkylamidobétaïnes et leurs dérivés, les sultaïnes, les alkyl polyaminocarboxylates, les alkylamphoacétates, ou encore des tensioactifs siliconés comme les diméthicone copolyols phosphates tels que celui vendu sous la dénomination PECOSIL PS 100® par la société PHOENIX CHEMICAL et leurs mélanges.

De préférence, les compositions selon l'invention comprennent également un élastomère de silicone amphiphile comportant des groupes, séquences ou greffons hydrophiles polyoxyalkylènes, en particulier polyoxyéthylène et/ou polyoxypropylènes, ou des groupes, séquences ou greffons hydrophiles polyglycérols, et pouvant posséder en plus des groupes alkyles latéraux, en particulier des groupes lauryles latéraux, notamment un élastomère de silicone polyglycérolé. A titre d'exemple, on utilise un organopolysiloxane réticulé élastomère pouvant être obtenu par réaction d'addition réticulation de diorganopolysiloxane contenant au moins un hydrogène lié au silicium et de composés polyglycérolés ayant des groupements à insaturation éthylénique, notamment en présence de catalyseur platine.

Comme élastomères de silicone polyglycérolés, on peut utiliser ceux vendus sous les dénominations "KSG-710", "KSG-810", "KSG-820", "KSG-830", "KSG-840" par la société Shin Etsu.

**Milieu physiologiquement acceptable :**

Par « milieu physiologiquement acceptable », on entend désigner un milieu convenant particulièrement à l'application d'une composition de l'invention sur la peau, ses phanères (comme par exemple cheveux, ongles,) ou les lèvres. Le milieu physiologiquement acceptable est généralement adapté à la nature du support sur lequel doit être appliquée la composition, ainsi qu'à l'aspect sous lequel la composition doit être conditionnée. Ce milieu peut comprendre au moins un solvant volatil siliconé ou organique, ce solvant étant de préférence compatible avec les résines a/ et b/ et compatible avec une utilisation cosmétique, comme spécifié ci-avant.
La composition selon l'invention peut se présenter sous diverses formes, notamment sous forme de poudres (libres ou compactes), de composition anhydre, de dispersion, émulsion, telle que notamment eau/huile ou eau/cire, huile/eau, multiples ou cire/eau, ou encore sous forme de gel.
Une composition de l'invention est de préférence une émulsion, en particulier directe ou inverse, ou une composition anhydre.
Une dispersion peut être effectuée en phase aqueuse ou en phase huileuse.
Une émulsion peut posséder une phase continue huileuse ou aqueuse. Une telle émulsion peut être, par exemple, une émulsion inverse (E/H) ou directe (H/E), ou encore une émulsion multiple (E/H/E ou H/E/H).
Dans le cas des émulsions, les émulsions inverses (E/H) sont préférentielles.
Une composition anhydre est une composition contenant moins de 2 % en poids d'eau, voire moins de 0,5 % d'eau, et notamment exempte d'eau. Le cas échéant, d'aussi faibles quantités d'eau peuvent notamment être amenées par des ingrédients de la composition qui peuvent en contenir des quantités résiduelles.
La composition selon l'invention peut se présenter sous la forme d'un fluide par exemple pâteux ou liquide. Elle peut également se présenter sous forme de poudre libre ou compacte, de pâte souple, d'une crème. Par exemple, elle peut être une émulsion huile-dans-eau, eau-dans-huile ou multiple, une émulsion solide notamment de type eau dans huile, un gel notamment anhydre, solide ou souple, sous forme de poudre libre ou compactée et même sous forme biphasique.
La composition selon l'invention se présente généralement sous la forme d'une composition de maquillage et/ou de soin des matières kératiniques, par exemple d'un fond de teint notamment à appliquer sur le visage ou le cou, d'un produit anti-cernes, d'un correcteur de teint, d'une crème teintée, d'un fard à joues, d'un rouge à lèvres, d'un baume à lèvres, d'une composition de maquillage pour le corps.
La composition selon l'invention peut comprendre une phase aqueuse.
La phase aqueuse comprend de l'eau. Une eau convenant à l'invention peut être une eau florale telle que l'eau de bleuet et/ou une eau minérale telle que l'eau de VITTEL, l'eau de LUCAS ou l'eau de LA ROCHE POSAY et/ou une eau thermale.
La phase aqueuse peut également comprendre des solvants organiques miscibles à l'eau (à température ambiante -25 °C) comme par exemple les monoalcools ayant de 2 à 6 atomes de carbone tels que l'éthanol, l'isopropanol ; les polyols ayant notamment de 2 à 20 atomes de carbones, de préférence ayant de 2 à 10 atomes de carbone, et préférentiellement ayant de 2 à 6 atomes de carbone, tels que le glycérol, le propylène glycol, le butylène glycol, le pentylène glycol, l'hexylène glycol, le dipropylène glycol, le diéthylène glycol ; les éthers de glycol (ayant notamment de 3 à 16 atomes de carbone) tels que les alkyl($C_1$-$C_4$)éther de mono, di- ou tripropylène glycol, les alkyl($C_1$-$C_4$)éthers de mono, di- ou triéthylène glycol, et leurs mélanges.
La phase aqueuse peut comprendre en outre des agents de stabilisation, par exemple le chlorure de sodium, le dichlorure de magnésium et le sulfate de magnésium.
La phase aqueuse peut également comprendre tout composé hydrosoluble ou hydrodispersible compatible avec une phase aqueuse tels que des gélifiants, des polymères filmogènes, des épaississants, des tensioactifs et leurs mélanges.
En particulier, une composition de l'invention peut comprendre une phase aqueuse en une teneur variant de 1 % à 80 % en poids, notamment de 5 % à 50 %, et plus particulièrement de 10 % à 45 % en poids par rapport au poids total de la composition.
Selon un autre mode de réalisation, une composition de l'invention peut être anhydre.
Une composition anhydre peut comprendre moins de 5 % en poids d'eau, par rapport au poids total de la composition, et en particulier moins de 3 %, notamment moins de 2 %, et plus particulièrement moins de 1 % en poids d'eau par rapport au poids total de la composition.
Plus particulièrement, une composition anhydre peut être dépourvue d'eau.
Une composition cosmétique conforme à la présente invention peut comprendre au moins une phase grasse liquide et/ou solide.
En particulier, une composition de l'invention peut comprendre au moins une phase grasse liquide, notamment au moins une huile comme mentionnée ci-avant.

On entend par huile, tout corps gras sous forme liquide à température ambiante (20-25 °C) et à pression atmosphérique. Une composition de l'invention peut comprendre une phase grasse liquide en une teneur variant de 1 à 90 %, en particulier de 5 à 80 %, en particulier de 10 à 70 %, et plus particulièrement, de 20 à 50 % en poids par rapport au poids total de la composition.
La phase huileuse convenant à la préparation des compositions cosmétiques selon l'invention peut comprendre des huiles hydrocarbonées, siliconées, fluorées ou non, ou leurs mélanges.
Les huiles peuvent être volatiles ou non volatiles telles que définies ci-dessus.
Selon un mode de réalisation particulier, la phase grasse de la composition selon l'invention peut ne contenir que des composés volatils.

La composition selon l'invention peut également contenir des ingrédients couramment utilisés en cosmétique, tels que les vitamines, les épaississants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents anti-pelliculaires, les agents propulseurs, ou leurs mélanges.

Une composition selon l'invention peut, notamment, se présenter sous la forme d'une composition de maquillage et/ou de soin de la peau ou des lèvres, en particulier un fond de teint.

Bien entendu, l'homme du métier veillera à choisir ce ou ces éventuels composés complémentaires, et/ou leur quantité, de manière telle que les propriétés avantageuses de la composition correspondante selon l'invention ne soient pas, ou substantiellement pas, altérées par l'adjonction envisagée.

Selon un autre aspect, l'invention concerne également un ensemble cosmétique comprenant :

i) un récipient délimitant au moins un compartiment, ledit récipient étant fermé par un élément de fermeture ; et
ii) une composition disposée à l'intérieur dudit compartiment, la composition étant conforme à l'invention.

Le récipient peut être sous toute forme adéquate. Il peut être notamment sous forme d'un flacon, d'un tube, d'un pot, d'un étui, d'une boite, d'un sachet ou d'un boîtier.

L'élément de fermeture peut être sous forme d'un bouchon amovible, d'un couvercle, d'un opercule, d'une bande déchirable, ou d'une capsule, notamment du type comportant un corps fixé au récipient et une casquette articulée sur le corps. Il peut être également sous forme d'un élément assurant la fermeture sélective du récipient, notamment une pompe, une valve, ou un clapet.

Le récipient peut être associé à un applicateur, notamment sous forme d'une brosse comportant un arrangement de poils maintenus par un fil torsadé. Une telle brosse torsadée est décrite notamment dans le brevet US 4 887 622. Il peut être également sous forme d'un peigne comportant une pluralité d'éléments d'application, obtenus notamment de moulage. De tels peignes sont décrits par exemple dans le brevet FR 2 796 529. L'applicateur peut être sous forme d'un pinceau, tel que décrit par exemple dans le brevet FR 2 722 380. L'applicateur peut être sous forme d'un bloc de mousse ou d'élastomère, d'un feutre, ou d'une spatule. L'applicateur peut être libre (houppette ou éponge) ou solidaire d'une tige portée par l'élément de fermeture, tel que décrit par exemple dans le brevet US 5 492 426. L'applicateur peut être solidaire du récipient, tel que décrit par exemple le brevet FR 2 761 959.

Le produit peut être contenu directement dans le récipient, ou indirectement. A titre d'exemple, le produit peut être disposé sur un support imprégné, notamment sous forme d'une lingette ou d'un tampon, et disposé (à l'unité ou plusieurs) dans une boîte ou dans un sachet. Un tel support incorporant le produit est décrit par exemple dans la demande WO 01/03538.

L'élément de fermeture peut être couplé au récipient par vissage. Alternativement, le couplage entre l'élément de fermeture et le récipient se fait autrement que par vissage, notamment via un mécanisme à baïonnette, par encliquetage, serrage, soudage, collage, ou par attraction magnétique. Par "encliquetage" on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.

Le récipient peut être au moins pour partie réalisé en matériau thermoplastique. A titre d'exemples de matériaux thermoplastiques, on peut citer le polypropylène ou le polyéthylène.
Alternativement, le récipient est réalisé en matériau non thermoplastique, notamment en verre ou en métal (ou alliage).

Le récipient peut être à parois rigides ou à parois déformables, notamment sous forme d'un tube ou d'un flacon tube.

Le récipient peut comprendre des moyens destinés à provoquer ou faciliter la distribution de la composition. A titre d'exemple, le récipient peut être à parois déformables de manière à provoquer la sortie de la composition en réponse à une surpression à l'intérieur du récipient, laquelle surpression est provoquée par écrasement élastique (ou non élastique) des parois du récipient. Alternativement, notamment lorsque le produit est sous forme d'un stick, ce dernier peut être entraîné par un mécanisme à piston. Toujours dans le cas d'un stick, notamment de produit de maquillage (rouge à lèvres, fond de teint, etc.), le récipient peut comporter un mécanisme, notamment à crémaillère, ou avec une

tige filetée, ou avec une rampe hélicoïdale, et apte à déplacer un stick en direction de ladite ouverture. Un tel mécanisme est décrit par exemple dans le brevet FR 2 806 273 ou dans le brevet FR 2 775 566. Un tel mécanisme pour un produit liquide est décrit dans le brevet FR 2 727 609.

Le récipient peut être constitué d'un boîtier avec un fond délimitant au moins un logement contenant la composition, et un couvercle, notamment articulé sur le fond, et apte à recouvrir au moins en partie ledit fond. Un tel boîtier est décrit par exemple dans la demande WO 03/018423 ou dans le brevet FR 2 791 042.

Le récipient peut être équipé d'un essoreur disposé au voisinage de l'ouverture du récipient. Un tel essoreur permet d'essuyer l'applicateur et éventuellement, la tige dont il peut être solidaire. Un tel essoreur est décrit par exemple dans le brevet FR 2 792 618.

La composition peut être à la pression atmosphérique à l'intérieur du récipient (à température ambiante) ou pressurisée, notamment au moyen d'un gaz propulseur (aérosol). Dans ce dernier cas, le récipient est équipé d'une valve (du type de celles utilisées pour les aérosols).

La composition de l'invention peut se présenter sous la forme d'un produit de soin ou de préférence de maquillage, en particulier coloré, de la peau, plus spécifiquement du visage comme un fond de teint, un fard à joues ou à paupières, un fard à joues ou à paupières, un produit anti-cernes, un blush, une poudre libre ou compactée, ou encore un produit de maquillage du corps comme un produit de tatouage semi-permanent ou de maquillage des lèvres comme un rouge à lèvres, un crayon à lèvres ou un brillant à lèvres, présentant éventuellement des propriétés de soin ou de traitement non thérapeutique.

De préférence, la composition selon l'invention se présente sous forme d'un fond de teint.

La composition selon l'invention peut être fabriquée par les procédés connus, généralement utilisés dans le domaine cosmétique.

Dans la demande, les teneurs, sauf mention express contraire, sont exprimées en poids par rapport au poids total de la composition.

Les exemples suivants ont pour but d'illustrer les compositions et procédés selon cette invention, mais ne sont en aucun cas limitatifs de la portée de l'invention. Toutes les parties et pourcentages dans les exemples sont en poids et toutes les mesures ont été obtenues à environ 23 °C, sauf indication contraire.

**Exemple N° 1 : Obtention du mélange de résines MO et T propyle selon l'invention**

Matériaux

Résine MQ = une résine MQ de formule $M_{0,43}Q_{0,57}$ et de $M_n$ = 3 230 dissoute dans du xylène à 70,8 % en poids de solides. La résine MQ a été fabriquée selon les techniques décrites par Daudt dans le brevet US 2 676 182.

Résine T propyle = une résine de propyle silsesquioxane à 74,8 % en poids dans du toluène. La résine de propyle silsesquioxane a été obtenue par hydrolyse de propyle trichlorosilane.

Différentes solutions de résine MQ et de résine de T propyle sont mélangées dans un tricol muni d'un agitateur. Des aliquots de chaque mélange sont placés dans une coupelle en aluminium de 2 pouces de diamètre, et chauffées sous vide à une température de 110°C pendant une heure, suivi de 1h25 à 140°C. Des observations qualitatives visuelles sur la clarté et la dureté des mélanges obtenus sont faites (voir tableau 1 suivant) :

**Tableau 1**

| Exemple # | MQ (g) | T propyle (g) | Aliquot (g) | Aliquot séché (g) | % en poids de résine MQ | Clarté | Dureté |
|---|---|---|---|---|---|---|---|
| 1-a | 0.00 | 13.46 | 2.0475 | 1.5361 | 0.0 | Clair | Aspect gomme ; solide mou |
| 1-b | 1.40 | 12.03 | 2.0643 | 1.5415 | 9.9 | Clair | Solide mou |
| 1-c | 2.88 | 16.74 | 2.0840 | 1.5517 | 14.0 | Clair | Plus dur que 1-b |
| 1-d | 4.19 | 9.39 | 2.0746 | 1.5414 | 29.7 | Clair | Plus dur que 1-c |
| 1-e | 5.72 | 8.14 | 2.1066 | 1.5606 | 39.9 | Clair | Plus dur que 1-d |
| 1-f | 7.11 | 6.82 | 2.0257 | 1.4968 | 49.6 | Clair | Plus dur que 1-e |

Les résultats obtenus montrent la miscibilité inattendue des résines MQ et T propyle, basée sur la clarté du mélange sans solvant et la dureté croissante à mesure que la quantité de résine MQ croît.

Selon une alternative nommée 1-g, on utilise le mélange décrit à l'exemple 22 de la demande WO2005/075567 dans lequel le rapport pondéral entre la résine MQ et la résine T propyle est de 85/15.

Selon une alternative nommée 1-h, on utilise le mélange de résines décrit à l'exemple 13 de la demande WO2007/145765 dans lequel le rapport pondéral entre la résine MQ et la résine T propyle est de 60/40.

**Exemple 2 : Rouge à lèvres liquide**

| Rouge à lèvres liquide | |
|---|---|
| Mélange de résines MQ+T propyle tel que décrit dans l'exemple 1-d ci-dessus | 65,1 |
| CI 77491 & Disodium Stearoyl Glutamate & Aluminium Hydroxide | 2,5 |
| Polydiméthylsiloxane 5cSt | 4,5 |
| Isododecane | 27,9 |

**Exemple 3 : Rouge à lèvres liquide**

| Rouge à lèvres liquide | |
|---|---|
| Mélange de résines MQ+T propyle tel que décrit dans l'exemple 1-f ci-dessus | 44,1 |
| Huile de Parleam | 10,0 |
| Polydiméthylsiloxane 5cSt | 20,0 |
| Silice Pyrogénée Hydrophile | 1,0 |
| Pigments enrobés perfluorés | 2,5 |
| Isododecane | 22,4 |

Mode opératoire

1. On réalise un broyat pigmentaire des pigments dans la phase huileuse en effectuant 3 passages du mélange à la broyeuse tricylindre.
2. On pèse dans un bêcher le broyat nécessaire à la composition, les matières premières non volatiles et les matières premières volatiles.
3. On place le mélange sous agitation RAYNERI pendant 45 min.
4. On coule la formule dans des bouillottes étanches à l'isododécane.

**Exemple 4a: Mascara waterproof**

| Mascara waterproof | |
|---|---|
| Mélange de résines MQ+T propyle tel que décrit dans l'exemple 1-f ci-dessus | 6 |
| Pigments enrobés PF | 6 |
| Cire d'abeille | 20 |
| Bentone organophile | 5,32 |
| Carbonate de propylène | 1,74 |

(suite)

| Mascara waterproof | |
|---|---|
| Isododécane | Qsp 100 |

La cire et les pigments sont chauffés jusqu'à fusion de la cire puis le mélange est homogénéisé, on laisse refroidir le mélange à température ambiante (25°C) puis on ajoute le mélange de résines (phase A).

Parallèlement, on prépare un gel en mélangeant la bentone, le carbonate de propylène et l'isododécane (phase B), puis on ajoute ce mélange à la phase A à température ambiante et l'ensemble est passé au broyeur tricylindre.

**Exemple 4b : Mascara watterproof**

| Mascara waterproof | |
|---|---|
| Mélange de résines MQ+T propyle tel que décrit dans l'exemple 1-f ci-dessus | 15 |
| Pigments enrobés NAI | 6 |
| Cire de candellila | 25 |
| Polydiméthylsiloxane 500 000cSt | 15 |
| Cyclopentasiloxane | Qsp 100 |

Les pigments et la cire sont chauffés jusqu'à fusion de la cire puis le mélange est homogénéisé. Parallèlement, on mélange l'huile siliconée et le mélange de résines à température ambiante (25°C), puis on ajoute ce mélange au mélange cire/pigments à température ambiante, et l'ensemble est passé au broyeur tricylindre.

**Exemple 5 : Fond de teint**

| Fond de teint | | |
|---|---|---|
| A1 | Pigments enrobés PF | 10 |
| | Cyclopentasiloxane (D5) | 5 |
| A2 | Mélange de résines MQ+T propyle tel que décrit dans l'exemple 1-f ci-dessus | 10 |
| | Oxypropylène en solution dans la D5 | 1,8 |
| | Isostearyl diglyceryl succinate | 0,6 |
| | Isododecane | QSP 100 |
| | D5 | 5 |
| | Poly diphenyl dimethylsiloxane et de cyclopenta dimethyl siloxane | 3 |
| A3 | Poudre de nylon | 8 |
| B | Eau | 41,4 |
| | Conservateurs | 1 |

Mode opératoire

- On réalise un broyat pigmentaire des pigments dans la phase huileuse en effectuant 3 passages du mélange A1 à la broyeuse tricylindre.

- On pèse dans un bêcher le broyat A1 nécessaire à la composition, les matières premières non volatiles et les matières premières volatiles du mélange A2 et A3.
- On pèse le mélange B. On chauffe B jusqu'à obtention d'une solution limpide, puis on fait revenir le mélange B à température ambiante.
- On réalise l'émulsion en ajoutant le mélange B au mélange A1+A2+A3, sous agitation Moritz, 1500 tours/minute, pendant 10 minutes.

**Exemple 6 : Poudre de teint**

| Poudre de teint | | |
|---|---|---|
| A | Pigments enrobés PF | 10 |
| | Talc | 64,7 |
| | Poudre de Nylon | 10 |
| | Conservateur | 0,3 |
| B | Huile de Parleam | 8 |
| | Mélange de résines MQ+T propyle tel que décrit dans l'exemple 1-f ci-dessus | 10 |

Mode opératoire

- On pèse les matières premières de la phase A et on homogénéise par un mélangeur pendant 10 minutes.
- On pèse les matières premières de la phase B, que l'on ajoute à la phase A.
- On homogénéise le mélange au mélangeur pendant 3 minutes.
- On passe le mélange A+B au broyeur Alpine.

**Exemple 7 :**

Les compositions suivantes ont été réalisées (émulsions E/H).

| | Composition | A (enrobage NAI invention) | B (enrobage PF invention) | C (enrobage Si comparatif) |
|---|---|---|---|---|
| | Nom INCI | Composition (%) | | |
| A1 | cetyl PEG/PPG-10/1 dimethicone (Abil EM90 de la société Goldschmidt) | 2,1 | 2,1 | 2,1 |
| | polyglyceryl-4 isostearate (ISOLAN GI34® par la société EVONIK GOLDSCHMIDT) | 2,8 | 2,8 | 2,8 |
| | hexyl laurate (Cetiol A de Cognis) | 2,1 | 2,1 | 2,1 |
| | Tristearin and Acetylated Glycol Stearate (Unitwix de la société United Guardian) | 1 | 1 | 1 |
| | Isododecane | 8,25 | 8,5 | 8,5 |
| A2 | Isododecane | 3,5 | 3,5 | 3,5 |
| | résine MQ / TPropyl 60/40 selon l'exemple 1-h décrit ci-dessus | 8 | 8 | 8 |
| A3 | Isododecane | 8,5 | 8,25 | 8,25 |
| | Disteardimonium Hectorite and Propylene Carbonate and Isododecane (bentone gel ISD V de Elementis) | 5 | 5 | 5 |
| | CI 77492 Iron oxyde and Disodium Stearoyl Glutamate and Aluminium Hydroxyde (1) | 3,8 | | |
| | CI 77491 Iron oxyde and Disodium Stearoyl Glutamate and Aluminium Hydroxyde (2) | 1,2 | | |
| | CI 77499 Iron oxyde and Disodium Stearoyl Glutamate and Aluminium Hydroxyde (3) | 0,4 | | |
| | CI 77891 Titanium Dioxide and Disodium Stearoyl Glutamate and Aluminium Hydroxyde (4) | 8,6 | | |
| | CI 77492 Iron oxyde and C9-15 Fluoroalcohol Phosphate (5) | | 3,8 | |
| | CI 77491 Iron oxyde and C9-15 Fluoroalcohol Phosphate (6) | | 1,2 | |
| | CI 77499 Iron oxyde and C9-15 Fluoroalcohol Phosphate (7) | | 0,4 | |
| | CI 77891 Titanium Dioxide andC9-15 Fluoroalcohol Phosphate (8) | | 8,6 | |
| | CI 77492 ENROBE DE TRIETHOXYCAPRYLYLSILANE (9) | | | 3,8 |
| | CI 77491 ENROBE DE TRIETHOXYCAPRYLYLSILANE (10) | | | 1,2 |
| | CI 77499 ENROBE DE TRIETHOXYCAPRYLYLSILANE (11) | | | 0,4 |
| | CI 77891 ENROBE DE TRIETHOXYCAPRYLYLSILANE (12) | | | 8,6 |
| A4 | Nylon-12 | 3,75 | 3,75 | 3,75 |
| | talc | 3,75 | 3,75 | 3,75 |
| B | Eau / solvant | 35,15 | 35,15 | 35,15 |
| | Phenoxyethanol | 0,65 | 0,65 | 0,65 |
| | Methylparaben | 0,35 | 0,35 | 0,35 |
| | Magnesium Sulfate | 0,65 | 0,65 | 0,65 |
| | Caprylyl Glycol | 0,45 | 0,45 | 0,45 |
| | | 100 | 100 | 100 |

(1) NAI-C33-9001-10 par la société MIYOSHI KASEI

(2) NAI-C33-8001-10 par la société MIYOSHI KASEI

(3) NAI-C33-7001-10 par la société MIYOSHI KASEI

(4) NAI-TAO-77891 par la société MIYOSHI KASEI

(5) PFX-5-Sunpuro Yellow C33-9001 par la société DAITO KASEI KOGYO

(6) PFX-5-Sunpuro Red C33-8001 par la société DAITO KASEI KOGYO

(7) PFX-5-Sunpuro Black C33-7001 par la société DAITO KASEI KOGYO

(8) PF 5 TiO2 A 100 par la société DAITO KASEI KOGYO

(9) UNIPURE YELLOW LC182 AS EM par la société LCW

(10) UNIPURE RED LC381 AS EM par la société LCW

(11) UNIPURE BLACK LC989 AS-EM par la société LCW

(12) UNIPURE WHITE LC 981 AS-EM par la société LCW

• Mode opératoire

On pèse les constituants de la phase A3. Le mélange est passé sur broyeur tri-cylindre.
On pèse ensuite les constituants de la phase A1 dans le bêcher principal et on le place au bain-marie (75-80°C). Lorsque le mélange est homogène, on ajoute les constituants de la phase A2. Après 5 minutes d'agitation au Moritz à 1500 tours/min, on refroidit le mélange jusqu'à température ambiante. On incorpore A3 à la phase A1+A2, sous agitation au Moritz à 1500 tours/min.
Puis on ajoute successivement les constituants de la phase A4, en gardant la même agitation.
On pèse les constituants de la phase B. On porte à ébullition la phase B, jusqu'à dissolution complète des constituants. On refroidit la phase B jusqu'à 50°C.
La phase B est ensuite ajoutée au filet dans la phase A1+A2+A3+A4, sous agitation au Moritz à 3200 tr/min. L'agitation est maintenue pendant minimum 10 minutes.

• Protocole de mesures instrumentales de la couleur immédiate et tenue de la couleur

On effectue une mesure colorimétrique de la peau avant et après maquillage en mesurant les indices Rouge, Jaune et luminance, respectivement a*, b*, L*. Pour chaque femme on saisit une image à l'aide d'une Chromasphère, de définition 410x410 pixels.
Les résultats sont exprimés de la manière suivante. La couleur est quantifiée par les indices Rouge, Jaune et luminance analysés par la caméra (respectivement a*, b*, L*). La tenue de la couleur est calculée par la variation de ces variables après 3heures de maquillage (deltaE94).
Plus précisément, les mesures sont effectuées sur un panel de personnes, qui sont gardées en salle d'attente climatisée (22 °C +/- 2 °C) 15 mn avant le début du test. Elles se démaquillent et une image d'une de leurs joues est acquise à l'aide de la chromasphère de définition 410x410 pixels. Cette image permet de mesurer la couleur à T0 avant maquillage.
Puis environ 100 mg de composition cosmétique sont pesés dans un verre de montre, et sont appliqués aux doigts nus sur le demi visage sur lequel la mesure à T0 a été réalisée.
Après un temps de séchage de 15 mn, une image de la joue maquillée est acquise à l'aide de la Chromasphère. Cette image permet de mesurer la couleur juste après maquillage (Timm). Les modèles retournent alors en salle climatisée pendant 3 h. Enfin, une image de la joue maquillée après 3h d'attente est acquise à l'aide de la Chromasphère. Cette image permet de mesurer la couleur après 3 h de maquillage (T3h).
Les résultats sont exprimés en calculant la différence (Timm - T0) qui mesure l'effet du maquillage. La différence (T3h - Timm) mesurant la tenue de cet effet est ensuite calculée. Chaque image obtenue à l'aide de la caméra est exploité en couleur. La couleur est quantifiée par les indices de rouge et de jaune, la luminance, l'écart de couleur (respectivement a*,b*L et deltaE). Le delta E, dE ou encore ΔE, est défini comme une mesure de différence entre deux couleurs.
Voici la formule établie en 1976 :

$$\left|\Delta E^* = \sqrt{((L_1 - L_2)^2 + (a_1 - a_2)^2 + (b_1 - b_2)^2}\right|$$

où:

$L_1,a_1,b_1$ sont les coordonnées dans l'espace colorimétrique de la première couleur à comparer et $L_2,a_2,b_2$ celles

de la seconde.

• <u>Protocole de mesures instrumentales de la matité immédiate et tenue de la matité</u>

On évalue l'effet matité et tenue de la matité de l'application, sur la peau d'un panel de personnes, de l'émusion E/H décrite précédemment. La matité et la tenue de la matité peuvent être mesurées au moyen du protocole décrit ci-après. La matité d'une région de la peau, par exemple, du visage est mesurée à l'aide d'une caméra polarimétrique, qui est un système d'imagerie polarimétrique en noir et blanc, avec laquelle des images en lumière polarisée parallèle (P) et croisée (C) sont acquises. Par analyse de l'image résultant de la soustraction des deux images (P-C), la brillance est quantifiée en mesurant le niveau de gris moyen des 5 % de pixels les plus brillants correspondant aux zones de brillance.
Plus précisément, les mesures sont effectuées sur un panel de personnes, qui sont gardées en salle d'attente climatisée (22 °C +/- 2 °C) 15 mn avant le début du test. Elles se démaquillent et une image d'une de leurs joues est acquise avec la caméra polarimétrique. Cette image permet de mesurer la brillance à T0 avant maquillage. Puis environ 100 mg de la composition décrite ci-dessus sont pesés dans un verre de montre, et sont appliqués aux doigts nus sur le demi visage sur lequel la mesure à T0 a été réalisée.
Après un temps de séchage de 15 mn, une image de la joue maquillée est acquise avec la caméra polarimétrique. Cette image permet de mesurer la brillance juste après maquillage (Timm). Les modèles retournent alors en salle climatisée pendant 3 h. Enfin, une image de la joue maquillée après 3h d'attente est acquise avec la caméra polarimétrique. Cette image permet de mesurer la brillance après 3 h de maquillage (T3h). Les résultats sont exprimés en calculant la différence (Timm - T0) qui mesure l'effet du maquillage. Une valeur négative signifie que le maquillage diminue la brillance de la peau et qu'il est donc matifiant.
La différence (T3h - Timm) mesurant la tenue de cet effet est ensuite calculée. La valeur obtenue doit être la plus faible possible ce qui signifie que la matité du maquillage ne change pas au cours de temps.

• <u>Protocole de mesures instrumentales de l'homogénéité immédiate et tenue de l'homogénéité</u>

On effectue une mesure colorimétrique de la peau avant et après maquillage en mesurant les moyennes pour les plans a* (vert-rouge), b*(bleu-jaune), L*(luminance). Pour chaque femme on saisit une image à l'aide d'une Chromasphère, de définition 410x410 pixels. Plus précisément, les mesures sont effectuées sur un panel de personnes, qui sont gardées en salle d'attente climatisée (22 °C +/- 2 °C) 15 mn avant le début du test. Elles se démaquillent et une image d'une de leurs joues est acquise à l'aide de la Chromasphère de définition 410x410 pixels. Cette image permet de mesurer la couleur à T0 avant maquillage. Puis environ 100 mg de composition cosmétique sont pesés dans un verre de montre, et sont appliqués aux doigts nus sur le demi visage sur lequel la mesure à T0 a été réalisée.
Après un temps de séchage de 15 mn, une image de la joue maquillée est acquise à l'aide de la Chromasphère. Cette image permet de mesurer la couleur juste après maquillage (Timm). Les modèles retournent alors en salle climatisée pendant 3 h. Enfin, une image de la joue maquillée après 3h d'attente est acquise à l'aide de la Chromasphère. Cette image permet de mesurer la couleur après 3 h de maquillage (T3h).
Chaque image obtenue à l'aide de la caméra est exploitée en coxellographie. L'écart type de chaque plan monochrome est calculé. Le produit des trois écarts types est égal à l'indice coxellographique. Ce paramètre est utilisé pour les calculs statistiques. Plus la peau est homogène, plus l'écart type est faible. L'indice coxellographique évolue dans le même sens puisque c'est le produit des écarts types des trois plans a*, b* et L*.
Pour les mesures effectuées, on considère que :

| | |
|---|---|
| + | effet léger ou tenue faible |
| ++ | effet moyen ou tenue moyenne |
| +++ | effet important ou tenue bonne |
| ++++ | effet très important ou tenue très bonne |

| **Compositions** | **A** (enrobage NAI) | **B** (enrobage PF) | **C** (enrobage Si) |
|---|---|---|---|
| Performance tenue de la matité | ++ | ++ | + |
| Performance tenue de la couleur | ++++ | ++++ | ++++ |
| Performance tenue de l'homogénéité | ++++ | +++ | +++ |

Les compositions présentent un effet matifiant et homogénéisant stisfaisant, voire très satisfaisant pour les pigments NAI.

Les mesures réalisées montrent qu'en terme de propriétés de tenue globale (matité, couleur et homogénéité) les pigments enrobés NAI et enrobés PF présentent les meilleurs résultats.

On reproduit des compositions selon cet exemple, dans lesquelles on remplace le mélange de résines MQ / TPropyl 60/40 selon l'exemple 1-h décrit ci-dessus par le mélange de résines MQ/TPropyl selon l'exemple 1-f décrit ci-dessus, comprenant un rapport pondéral MQ/TPropyle d'environ 50/50, avec lequel on obtient des résultats similaires.

## Revendications

1. Procédé cosmétique de maquillage et/ou de soin des matières kératiniques comprenant l'application sur lesdites matières kératiniques, notamment de la peau, d'une composition comprenant, dans un milieu physiologiquement acceptable :

   a) une résine de siloxane comprenant au moins 80% en moles d'unités :

   (i) $(R'_3SiO_{1/2})_a$ (ci-après unités "M") et
   (ii) $(SiO_{4/2})_b$ (ci-après unités « Q »)

   dans lesquelles

   • R' représente indépendamment un groupement alkyle ayant de 1 à 8 atomes de carbone, un groupement aryle, un groupement carbinol ou un groupement amino,

   avec la condition qu'au moins 95% en moles des groupes R' sont des groupes alkyles,

   • a et b ont des valeurs strictement supérieures à 0 ;
   • et le rapport a/b est compris entre 0,5 et 1,5,

   b) une résine propyl silsesquioxane comprenant au moins 80% en moles d'unités $(R''SiO_{3/2})$ (ci-après unités "T") dans lesquelles R" représente indépendamment un groupement alkyle ayant de 1 à 8 atomes de carbone, un groupement aryle, un groupement carbinol ou un groupement amino, avec la condition qu'au moins 80% en moles des groupes R" sont des groupes propyles,
   le rapport pondéral entre les résines a) et b) étant compris entre 1/99 et 99/1, en particulier entre 85/15 et 15/85, les résines a) et b) n'étant pas liées l'une avec l'autre par des liaisons covalentes,
   et le nombre d'unités M du mélange final étant strictement inférieur au nombre d'unités (T+Q),
   et
   c) au moins une matière colorante pulvérulente traitée en surface par un agent hydrophobe choisi parmi un composé de nature fluorée, un acide aminé, les acides aminés N-acylés ou leurs sels, et un de leurs mélanges.

2. Procédé cosmétique de maquillage et/ou de soin des matières kératiniques comprenant l'application sur lesdites matières kératiniques, notamment de la peau, d'une composition comprenant, dans un milieu physiologiquement acceptable :

   a) une résine de siloxane comprenant au moins 80% en moles d'unités :

   (i) $(R'_3SiO_{1/2})_a$ (ci-après unités "M") et
   (ii) $(SiO_{4/2})_b$ (ci-après unités « Q »)

   dans lesquelles

   • R' représente indépendamment un groupement alkyle ayant de 1 à 8 atomes de carbone, un groupement aryle, un groupement carbinol ou un groupement amino,

   avec la condition qu'au moins 95% en moles des groupes R' sont des groupes alkyles,

   • a et b ont des valeurs strictement supérieures à 0 ;

• et le rapport a/b est compris entre 0,5 et 1,5,

b) une résine propyl silsesquioxane filmogène comprenant au moins 80% en moles d'unités (R"$SiO_{3/2}$) (ci-après unités "T") dans lesquelles R" représente indépendamment un groupement alkyle ayant de 1 à 8 atomes de carbone, un groupement aryle, un groupement carbinol ou un groupement amino, avec la condition qu'au moins 40% en moles des groupes R" sont des groupes propyles,
le rapport pondéral entre les résines a) et b) étant compris entre 1/99 et 99/1, en particulier entre 85/15 et 15/85, les résines a) et b) n'étant pas liées l'une avec l'autre par des liaisons covalentes,
et le nombre d'unités M du mélange final étant strictement inférieur au nombre d'unités (T+Q),
et
c) au moins une matière colorante pulvérulente traitée en surface par un agent hydrophobe choisi parmi un composé de nature fluorée, un acide aminé, les acides aminés N-acylés ou leurs sels, et un de leurs mélanges.

**3.** Procédé selon la revendication 1 ou 2, **caractérisé en ce que** les résines a) et b) de siloxane et propyl silsesquioxane sont formulées dans la composition via un mélange susceptible d'être obtenu selon le procédé suivant :

• Mélange d'une solution de résine de siloxane avec une solution de résine propyl silsesquioxane, puis
• Chauffage, sous les conditions spécifiques suivantes :

- chauffage de façon homogène pendant au moins 1 heure, de préférence entre 1h et 5h, à une seule température ou à des paliers de températures compris entre 90°C et 250°C;
- à la condition que ce chauffage se fasse sans présence d'un catalyseur de condensation chimique entre les deux résines.

**4.** Procédé selon l'une quelconque des revendications précédentes, caractérisé en que ladite composition est une émulsion, en particulier directe ou inverse, ou une composition anhydre.

**5.** Procédé selon l'une quelconque des revendications précédentes, caractérisé en que ladite composition comprend une quantité de résines a) et b) de siloxane en matière active (matière sèche) allant de 0,5 à 60% en poids, par rapport au poids total de la composition, de préférence de 3 à 60 % en poids, et mieux de 4 à 60% en poids.

**6.** Procédé selon l'une quelconque des revendications précédentes, **caractérisé en ce que** la matière colorante pulvérulente traitée en surface par un agent hydrophobe peut être choisie parmi les pigments, les nacres et leurs mélanges.

**7.** Procédé selon l'une quelconque des revendications précédentes, **caractérisé en ce que** la teneur de ladite matière colorante pulvérulente traitée en surface par un agent hydrophobe va de 0,1 à 50% en poids, par rapport au poids total de la composition, mieux de 0,5 à 30 % en poids.

**8.** Procédé selon l'une quelconque des revendications précédentes, **caractérisé en ce que** la matière colorante pulvérulente traitée en surface par un agent hydrophobe est choisie parmi :

- L'oxyde de fer jaune/phosphate de perfluoroalkyle,
- L'oxyde de fer rouge/phosphate de perfluoroalkyle,
- L'oxyde de fer noir/phosphate de perfluoroalkyle,
- Le dioxyde de titane/phosphate de perfluoroalkyle,
- L'oxyde de fer jaune/perfluoropolymethylisopropylether,
- Le DC Red 7/perfluoropolymethylisopropylether,
- Le DC Red 6/PTFE,

et leur mélange.

**9.** Procédé selon l'une quelconque des revendications précédentes, **caractérisé en ce que** la composition comprend en outre au moins un composé choisi parmi les huiles, les tensioactifs non ioniques, les élastomères siliconés amphiphiles, les composés pâteux d'origine non animale, les agents rhéologiques épaississants ou gélifiants de phase grasse, les cires, les agents gélifiants hydrophiles, les charges, les matières colorantes pulvérulentes non traitées en surface par un agent hydrophobe, les polymères filmogènes, les tensioactifs ioniques, les fibres et leurs mélanges.

**10.** Composition de maquillage et/ou de soin des matières kératiniques, susceptible d'être mise en oeuvre dans un procédé selon l'une des revendications précédentes comprenant, dans un milieu physiologiquement acceptable, les résines a) et b) de siloxane telles que définies dans l'une des revendications 1 à 3 et au moins une matière colorante pulvérulente traitée en surface par un agent hydrophobe choisi parmi un composé de nature fluorée, un acide aminé, les acides aminés N-acylés ou leurs sels, et un de leurs mélanges.

**11.** Composition selon la revendication 10, **caractérisée en ce que** la matière colorante pulvérulente traitée en surface par un agent hydrophobe est telle que définie dans l'une des revendications 6, 7, et 8.

**12.** Ensemble cosmétique comprenant :

a. un récipient délimitant au moins un compartiment, ledit récipient étant fermé par un élément de fermeture ; et
b. une composition disposée à l'intérieur dudit compartiment, la composition étant définie selon la revendication 10 ou 11.

**13.** Ensemble cosmétique tel que défini dans la revendication 12 comprenant en outre un applicateur sous forme d'un bloc de mousse ou d'élastomère, d'un feutre, ou d'une spatule.

## Patentansprüche

**1.** Kosmetisches Verfahren zum Schminken und/oder zur Pflege von Keratinmaterialien, umfassend die Applikation auf besagte Keratinmaterialien, insbesondere Haut, einer Zusammensetzung, umfassend in einem physiologisch verträglichen Milieu:

a) ein Siloxanharz, umfassend wenigstens 80 Mol-% der Einheiten:

(i) $(R'_3SiO_{1/2})_a$ (nachstehend "M"-Einheiten) und
(ii) $(SiO_{4/2})_b$ (nachstehend "Q"-Einheiten),

in denen

• R' unabhängig für eine Alkylgruppe mit 1 bis 8 Kohlenstoffatomen, eine Arylgruppe, eine Carbinolgruppe oder eine Aminogruppe steht,

unter der Bedingung, dass wenigstens 95 Mol-% der R'-Gruppen Alkylgruppen sind,

• die Werte von a und b stets größer als 0 sind;
• und das Verhältnis a/b zwischen 0,5 und 1,5 liegt,

b) ein Propylsilsesquioxanharz, umfassend wenigstens 80 Mol-% $(R''SiO_{3/2})$-Einheiten (nachstehend "T"-Einheiten), in denen R" unabhängig für eine Alkylgruppe mit 1 bis 8 Kohlenstoffatomen, eine Arylgruppe, eine Carbinolgruppe oder eine Aminogruppe steht, unter der Bedingung, dass wenigstens 80 Mol-% der R"-Gruppen Propylgruppen sind, wobei
das Gewichtsverhältnis der Harze a) und b) zwischen 1/99 und 99/1, insbesondere zwischen 85/15 und 15/85, liegt,
die Harze a) und b) nicht durch kovalente Bindungen miteinander verbunden sind,
und die Anzahl an M-Einheiten des Endgemisches stets kleiner als die Anzahl an (T+Q)-Einheiten ist,
und
c) wenigstens einen pulverförmigen Farbstoff, der mit einem hydrophoben Agens oberflächenbehandelt ist, das aus einer fluorierten Verbindung, einer Aminosäure, N-acylierten Aminosäuren oder ihren Salzen und einem Gemisch davon ausgewählt ist.

**2.** Kosmetisches Verfahren zum Schminken und/oder zur Pflege von Keratinmaterialien, umfassend die Applikation auf besagte Keratinmaterialien, insbesondere Haut, einer Zusammensetzung, umfassend in einem physiologisch verträglichen Milieu:

a) ein Siloxanharz, umfassend wenigstens 80 Mol-% der Einheiten:

(i) $(R'_3SiO_{1/2})_a$ (nachstehend "M"-Einheiten) und
(ii) $(SiO_{4/2})_b$ (nachstehend "Q"-Einheiten),

in denen

- R' unabhängig für eine Alkylgruppe mit 1 bis 8 Kohlenstoffatomen, eine Arylgruppe, eine Carbinolgruppe oder eine Aminogruppe steht,

unter der Bedingung, dass wenigstens 95 Mol-% der R'-Gruppen Alkylgruppen sind,

- die Werte von a und b stets größer als 0 sind;
- und das Verhältnis a/b zwischen 0,5 und 1,5 liegt,

b) ein filmbildendes Propylsilsesquioxanharz, umfassend wenigstens 80 Mol-% $(R''SiO_{3/2})$-Einheiten (nachstehend "T"-Einheiten), in denen R'' unabhängig für eine Alkylgruppe mit 1 bis 8 Kohlenstoffatomen, eine Arylgruppe, eine Carbinolgruppe oder eine Aminogruppe steht, unter der Bedingung, dass wenigstens 40 Mol-% der R" -Gruppen Propylgruppen sind, wobei
das Gewichtsverhältnis der Harze a) und b) zwischen 1/99 und 99/1, insbesondere zwischen 85/15 und 15/85, liegt,
die Harze a) und b) nicht durch kovalente Bindungen miteinander verbunden sind,
und die Anzahl an M-Einheiten des Endgemisches stets kleiner als die Anzahl an (T+Q)-Einheiten ist,
und
c) wenigstens einen pulverförmigen Farbstoff, der mit einem hydrophoben Agens oberflächenbehandelt ist, das aus einer fluorierten Verbindung, einer Aminosäure, N-acylierten Aminosäuren oder ihren Salzen und einem Gemisch davon ausgewählt ist.

**3.** Verfahren gemäß Anspruch 1 oder 2, **dadurch gekennzeichnet, dass** das Siloxanharz a) und das Propylsilsesquioxanharz b) in der Zusammensetzung über ein Gemisch formuliert werden, das nach folgendem Verfahren erhalten werden kann:

- Mischen einer Siloxanharz-Lösung mit einer Propylsilsesquioxanharz-Lösung, dann
- Erhitzen unter folgenden spezifischen Bedingungen:

  - gleichmäßiges Erhitzen für wenigstens 1 Stunde, vorzugsweise zwischen 1 h und 5 h, auf eine bestimmte Temperatur oder auf Temperaturstufen zwischen 90°C und 250°C;
  - unter der Bedingung, dass dieses Erhitzen nicht in Gegenwart eines Katalysators einer chemischen Kondensation der beiden Harze erfolgt.

**4.** Verfahren gemäß einem der vorhergehenden Ansprüche, **dadurch gekennzeichnet, dass** besagte Zusammensetzung eine Emulsion, insbesondere eine inverse oder direkte Emulsion, oder eine wasserfreie Zusammensetzung ist.

**5.** Verfahren gemäß einem der vorhergehenden Ansprüche, **dadurch gekennzeichnet, dass** besagte Zusammensetzung eine Menge an Siloxanharzen a) und b) als Wirkstoff (Trockensubstanz) umfasst, die von 0,5 bis 60 Gew.-%, bevorzugt von 3 bis 60 Gew.-% und bevorzugter von 4 bis 60 Gew.-% bezogen auf das Gesamtgewicht der Zusammensetzung reicht.

**6.** Verfahren gemäß einem der vorhergehenden Ansprüche, **dadurch gekennzeichnet, dass** der mit einem hydrophoben Agens oberflächenbehandelte pulverförmige Farbstoff aus Pigmenten, Perlglanzpigmenten und Gemischen davon ausgewählt sein kann.

**7.** Verfahren gemäß einem der vorhergehenden Ansprüche, **dadurch gekennzeichnet, dass** der Gehalt an besagtem pulverförmigem Farbstoff, der mit einem hydrophoben Agens oberflächenbehandelt ist, von 0,1 bis 50 Gew.-%, bevorzugter von 0,5 bis 30 Gew.-%, bezogen auf das Gesamtgewicht der Zusammensetzung reicht.

**8.** Verfahren gemäß einem der vorhergehenden Ansprüche, **dadurch gekennzeichnet, dass** der pulverförmige Farbstoff, der mit einem hydrophoben Agens oberflächenbehandelt ist, ausgewählt ist aus:

- Eisenoxidgelb/Perfluoralkylphosphat,
- Eisenoxidrot/Perfluoralkylphosphat,
- Eisenoxidschwarz/Perfluoralkylphosphat,
- Titandioxid/Perfluoralkylphosphat,
- Eisenoxidgelb/Perfluorpolymethylisopropylether,
- DC Red 7/Perfluorpolymethylisopropylether,
- DC Red 6/PTFE

und einem Gemisch davon.

**9.** Verfahren gemäß einem der vorhergehenden Ansprüche, **dadurch gekennzeichnet, dass** die Zusammensetzung außerdem wenigstens eine Verbindung umfasst, die aus Ölen, nichtionischen Tensiden, amphiphilen Silikonelastomeren, pastösen Verbindungen nichttierischen Ursprungs, viskositätserhöhenden oder gelbildenden rheologischen Mitteln der Fettphase, Wachsen, hydrophilen gelbildenden Mitteln, Füllstoffen, pulverförmigen Farbstoffen, die nicht mit einem hydrophoben Agens oberflächenbehandelt sind, filmbildenden Polymeren, ionischen Tensiden, Fasern und Gemischen davon ausgewählt sind.

**10.** Zusammensetzung zum Schminken und/oder zur Pflege von Keratinmaterialien, die für eine Verwendung in einem Verfahren gemäß einem der vorhergehenden Ansprüche geeignet ist, umfassend in einem physiologisch verträglichen Milieu die wie in einem der Ansprüche 1 bis 3 definierten Siloxanharze a) und b) und wenigstens einen pulverförmigen Farbstoff, der mit hydrophoben Agens oberflächenbehandelt ist, das aus einer fluorierten Verbindung, einer Aminosäure, N-acylierten Aminosäuren oder ihren Salzen und einem Gemisch davon ausgewählt ist.

**11.** Zusammensetzung gemäß Anspruch 10, **dadurch gekennzeichnet, dass** der pulverförmige Farbstoff, der mit einem hydrophoben Agens oberflächenbehandelt ist, wie in einem der Ansprüche 6, 7 und 8 definiert ist.

**12.** Kosmetikset umfassend:

a. einen Behälter, der wenigstens eine Kammer begrenzt, wobei besagter Behälter mit einem Verschließelement verschlossen ist; und
b. eine Zusammensetzung, die im Innern besagter Kammer angeordnet ist, wobei die Zusammensetzung gemäß einem der Ansprüche 10 oder 11 definiert ist.

**13.** Kosmetikset, wie in Anspruch 12 definiert, umfassend außerdem einen Applikator in Form eines Schaumstoff- oder Elastomerblocks, eines Filzes oder einer Spachtel.

## Claims

**1.** A cosmetic method for making up and/or caring for keratin materials, comprising the application, to said keratin materials, in particular the skin, of a composition comprising, in a physiologically acceptable medium:

a) a siloxane resin comprising at least 80 mol% of units:

(i) $(R'_3SiO_{1/2})_a$ (hereinafter "M" units) and
(ii) $(SiO_{4/2})_b$ (hereinafter "Q" units)

in which

• R' independently represents an alkyl group containing from 1 to 8 carbon atoms, an aryl group, a carbinol group or an amino group,

with the condition that at least 95 mol% of the R' groups are alkyl groups,

• a and b are values strictly greater than 0,
• and the ratio a/b is between 0.5 and 1.5;

b) a propyl silsesquioxane resin comprising at least 80 mol% of $(R''SiO_{3/2})$ units (hereinafter "T" units) in which

R" independently represents an alkyl group containing from 1 to 8 carbon atoms, an aryl group, a carbinol group or an amino group, with the condition that at least 80 mol% of the R" groups are propyl groups;
the weight ratio between the resins a) and b) being between 1/99 and 99/1, in particular between 85/15 and 15/85, the resins a) and b) not being bonded to one another via covalent bonds,
and the number of M units in the final mixture being strictly less than the number of (T+Q) units;
and
c) at least one pulverulent dyestuff that has been surface-treated with a hydrophobic agent chosen from a compound of fluorinated nature, an amino acid, N-acylated amino acids or salts thereof, and a mixture thereof.

**2.** A cosmetic method for making up and/or caring for keratin materials, comprising the application, to said keratin materials, in particular the skin, of a composition comprising, in a physiologically acceptable medium:

a) a siloxane resin comprising at least 80 mol% of units:

(i) $(R'_3SiO_{1/2})_a$ (hereinafter "M" units) and
(ii) $(SiO_{4/2})_b$ (hereinafter "Q" units)

in which

• R' independently represents an alkyl group containing from 1 to 8 carbon atoms, an aryl group, a carbinol group or an amino group,

with the condition that at least 95 mol% of the R' groups are alkyl groups,

• a and b are values strictly greater than 0,
• and the ratio a/b is between 0.5 and 1.5;

b) a film-forming propyl silsesquioxane resin comprising at least 80 mol% of $(R''SiO_{3/2})$ units (hereinafter "T" units) in which R" independently represents an alkyl group containing from 1 to 8 carbon atoms, an aryl group, a carbinol group or an amino group, with the condition that at least 40 mol% of the R" groups are propyl groups,
the weight ratio between the resins a) and b) being between 1/99 and 99/1, in particular between 85/15 and 15/85, the resins a) and b) not being bonded to one another via covalent bonds,
and the number of M units in the final mixture being strictly less than the number of (T+Q) units;
and
c) at least one pulverulent dyestuff that has been surface-treated with a hydrophobic agent chosen from a compound of fluorinated nature, an amino acid, N-acylated amino acids or salts thereof, and a mixture thereof.

**3.** The method as claimed in claim 1 or 2, **characterized in that** the siloxane and propyl silsesquioxane resins a) and b) are formulated in the composition via a mixture that can be obtained according to the following method:

• mixing a solution of siloxane resin with a solution of propyl silsesquioxane resin, then
• heating, under the following specific conditions:

- heating uniformly for at least one hour, preferably between 1 h and 5 h, at a single temperature or at temperature holds of between 90°C and 250°C;
- provided that this heating is carried out without the presence of a catalyst for chemical condensation between the two resins.

**4.** The method as claimed in any one of the preceding claims, **characterized in that** said composition is an emulsion, in particular a direct or inverse emulsion, or an anhydrous composition.

**5.** The method as claimed in any one of the preceding claims, **characterized in that** said composition comprises an amount of siloxane resins a) and b), with respect to active material (dry matter), ranging from 0.5% to 60% by weight, relative to the total weight of the composition, preferably from 3% to 60% by weight, and better still from 4% to 60% by weight.

**6.** The method as claimed in claim 1 or 2, **characterized in that** the pulverulent dyestuff that has been surface-treated

with a hydrophobic agent may be chosen from pigments and pearlescent agents, and mixtures thereof.

**7.** The method as claimed in any one of the preceding claims, **characterized in that** the content of said pulverulent dyestuff that has been surface-treated with a hydrophobic agent ranges from 0.1 % to 50% by weight, relative to the total weight of the composition, better still from 0.5% to 30% by weight.

**8.** The method as claimed in any one of the preceding claims, **characterized in that** the pulverulent dyestuff that has been surface-treated with a hydrophobic agent is chosen from:

- yellow iron oxide/perfluoroalkyl phosphate,
- red iron oxide/perfluoroalkyl phosphate,
- black iron oxide/perfluoroalkyl phosphate,
- titanium dioxide/perfluoroalkyl phosphate,
- yellow iron oxide/perfluoropolymethyl isopropyl ether,
- DC Red 7/perfluoropolymethyl isopropyl ether,
- DC Red 6/PTFE,

and mixtures thereof.

**9.** The method as claimed in any one of the preceding claims, **characterized in that** the composition also comprises at least one compound chosen from oils, nonionic surfactants, amphiphilic silicone elastomers, pasty compounds of nonanimal origin, fatty-phase thickening or gelling rheological agents, waxes, hydrophilic gelling agents, fillers, pulverulent dyestuffs that have not been surface-treated with a hydrophobic agent, film-forming polymers, ionic surfactants, fibers, and mixtures thereof.

**10.** A composition for making up and/or caring for keratin fibers, that can be used in a method as claimed in any one of the preceding claims, comprising, in a physiologically acceptable medium, the siloxane resins a) and b) as defined in any one of claims 1 to 3 and at least one pulverulent dyestuff that has been surface-treated with a hydrophobic agent chosen from a compound of fluorinated nature, an amino acid, N-acylated amino acids or salts thereof, and a mixture thereof.

**11.** The composition as claimed in claim 10, **characterized in that** the pulverulent dyestuff that has been surface-treated with a hydrophobic agent is as defined in one of claims 6, 7 and 8.

**12.** A cosmetic assembly comprising:

a. a container delimiting at least one compartment, said container being closed by means of a closing member; and
b. a composition placed inside said compartment, the composition being defined as claimed in claim 10 or 11.

**13.** The cosmetic assembly of claim 12, also comprising an applicator in the form of a block of foam or elastomer, a felt or a spatula.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 2814601 A
- US 2857356 A
- WO 2005075567 A
- WO 2007145765 A
- US 4578266 A
- EP 486135 A
- JP H0586984 B
- US 5236986 A
- US 5412004 A
- US 5837793 A
- US 5811487 A
- WO 2007068371 A
- US 5783657 A
- US 5874069 A
- US 5919441 A
- US 6051216 A
- US 5981680 A
- WO 2003106614 A
- EP 1068854 A
- EP 1086945 A
- WO 0247031 A
- WO 9323008 A
- FR 2910809
- US 20040234477 A
- US 5538793 A
- EP 898958 A
- FR 2792190 A
- EP 0216479 A
- US 3915921 A
- US 4509949 A
- US 5188899 A
- WO 04055081 A
- FR 2232303 A
- US 5162410 A
- WO 2004073626 A
- US 4693935 A
- US 4981903 A
- US 4981902 A
- EP 1411069 A
- WO 04028488 A
- EP 0963751 A
- WO 0196450 A
- GB 2407496 A, Dow Corning
- US 4887622 A
- FR 2796529
- FR 2722380
- US 5492426 A
- FR 2761959
- WO 0103538 A
- FR 2806273
- FR 2775566
- FR 2727609
- WO 03018423 A
- FR 2791042
- FR 2792618
- US 2676182 A


### Littérature non-brevet citée dans la description


- *J. Soc. Cosm. Chem.,* 1954, vol. 5, 249-256
- Encyclopedia of Chemical Technology, KIRK-OTHMER. WILEY, 1979, vol. 22, 333-432
- Silica silylate. CTFA, 1995
- Silica diméthyl silylate. CTFA, 1995
- **P. TERECH.** Specialist Surfactants. 1997, 209-263